# EUROPEAN PATENT APPLICATION

(11) **EP 0 875 574 A2**
(43) Date of publication of application: **04.11.1998**
(21) Application number: 98201115.7
(22) Date of filing: 08.04.1998
(51) Int. Cl.: C12N 15/31, A61K 39/102, A61K 39/295, G01N 33/569, G01N 33/68, C12N 1/20, C12N 1/21

(54) **Live attenuated bacteria of the species Actinobacillus pleuropneumoniae**

(30) Priority: 10.04.1997 EP 97201032
(71) Applicant: Akzo Nobel N.V., 6824 BM Arnhem (NL)
(72) Inventor: Segers, Ruud, P.A.M., 5831 MZ Boxmeer (NL); Frey, Joachim, 3054 Schupfen (CH)
(74) Representative: Mestrom, Joannes Jozef Louis

(57) **Abstract**

The present invention relates to live attenuated bacteria of the genus *Actinobacillus pleuropneumoniae* that have a mutation in an *apxIV* gene such that no functional ApxlV toxin can be produced. The invention also relates to methods for the production of such bacteria. Also vaccines comprising such bacteria and methods for the production of such vaccines are part of the invention. The invention further relates to subunit vaccines comprising an ApxlV toxin, to methods for the production of such vaccines and to methods for the protection of animals against infection with bacteria of the genus *Actinobacillus pleuropneumoniae.* In addition, the invention relates to the promotor of the *apxlV* gene. Finally, the invention relates to diagnostic tests for the selective diagnosis of *Actinobacillus pleuropneumoniae* infections and to diagnostic tests discriminating between *Actinobacillus pleuropneumoniae* field strains and vaccine strains.

## Description

The present invention relates to live attenuated bacteria of the genus *Actinobacillus pleuropneumoniae,* having a mutation in a gene encoding a toxin, methods for the production of such bacteria, to vaccines comprising such bacteria, methods for the production of such vaccines, to vaccines comprising a toxin, methods for the production of such vaccines and methods for the protection of animals against infection with bacteria of the genus *Actinobacillus pleuropneumoniae.*
Bacteria belonging to the genus *Actinobacillus* all produce so-called RTX-toxins. (RTX stands for repeat in toxin).
It is the presence of these RTX-toxins that highly contributes to the pathogenic character of these bacteria.
The RTX-toxins have been extensively reviewed by Braun et al. (Critical Rev. in Microbiol. 18(2):115-158 (1991)). RTX-toxins in Gram-negative strains have also been reviewed in Welch, R.A. (Molecular Microbiology 5/3: 521-528 (1991)) and in Welch et al. (Inf. Agents and Disease 4: 254-272 (1995)).
All known RTX-toxins display some kind of cytotoxic or cytolytic activity. The target-cell-and host-specificity differ however, depending on the toxin and on differences in acylation (McWhinney et al.; J. Bact. 174: 291-297 (1992) and Hackett et al.; J. Biol. Chem. 270: 20250-20253 (1995)) . As a result of the difference in target cells, the various toxins of the RTX-toxin family are known e.g. as haemolysin, cytolysin or cytotoxin.
The genus *Actinobacillus* comprises a number of different species, inter alia, *Actinobacillus pleuropneumoniae, A. actinomycetemcomitans, A. suis, A. rossii, A. equuli and A. lig-nieresii.*
*Actinobacillus pleuropneumoniae* produces serotype-dependent RTX-toxins that are cytotoxic/cytolytic to pig, horse, bovine and human erythrocytes, to rabbit and porcine neutrophils and to porcine alveolar macrophages. (Rosendal et al; Am. J. Vet. Res. 49: 1053-1058 (1988), Maudsley J.R. and Kadis S; Can. J. Microbiol. 32: 801-805 (1986), Frey. J and Nicolet. J; Inf. & Imm. 56:2570-2575 (1988), Bendixon et al; Inf. & Imm. 33: 673-676 (1981), Kamp, E.M. and van Leengoed, L.A.M.G.; J. Clin. Microbiol. 27: 1187-1191 (1989)).
Infections with *Actinobacillus* in pigs are the cause of severe economic losses to pig industry, due to acute mortality in young pigs and reduced weight gain in older animals.
The genetic organisation of the operons involved in the synthesis, activation and transportation of the RTX toxins in Gram-negative bacteria has been reviewed recently by Coote, J.G. (FEMS Microbiology reviews 88: 137-162 (1992)). Frey has specifically reviewed the known three RTX-toxins in *Actinobacillus pleuropneumoniae* in Bacterial Protein Toxins, Zbl Bakt. Suppl. 24, p. 322-, Freer et al. (Eds.), Gustaf Fischer, Stutttgart, Jena, New York, 1994.
In *Actinobacillus pleuropneumoniae,* this kind of RTX-operon contains four genes: the actual Toxin-gene (A), an Activator-gene (C), and two genes (B and D) encoding proteins involved in secretion of the toxin into the surrounding medium. The primary translation-product of the Toxin-gene (A) is a non-toxic protein, of which the toxic activity is activated by the Activator-gene (C) product.
Until recently, it was assumed that only three RTX-toxins, all having the above-described genetic organisation or at least having the Toxin-gene (A) and Activator-gene (C) , existed in *Actinobacillus* species.
These three RTX-toxins, ApxI, Apx-II and Apx-III have respectively a pronounced haemolytic activity (Apxl), a mild haemolytic activity (Apx-ll) or a macrophage-cytotoxic activity (Apx-lll).

The various toxic activities are fairly randomly divided over the serotypes. There are four subgroups:
a subgroup A, represented by serotypes 1, 5, 9 and 11, producing Apxl and Apx-ll,
a subgroup B, represented by serotypes 2, 3, 4, 6 and 8, producing Apx-ll and Apx-lll,
a subgroup C, represented by serotype 10, producing Apxl only,
a subgroup D, represented by serotype 7 and 12, producing Apx-II only,

It is known, that Apxl, -II, and -III all are essential elements in universal vaccines against *Actinobacillus pleuropneumoniae* infection: a vaccine not comprising at least Apxl, -II, and -III will not provide protection against all *Actinobacillus pleuropneumoniae* serotypes. Also, a vaccine not comprising at least the Apx-toxins of one specific serotype will not even induce protection against that single serotype.

Subunit vaccines based on in vitro synthesised RTX-toxins from *A. pleuropneumoniae* that lost their toxicity have been described earlier, e.g. in European Patent EP No. 0.354.628, in which subunit vaccines based upon a haemolysin and a cytotoxin of *A. pleuropneumoniae* are disclosed, and in European Patent EP No 0.453.024, in which *A. pleuropneumoniae* subunit vaccines based upon haemolysins, cytotoxins and outer membrane proteins are disclosed.

There are however four important disadvantages to subunit vaccines in general:
- high amounts of antigenic material are needed in order to adequately trigger the immune system.
- usually, only B-cell immunity is triggered.
- several protective antigens are only triggered *in vivo,* and therefore can not be present in subunit vaccines.
- a live pathogenic bacterium has many important immunogenic molecules, such as Outer Membrane Proteins and capsular polysaccharides, all potentially important for protection and thus to be included in an efficient subunit vaccine.
Next to the obvious problems mentioned under points one and two, especially the fourth point makes it difficult to make an efficient subunit vaccine.
This is e.g. illustrated by the *A. pleuropneumoniae* subunit vaccine disclosed in European Patent EP No 0.453.024 mentioned above, in which four different subunits (three RTX-toxins and an outer membrane protein) are combined in one vaccine.

It is clear, that in order to overcome the disadvantages of subunit vaccines against *Pasteurellaceae*-infection, a live attenuated vaccine would be highly desirable.
A live attenuated vaccine has the following advantages:
it can be administered in low doses (it is self-replicating)
it closely mimics the natural/wild-type infection
it provides all the possible immunologically important antigens at the same time.
Nevertheless, in spite of the clear advantages, no live vaccines based on *Actinobacillus pleuropneumoniae* were commercially available prior to the present invention. The reason for this lies in the following paradox: as mentioned before, Apxl, -II, and -III all are essential elements of universal vaccines against *Actinobacillus pleuropneumoniae* infection. Live vaccines therefore have to produce these three RTX-toxins. These three RTX-toxins are however strong virulence factors in all *Actinobacillus* species (see e.g. Coote, J.G.; FEMS Microbiology reviews 88: 137-162 (1992), Tascon et al.; Mol. Microbiol. 14: 207-216 (1994)), Jansen et al.; Inf. & Imm. 63: 27-37 (1995)).
Deletion of the RTX-toxins in order to attenuate the virulence of live App strains is technically feasible, but this does not provide a solution for the dilemma: such RTX-negative strains would be useless as live attenuated vaccine strains since they do no longer induce immunity in the host against the haemolytic/cytotoxic activity of *Actinobacillus pleuropneumoniae* field strains.
Virulence factors that, although important in the induction of immunity, do play a less important role in building up immunity than Apxl, -II and -III, and thus can in principle be deleted are however currently not known.

It would thus be highly desirable to have a site on the genome of App that attributes to virulence and therefore leads to an attenuated App strain when modified, whereas at the same time it is, although useful in triggering immunity, dispensable from a vaccine point of view. No such sites are however currently known. Moreover, it would be highly desirable if such a site would be universally present in all App strains, instead of being restricted to certain serotypes. Such a site would then allow all different serotypes to be attenuated by deletion of that same site.

It is one of the objectives of the present invention to provide such an attenuation site, universally present in all *Actinobacillus pleuropneumoniae* strains regardless their serotype.

Recently, a new gene was found in a serotype 1 strain of *Actinobacillus pleuropneumoniae* (Thesis T.J. Anderson Nov. 1995).
Although this gene does not resemble the known *Actinobacillus* Apxl, -II and -III genes, it bears resemblance to RTX-toxin genes known from bacteria belonging to *Neisseria meningitidis,* for which reason it was named RTX-gene *apxIV.* The gene however differs in almost all aspects from the three known RTX-toxin genes *apxl, -II* and -III present in the various species of the *Actinobacillus* family as described above. First of all, the genomic organisation is completely different. Secondly, there is no activator-mechanism as is found for the known Apx-toxins. In the third place, no specific *in vivo* haemolytic or cytotoxic activity could at that time be attributed to the gene, or it's possible gene product.
It was now surprisingly found that this gene, fully in contrast with the three known RTX-genes, is present in all bacteria of the species *Actinobacillus pleuropneumoniae,* regardless their serotype. This was determined by hybridisation of a probe comprising apxlV coding sequences with restriction fragments of the DNA from *Actinobacillus pleuropneumoniae* of all serotypes as described in Example 6 and 7.
Unexpectedly it was found now, that *apxIV* deletion mutants are viable, but they behave less virulent compared to their apxIV-possessing parent strains.
Therefore, it was determined that the gene product, the ApxlV toxin is a virulence factor in all *Actinobacillus pleuropneumoniae* strains. This is an unexpected conclusion, since up until now, no effects at al, let alone effects possibly influencing virulence had been attributed to the gene product *in vivo.* In fact, up until now there was not even proof that the gene was expressed in *Actinobacillus pleuropneumoniae in vivo* or *in vitro* anyway.
It therefore is one of the merits of the invention that it was found that:
- the *apxIV* gene is present in all *A. pleuropneumoniae* strains regardless the serotype,
- the *apxIV* gene product is a virulence factor in all *A. pleuropneumoniae* serotypes,
- *A. pleuropneumoniae* strains with a deletion in the *apxIV* gene are still viable but have a decreased virulence without significantly impairing the immunogenic properties of the strains,

Therefore, the invention provides for the first time live attenuated bacteria of the species *Actinobacillus pleuropneumoniae,* that do not produce a functional ApxIV toxin.

A functional ApxlV toxin is considered to be a protein that has all the characteristics of the ApxlV toxin as expressed in a wild-type bacterium, and is expressed at the wild-type level. Therefore, a non-functional ApxlV toxin is considered to be a toxin that lacks some or all of the characteristics of the ApxlV toxin as expressed in a wild-type bacterium, and/or is expressed at a level, insufficient to obtain wild-type effects of ApxlV toxin.

The inability to produce the ApxlV toxin can e.g. be due to modifications in the coding sequence encoding the ApxlV toxin. It may also be e.g. the result of modifications in regions known to be involved in transcription of the *apxIV* gene, such as the promotor region, or of modifications in regions involved in translation, such as the ribosome binding site.

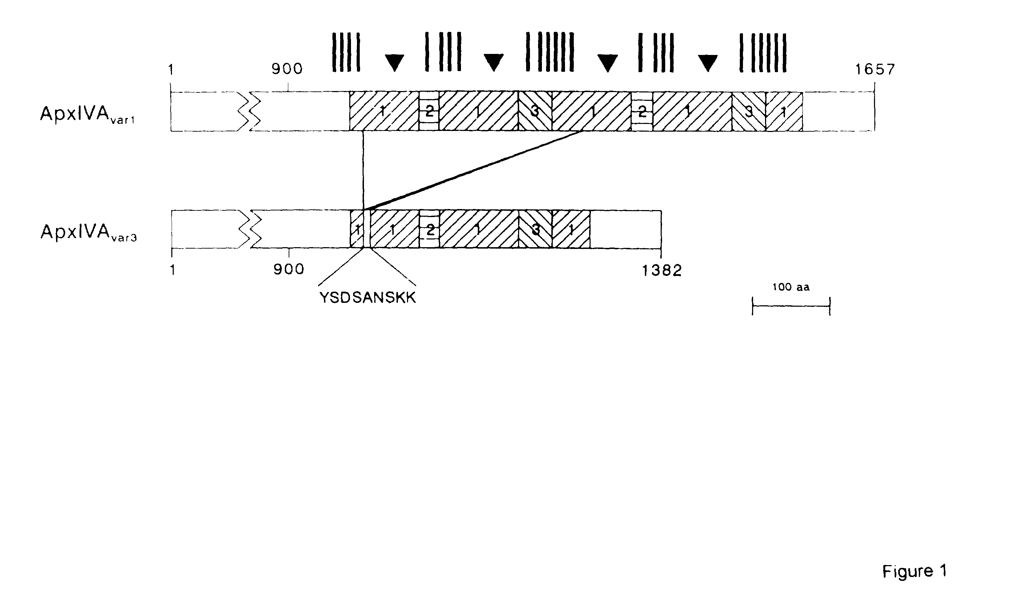

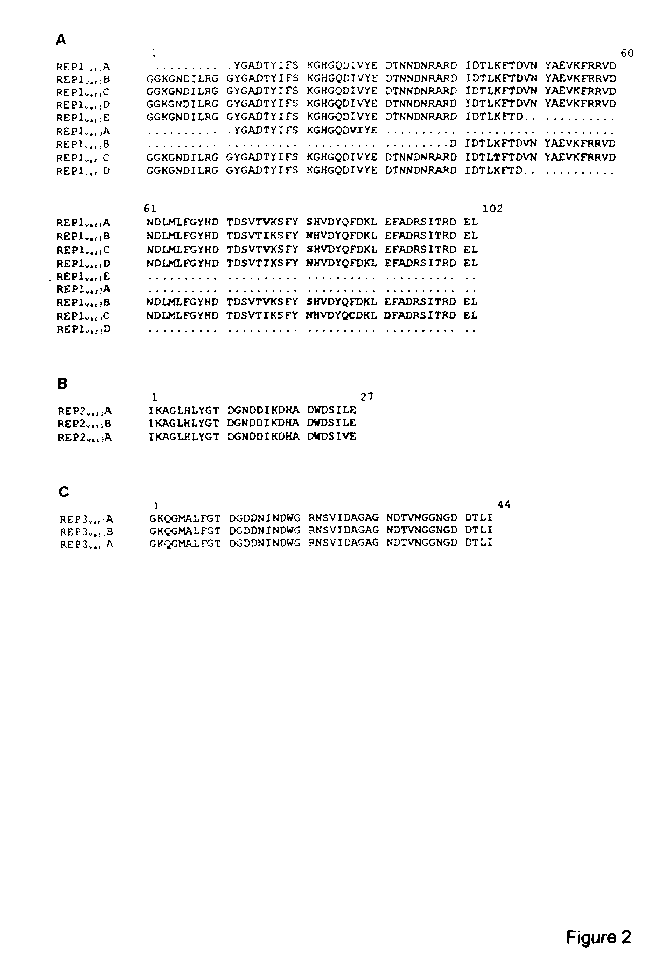

The overall structure of the *apxIV* gene is given in figure 1.
In this figure, the direct repeat regions, characteristic for ApxlV toxin are indicated by dashed boxes, whereas the also ApxlV-specific glycine-rich nonapeptide regions are indicated by black arrows. The repeats are found at the C-terminal part of ApxlV. These characteristic features are present in all *Actinobacillus pleuropneumoniae* serotypes. The nucleic acid sequence and amino acid sequence of two serotypes are represented in SEQ. ID. No. 1-4. SEQ. ID. NO. 1 shows the nucleic acid sequence of the *apxIV* gene of App serotype 1, and SEQ. ID. NO. 2 shows the matching amino acid sequence of the serotype 1 ApxIV toxin. SEQ. ID. NO. 3 shows the nucleotide sequence of the *apxIV* gene of App serotype 3, whereas SEQ. ID. NO. 4 shows the matching amino acid sequence of the serotype 3 ApxlV toxin. Figure 2 shows the strikingly high level of conservation at amino acid level, especially in the N-terminal 650 amino acids, between the Apx-toxins of the various *Actinobacillus pleuropneumoniae* serotypes. This is also a remarkable characteristic of the *apxIV* genes. It is clear from figure 1, that a variation in the number of repeats at the C-terminal part of the toxin may occur, depending on the serotype. This variation accounts for the difference in size of the genes and encoded toxins obtained from the various serotypes.

There may be some variation in nucleic acid sequence even between *apxIV* genes isolated from different isolates of *Actinobacillus pleuropneumoniae,* belonging to the same serotype. This is due to natural variation well known in the art to exist in all organisms. It is possible that some amino acids in the ApxlV toxin encoded by the *apxIV* gene are replaced by others in the ApxlV toxin of another serotype, while the polypeptide is not altered in its function. For instance, a polypeptide containing Asp at a certain site, and its variant containing Asn at the comparable site still have the same properties. This process in which an amino acid is replaced by an functionally analogous amino-acid is called functional displacement. In this case the variant proteins are called functional variants. Another cause of variation is the phenomenon of degeneracy of the genetic code. Shortly, it means, that e.g. the amino acid glutamic acid is coded for by both GAT and GAA. This phenomenon holds for all amino acids, except Met and Trp. Thus, it is obvious, that e.g. the ApxlV toxin of serotype 1, as given in the present invention can not only be coded for by the nucleotide sequence given in SEQ ID NO: 1 but also by a very large variety of other sequences, still all giving the same or functionally the same polypeptides.

Therefore, a variant apxlV sequence encoding a polypeptide that is functionally comparable to the ApxlV toxin falls within the scope of the present invention.

Live attenuated bacteria according to the invention can be obtained in several ways. One possible way of obtaining such bacteria is by means of classical methods such as the treatment of wild-type *Actinobacillus pleuropneumoniae* bacteria with mutagenic agents such as base analogues, treatment with ultraviolet light or temperature treatment. Strains that do not produce a functional ApxlV toxin do not or to a lesser extend induce anti-ApxlV toxin antibodies, and therefore can easily be selected in animal tests. The necessary antiserum can be obtained as described below in Example 3.
Another possibility is to deliberately introduce, using recombinant DNA-technology, a well-defined mutation in the gene encoding the ApxlV toxin. Such a mutation may be an insertion, a deletion, a replacement of one nucleotide by another one or a combination thereof, with the only proviso that the mutated gene no longer encodes a functional ApxlV toxin. It can easily be seen, that especially mutations introducing a stop-codon in the open reading frame, or mutations causing a frame-shift in the open reading frame are very suitable to obtain a strain which no longer encodes a functional ApxlV toxin. Such mutations can e.g. be made by means of in vitro site directed mutagenesis using the Transformer® kit sold by Clontech. Many other standard recombinant DNA techniques such as digestion of the gene with a restriction enzyme, followed by endonuclease treatment and religation, are equally applicable.

Therefore, in a preferred form, this embodiment of the invention relates to live attenuated bacteria in which the gene encoding the ApxlV toxin comprises a mutation.

Well-defined mutations involving the deletion of fragments of the *apxlV* gene or even the whole gene, or the insertion of heterologous DNA-fragments, when compared to classically induced mutations, have the advantage that they will not revert to the wild-type situation.
Thus, in a more preferred form, this embodiment of the invention refers to live attenuated bacteria in which the gene encoding the ApxlV toxin comprises an insertion and/or a deletion.

Given the large amount of vaccines given nowadays to pigs, it is clear that combined administration of several vaccines would be desirable, if only for reasons of decreased vaccination costs. It is therefore very attractive to use live attenuated vaccine strains as a recombinant carrier for heterologous genes, encoding antigens selected from other pathogenic micro-organisms or viruses. Administration of such a recombinant carrier has the advantage that after administration of such a carrier, immunity is induced against two or more diseases at the same time. The live attenuated bacteria according to the present invention provide a very suitable carrier for heterologous genes, since the gene encoding the ApxlV toxin can be used as an insertion site for such heterologous genes. The use of the *apxIV* gene as an insertion site has the advantage that at the same time the *apxIV* gene is inactivated, and the newly introduced heterologous gene can be expressed in accordance with the homologous *Actinobacillus pleuropneumoniae* genes. The construction of such recombinant carriers can be done routinely, using standard molecular biology techniques such as homologous recombination. Therefore, in an even more preferred embodiment, the present invention relates to live attenuated bacteria of the species *Actinobacillus pleuropneumoniae* that do not produce a functional ApxlV toxin, and in which there is a heterologous gene inserted in the *apxIV* gene. Such a heterologous gene can, as mentioned above, e.g. be a gene encoding an antigen selected from other pathogenic micro-organisms or viruses. Another possibility is to insert a gene encoding a protein involved in triggering the immune system, such as an interleukine or an interferone.

In a still even more preferred form of the invention, the heterologous gene encodes one or more antigens selected from the group consisting of Porcine Reproductive Respiratory Syndrome (PRRS) virus, Pseudorabies virus, Porcine Influenza virus, Porcine Parvovirus, Transmissible Gastroenteritis virus, rotavirus, *Escherichia coli, Erysipelothrix rhusiopathiae, Pasteurella multocida, Bordetella bronchiseptica, Haemophilus parasuis* and *Streptococcus suis.*

There is however a serious pitfall in expression of heterologous genes in recombinant carriers: it is known that several proteins are toxic if they are expressed in heterologous bacteria. Therefore, genes encoding such proteins can never be introduced in heterologous carriers, since successful recombinants will eventually die as a result of the expression a certain amount of the heterologous gene. The P2-protein of *Haemophilus influenzae,* to name just one example, can simply not be expressed in *E. coli.* (Munson et al., Infect. & Immun. 57: 88-94 (1989)). It is one of the objectives of the present invention to offer a recombinant live carrier that does not have this drawback. It was unexpectedly found, that although the *apxIV* gene is efficiently expressed *in vivo (see Example 5),* it is not expressed *in vitro* (see Example 4). This was concluded from the failure to show the presence of ApxIV toxin in *in vitro* grown *A. pleuropneumoniae* cultures. This means that ApxlV expression is switched on or off, depending on the environment in which *A. pleuropneumoniae* is grown. This feature offers an unexpected advantage over known live recombinant carriers: if the expression of the heterologous gene is brought under the control of the *apxIV* promoter, the live attenuated P. pleuropneumoniae carrier according to the invention can be grown in vitro to high densities, regardless the inserted heterologous gene, since the foreign gene will not be expressed under these conditions. After administering a number of bacteria to the host, the expression of the heterologous gene will start and at some time during replication or after the death of the bacterium it will become available to the immune system of the host. The heterologous gene to be expressed can be functionally linked to the *apxIV* promoter by e.g. replacing the coding sequence of the apxIV gene by the coding region of the heterologous gene. It is not necessary to replace the whole *apxIV* gene: it suffices to replace the ATG-codon of ApxlV by the coding region of the heterologous gene including its stop-codon. It is also possible to express a heterologous gene under the influence of the *apxlV* promoter by making a fusion construct. This can be made by inserting the heterologous gene in frame with the *apxIV* reading frame downstream of the *apxIV* ATG codon.
The wording "functionally linked to the *apxIV* promoter" means that transcription of the heterologous gene starts at the apxlV promoter.
It goes without saying that each location of the inserted heterologous gene in which it is functionally linked to the *apxIV* promotor falls within the scope of the invention.
Therefore, the most preferred form of this embodiment relates to live attenuated bacteria according to the present invention, carrying a heterologous gene that is functionally linked to the promotor region of the *apxIV* gene.

The surprising finding that the native *apxIV* promotor is a switchable promotor that is switched off *in vitro* and switched on *in vivo* makes this promotor a very versatile expression tool both in it's natural host and as a heterologous promotor in other bacteria. When used as a heterologous promotor in other bacteria, the DNA comprising the promotor can be isolated from its host and transferred to a bacterium other than *Actinobacillus pleuropneumoniae.* Another option that has now become feasible, is the cloning of several copies of the *apxIV* promotor each controlling the expression of another gene. This can be done in the host bacterium *Actinobacillus pleuropneumoniae,* but this principle of multiple copies is equally applicable to other bacteria. As mentioned above, the promotor can be used for the selective *in vivo* expression of one or more heterologous genes encoding antigens selected from other pathogenic micro-organisms or viruses. The promotor can also be used for the expression of a heterologous DNA sequence encoding a cytokine such as an interleukin, Tumor Necrosis Factor or an interferon. Several cytokines, e.g. interferons are known to play an important role as immune modulators. Thus it may be advantageous to express such genetic information under the control of the *apxIV* promotor. Therefore, another embodiment of the invention relates to a nucleotide sequence harbouring the promotor controling the expression of the *apxIV* gene.
The switchable promotor that in the native situation controls the expression of the *apxIV* gene, was now found to be located in the DNA fragment between position 451 and 1132 of SEQ ID NO: 5. It is clear, that those parts of this DNA fragment that are not essential promotor elements need not necessarily be present in the fragment. Thus, shorter fragments of this DNA fragment in which the promotor activity is retained, are equally suitable for the expression of heterologous genes. Therefore, a more preferred form of this embodiment relates to a nucleotide sequence comprising the DNA fragment from position 451 to 1132 of SEQ ID NO: 5 or a subfragment thereof still having promotor activity.

Bacterial promotors all share two consensus regions, the so-called -10 and the -35 region. Although the flanking sequence of these consensus regions may to a certain extend influence the efficiency of the promotor, it can be advantageous to use only that part of the promotor region that comprises the DNA fragment between -35 and the ATG codon. This DNA fragment is located between position 617 and position 641 of SEQ ID NO: 5. Therefore, in a more preferred form of this embodiment the invention relates to a nucleotide sequence comprising the DNA fragment from position 617 to 641 of SEQ ID NO: 5.

The present invention also relates to ApxlV toxin as a subunit vaccine component.

Subunit vaccines will most probably comprise the three known Apx-toxins. This was mentioned above. Since it was unexpectedly found, that the ApxlV toxin is however present in all *A. pleuropneumoniae* serotypes as mentioned above, it is a desirable additional component of subunit vaccines: neutralising antibodies raised against the ApxlV toxin provide protection against the ApxIV toxin produced by each and every *Actinobacillus pleuropneumoniae* strain, regardless the serotype. Therefore, another embodiment of the invention relates to subunit vaccines for the protection of animals against infection with a bacterium of the species *Actinobacillus pleuropneumoniae,* that comprise purified ApxIV toxin. The ApxlV toxin can be administered alone, or in combination with any or all of the toxins Apxl, -II and -III mentioned above and/or e.g. in combination with Outer Membrane Proteins (OMPs) of *Actinobacillus pleuropneumoniae.* Such vaccines can easily be prepared by admixing ApxlV toxin in an amount sufficient to induce an immune response, and a pharmaceutically acceptable carrier. Production of the ApxlV toxin is possible by introducing the *apxIV* gene in a suitable expression vector, expression of the gene and isolation of the toxin. Many versatile expression systems are known in the art, such as bacterial expression systems, baculovirus expression systems and mammalian cell expression systems. In Example 3 it is described how to obtain the ApxlV toxin by expression of the gene in E. coli.

Still another embodiment of the invention relates to live attenuated vaccines comprising live attenuated bacteria as described above for the protection of animals against infection with a bacterium of the species *Actinobacillus pleuropneumoniae.* Such vaccines can be obtained by admixing live attenuated bacteria with a pharmaceutically acceptable carrier. These vaccines comprise at least an immunogenically effective amount of the live attenuated producing bacterium according to the invention. Immunogenically effective means that the amount of live attenuated bacterium administered at the moment of vaccination is sufficient to induce in the host an effective immune response to virulent forms of the RTX-toxin producing bacterium. The useful dosage to be administered will vary depending on the age, weight and animal vaccinated, the mode of administration and the type of pathogen against which vaccination is sought. The vaccine may comprise any dose of bacteria, sufficient to evoke an immune response. Doses ranging between 10³ and 10¹⁰ bacteria are e.g. very suitable doses.

The pharmaceutically acceptable carrier may be as simple as water, but it may e.g. also comprise culture fluid in which the bacteria were cultured. Another suitable carrier is e.g. a solution of physiological salt concentration. Other examples of pharmaceutically acceptable carriers or diluents useful in the present invention include stabilisers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

Optionally, one or more compounds having adjuvant activity may be added to the vaccine. Adjuvantia are non-specific stimulators of the immune system. They enhance the immune response of the host to the invading pathogen. Examples of adjuvantia known in the art are Freunds Complete and Incomplete adjuvans, vitamin E, non-ionic block polymers, muramyldipeptides, ISCOMs (immune stimulating complexes, cf. for instance European Patent EP 109942), Saponins, mineral oil, vegetable oil, and Carbopol (a homopolymer). Adjuvantia, specially suitable for mucosal application are e.g. the E. coli heat-labile toxin (LT) or Cholera toxin (CT).
Other suitable adjuvants are for example aluminium hydroxide, phosphate or oxide, oil-emulsions (e.g. of Bayol F (^{R}) or Marcol 52 (^{R}), saponins or vitamin-E solubilisate.

Therefore, in a preferred form, the vaccines according to the present invention comprise an adjuvant.

For administration to animals, the vaccine according to the presentation can be given inter alia intranasally, intradermally, subcutaneously, by aerosol or intramuscularly.

There are several ways to store both subunits and live organisms. Storage in a refrigerator is e.g. a well-known method. Also often used is storage at -70°C in a buffer containing glycerol. Bacteria can also be kept in liquid nitrogen. Freeze-drying is another way of conservation. Freeze-dried bacteria can be stored and kept viable for many years. Storage temperatures for freeze-dried bacteria may well be above zero degrees, without being detrimental to the viability. Freeze-drying is equally applicable for subunits.
Freeze-drying can be done according to all well-known standard freeze-drying procedures. Optional beneficial additives, such as e.g. skimmed milk, trehalose, gelatin or bovine serum albumin can be added in the freeze-drying process.
Therefore, in a more preferred embodiment, the vaccine according to the present invention is in a freeze-dried form.

In an even more preferred form of this embodiment, the vaccine additionally comprises one or more antigens selected from other pathogenic micro-organisms or viruses. Such a vaccine can be obtained by adding one or more antigens selected from other pathogenic bacteria or viruses to the live attenuated bacterium according to the invention and a pharmaceutically acceptable carrier as described above.
Of course, it is possible to add not only one or more antigens, but also one or more of the whole pathogens as such, in an inactivated or live form.
It can alternatively be obtained by cloning the genetic information encoding one or more antigens selected from other pathogenic micro-organisms or viruses into the live attenuated bacterium, using known recombinant DNA technology as described above.
Such vaccines are of course less stressing for the animal to be vaccinated than separate vaccinations with each of the pathogens, both from a medical and a physical point of view.

In a still even more preferred form, these antigens are selected from the group consisting of Porcine Reproductive Respiratory Syndrome (PRRS) virus, Pseudo-rabies virus, Porcine Influenza virus, Porcine Parvovirus, Transmissible Gastroenteritis virus, rotavirus, *Escherichia coli, Erysipelothrix rhusiopathiae, Pasteurella multocida, Bordetella bronchiseptica, Haemophilus parasuis* and *Streptococcus suis.*

The invention also relates to methods for the preparation of a live attenuated bacterium of the species *Actinobacillus pleuropneumoniae* that is not capable of producing a functional ApxlV toxin. These method comprise the introduction of a mutation in the gene encoding the apxlV protein. Both classical mutation techniques, using mutagenic agents, and recombinant DNA techniques well-known in the art for insertion, replacement or deletion of genetic information from the apxlV gene are applicable.

In a preferred form, the above mentioned methods are used for the introduction of a deletion.

Methods for the preparation of a live attenuated vaccine according to the invention, that comprise admixing bacteria according to the invention with a pharmaceutically acceptable carrier are also part of the invention.

Also falling within the scope of the invention are methods for the preparation of a subunit vaccine. Such methods comprise the mixing of purified ApxlV toxin with a pharmaceutically acceptable carrier.

Another generally acknowledged problem in the field of vaccination with live vaccines is the following: the presence of antibodies against a certain pathogen in the serum of a host animal indicates that the host has been infected with the pathogen, either in a virulent or attenuated form. It is however impossible to discriminate between field-infected animals and animals vaccinated with a live vaccine strain. The live attenuated *Actinobacillus pleuropneumoniae* according to the present invention offers a solution to this problem as follows:
As described in Example 3, the *apxlV* gene of *Actinobacillus pleuropneumoniae* serotype 1 has been isolated and expressed in a heterologous host cell. This expression product was subjected to PAAGE-gelelectroforesis and then used for Westem-blotting. The blots were incubated with convalescent serum obtained from a deliberately *Actinobacillus pleuropneumoniae*-infected pigs, and sera from field-strains. It was found, that the *apxIV* gene is expressed *in vivo* in all *Actinobacillus pleuropneumoniae* field strains tested. This implicates, that pigs infected with *Actinobacillus pleuropneumoniae* will always have antibodies against the strain with which they were infected, regardless the serotype of the infectious strain.
The live attenuated bacteria according to the present invention can, due to the deletion of the *apxIV* gene, no longer make ApxIV toxin. Therefore animals vaccinated with a live attenuated *Actinobacillus pleuropneumoniae* strain according to the invention will not have antibodies against ApxlV toxin in their serum.
In a comparative test, e.g. an ELISA test, such sera will therefore react with all immunogenic *Actinobacillus pleuropneumoniae-*proteins such as e.g. ApxI, II and/or III, but not with ApxIV. Sera from pigs infected with an *Actinobacillus pleuropneumoniae* field strain however will react with all immunogenic *Actinobacillus pleuropneumoniae-*proteins, including ApxIV. Therefore, the live attenuated *Actinobacillus pleuropneumoniae* according to the present invention tums out to be a very suitable marker vaccine, i.e. a vaccine strain that can be discriminated from a field strain.
A diagnostic test for the discrimination between vaccine strains and field strains can be a simple ELISA-test in which purified ApxlV toxin is coated to the wall of the wells of an ELISA-plate. Incubation with serum from pigs to be tested, followed by e.g. incubation with a labelled anti-pig antibody can then reveal the presence or absence of antibodies against ApxlV toxin.
Another example of a diagnostic test system is e.g. the incubation of a Westem blot comprising purified ApxlV toxin with serum of pigs to be tested, followed by detection of specific anti-ApxlV antibodies.
Therefore, diagnostic test for the discrimination between sera from pigs infected with *Actinobacillus pleuropneumoniae* field strains and from pigs vaccinated with a vaccine comprising live attenuated vaccine *Actinobacillus pleuropneumoniae* strains according to the invention, that comprise purified ApxlV toxin. also fall within the scope of the invention.

Still another problem seen in pig health care is the following: It is difficult to determine in a both quick and unambiguous manner if a pig is infected with *Actinobacillus pleuropneumoniae* or *A. suis,* or possibly a combination of both. Diagnostic tests for the specific detection of A. suis are currently not available. This is mainly due to the fact that A. pleuropneumoniae and A. suis share so many antigens. As an example may serve, that two highly antigenic Apx-toxins; Apxl and Apxll have highly conserved homologues in e.g. A. suis (Van Ostaayen et al., submitted for publication).
The known RTX-genes, encoding the Apxl, -II and -III toxins or homologues are found in practically all members of the genus *Actinobacillus,* such as *A. pleuropneumoniae, A. suis, A. rossii* and *A. equuli.* Thus, it was initially assumed by the inventors, that the new RTX-toxin ApxIV would also be common to all members of the genus *Actinobacillus.*
It was however found after testing a the swine-pathogenic *Actinobacillus,* again surprisingly in contrast with the known three RTX-genes, that this novel RTX-gene *apxIV* is only present in the swine-pathogen *Actinobacillus pleuropneumoniae.* It is absent in all other common swine pathogenic *Actinobacillus* species, and therefore it is also absent in *Actinobacillus suis.* See Example 6 and 7.
Therefore, it was surprisingly noticed that the presence of antibodies against ApxlV in the serum of a pig is a quick and unambiguous proof that the pig has been infected with A. pleuropneumoniae, and not with A. suis or any other swine-pathogen *Actinobacillus* species.
Thus the present invention also provides a diagnostic test based on the presence or absence of antibodies against ApxlV, and therefore a discriminating test for specifically distinguishing an infection with *A. pleuropneumoniae* from an infection with *A. suis* Such a test can e.g. be an ELISA test that comprises in separate wells the ApxI and -II toxins, present in both *A. pleuropneumoniae* and *A. suis* and the purified ApxIV toxin. Serum from A. suis-infected animals will react only with the wells comprising the Apxl and -II whereas *A. pleuropneumoniae-infected* animals will also react with the well comprising the purified ApxlV toxin.

### Example 1:

### Cloning and analysis of the apxIV gene of A. pleuropneumoniae serotype 1.

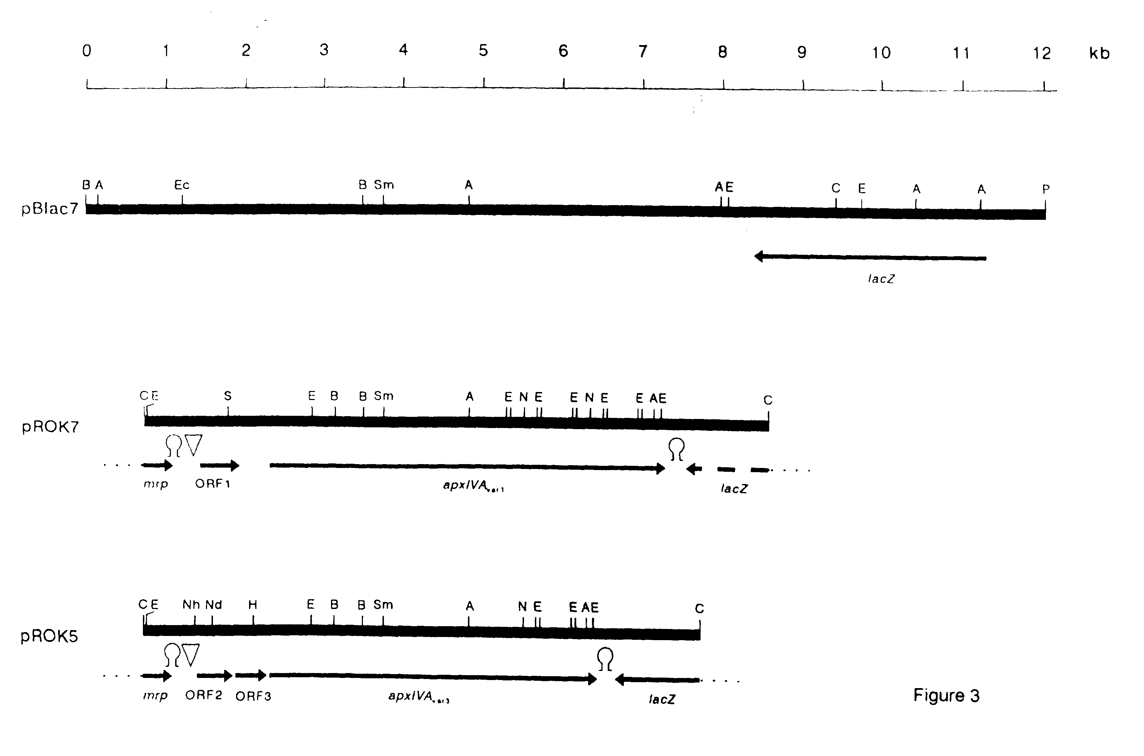

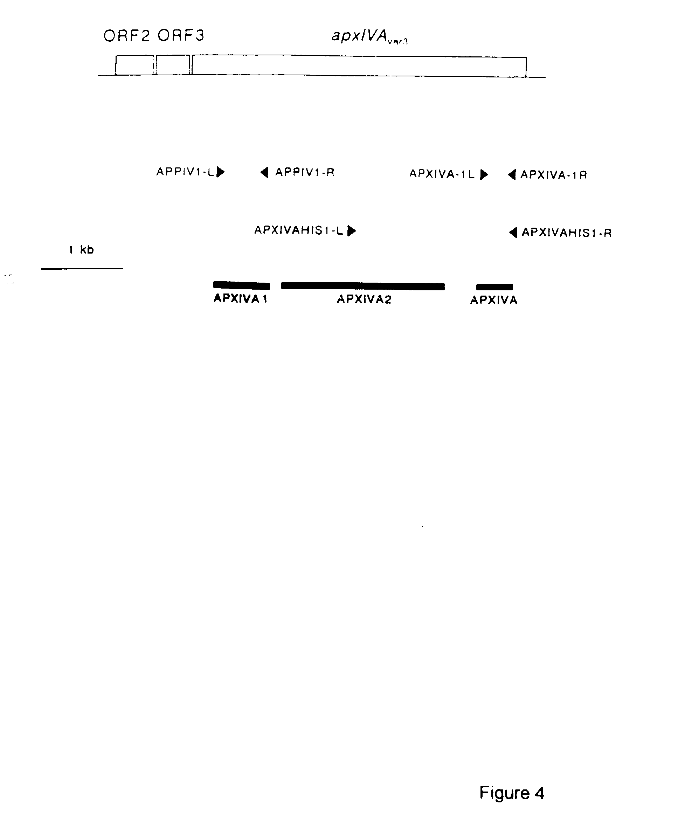

Standard molecular biological procedures (plasmid DNA isolation, restriction digestion, agarose gel electrophoresis, Southem blotting, ligation, transformation, electroporation) were, unless stated otherwise, essentially performed as described in Sambrook et al. (Molecular Cloning, A Laboratory Manual, Cold Spring Harbor, N.Y., 1989) or Ausubel et al., (Current Protocols in Molecular Biology. John Wiley & Sons, N.Y., 1987). PCR was performed essentially as described in Innis et al., (PCR protocols, A guide to Methods and Applications, Academic Press Inc., San Diego, 1990). Chromosomal DNA isolation was performed according to Pitcher et al., (Lett. Appl. Microbiol., 8;151-156, 1989). The origin of all *A. pleuropneumoniae* reference strains (serotype 1 : strain 4074; serotype 2: strain S1536: serotype 3: S1421; serotype 4 M62; serotype 5a: K17; serotype 5b: L20; serotype 6: fem⌀; serotype 7: WF83; serotype 8: 405; serotype 9: CVI13261; serotype 10: 13039; serotype 11: 56153 and serotype 12: 8329) is described by Frey and Nicolet, (J. Clin. Microbiol., 28;232-236, 1990). *A. pleuropneumoniae* serotype 3 strain HV114 is a field isolate (i.e. one of the serotype 3 strains tested in Beck et al., J. Clin. Microbiol., 32;2749-2754, 1994). Other *Actinobacillus* strains used; *A. rossii:* ATCC 27072; *A. equuli:* ATCC 19392; *A. suis:* ATCC 15558. *Pasteurella haemolytica* type 1 strain ATCC 14003 was used.
*E. coli* host strains used: XL1-blue (Stratagene, La Yolla, Ca; genotype: *recA 1 endA 1 gyrA96 thi*- *1 hsdR17 supE44 relA 1 lac* [F' *proAB lacl*^{*q*} *ZDM15* Tn10 (Tet^{r})]) and HMS174(DE3) (AMS Biotechnology Ltd, Switzerland; genotype: F⁻ *recA (r*_{*K12-*} *m*_{*K12+*}*)* rif^{r} IDE3).
On the basis of the preliminary sequence data obtained from the thesis of T.J. Anderson (University of Guelph, 1995), two primers, designated APXIVA-1L (5'-TGGCACTGACGGTGATGA-3') and APXIVA-1R (5'-GGCCATCGACTCAACCAT-3'), were synthesised. These primers were used in a PCR amplification, with chromosomal DNA from *A*. *pleuropneumoniae* serotype 3 strain HV114 and serotype 1 reference strain 4074 as a template. With both strains a fragment of 442 bp was amplified. The fragment derived from the serotype 3 chromosomal DNA was labelled with Digoxigenin-11-dUTP (Boehringer Mannheim) according to the protocol of the manufacturer (this fragment was designated probe APXIVA, see fig. 4). The labelled probe was subsequently used to hybridize a Southem blot containing *Clal* digested chromosomal DNA from strain 4074. The probe hybridised with a fragment of approximately 8.0 kb. The *apxIV* gene from serotype 1 strain 4074 was isolated by ligating *Cla*I digested chromosomal DNA into *Clal* digested pBluescript II SK⁻ (Stratagene USA). *E. coli* strain XL1-blue was transformed with the ligated DNA and transformants were selected on an LB plate with 100 mg/ml of ampicillin. Clones harbouring the *apxIV* were selected by colony hybridisation of a nitrocellulose replica of the plate with the APXIVA probe. Thus, a plasmid designated pROK7 was isolated and shown to harbour a *Cla*I insert of approximately 8 kb. The first 6736 bp of the *Cla*l insert were sequenced (SEQID 1 ) and an open reading frame of 4971 nucleotides was identified encoding a protein of 1657 amino acid residues (SEQID 2) with a predicted size of approximately 186 kD. The gene was designated *apxIV*_ *var1* (see fig. 3).

### Example 2:

### Cloning and analysis of the apxIV gene of A. pleuropneumoniae serotype 3.

The labelled probe APXIVA (mentioned in example 1) was used to hybridize a Southem blot containing *Cla*I digested chromosomal DNA from strain HV114. The probe hybridised with a fragment of approximately 7.0 kb. The isolated chromosomal DNA from HV114 was digested with *Cla*I*,* and ligated with *Cla*I digested pBluescript II SK⁻(Stratagene USA). *E. coli* strain XL1-blue was transformed with the ligated DNA and transformants were selected on an LB plate with 100 mg/ml of ampicillin. Clones harbouring the *apxIV* were selected by colony hybridisation of a nitrocellulose replica of the plate with the APXIVA probe. Thus, a plasmid designated pROK5 was isolated and shown to harbour a *Cla*l insert of approximately 7 kb. The insert was analysed by sequence analysis (SEQID 3). An open reading frame of 4146 bp was identified encoding a protein of 1382 amino acid residues (SEQID 4), with a predicted size of approximately 154 kD. The gene was designated *apxIV_var3 (see* fig. 3) .

### Example 3:

### EXPRESSION OF ApxIV var3-polyhistidine fusion proteins in E. coli

From plasmid pROK5, a deletion clone was made which contains the 3' end of the *apxlV* gene, starting at the *Bam*HI site (nucleotide No. 2747 in SEQ ID No: 3) up to the *Clal* site at the end of the insert downstream of the *apxIV* gene. This plasmid was designated pROK1. Using oligonucleotides APXIVAHIS1-L (5'-AGCCATATGGGCGAnTAAAnTCAG-3') and APXIVAHIS1-R (5'-TATGGATCCTCCGTGCTTCTGAGC-3') and DNA from plasmid pROK1 as a template, a DNA fragment of 2.1 kb was amplified (see fig. 4) containing the region from bp 3520 to 5643 in *apxIV*_ *var3* (SEQID 3) flanked with *Nde*l and *Bam*HI restriction sites at the 5' and 3' end respectively. After cloning of the *Nde*I*/Bam*Hl digested PCR fragment in expression vector pETHIS-1, digested with the same enzymes, a plasmid designated pJFFapxIV6/10his-1 was obtained. Plasmid pETHIS-1 is a derivative of pET14b (Novagen Inc., Madison, Wi.) where the multiple cloning site has been extended and a region encoding a histidine decamer has been inserted. Consequently, The pJFFapxlV6/10his-1 plasmid contains a translational fusion encoding a histidine hexamer, followed by amino acid residues 653 up to 1360 from SEQID 4, followed by a histidine decamer, under the control of a T7 promoter. The plasmid was transferred to *E. coli* strain HMS174(DE3) with pLysS, which contains an IPTG inducible T7 RNA polymerase gene as well as the T7 lysozyme gene for increased stability. The strain was grown in LB medium containing 25 mg/ml of chloramphenicol and 100 mg/ml of ampicillin, up to an OD₆₅₀ of 0.5, and induced with isopropyl-b-D-thiogalactopyranoside at a concentration of 10 mM. After the addition of IPTG, the cells were incubated at 37°C for 2.5 hours, the cells were harvested by centrifugation, and fusion protein with the expected size of 80 kD was isolated in the form of inclusion bodies. The inclusion bodies were solubilized in a solution of 6M guanidine hydrochloride, 300 mM NaCl and 50 mM NaH₂PO₄ at pH 8.0 and the 80 kD fusion protein was further purified by Immobilised Metal Affinity Chromatography (IMAC) (Schmitt et al., Molecular Biology Reports 18;223-230, 1993) using Ni²⁺ chelated columns (Qiagen AG, Basel). Pure protein was eluted from the column at pH 5.0. Pooled fractions were dialysed against a solution of 300 mM NaCl and 50 mM NaH₂PO₄ at pH 8.0. A rabbit was immunised with 500 mg of the polyhistidine fusion product, mixed with 1 volume of Complete Freunds Adjuvant (Difco Labs, Detroit, Ml). A booster dose of the same amount, mixed with incomplete Fre-und Adjuvant was given 3 weeks later. Four weeks after the booster, the rabbit was bled and a hyperimmune serum comprising anti-ApxlV toxin antibodies , designated serum 522-409, was obtained.

### Example 4:

### Expression of apxIV genes in in vitro grown A. pleuropneumoniae

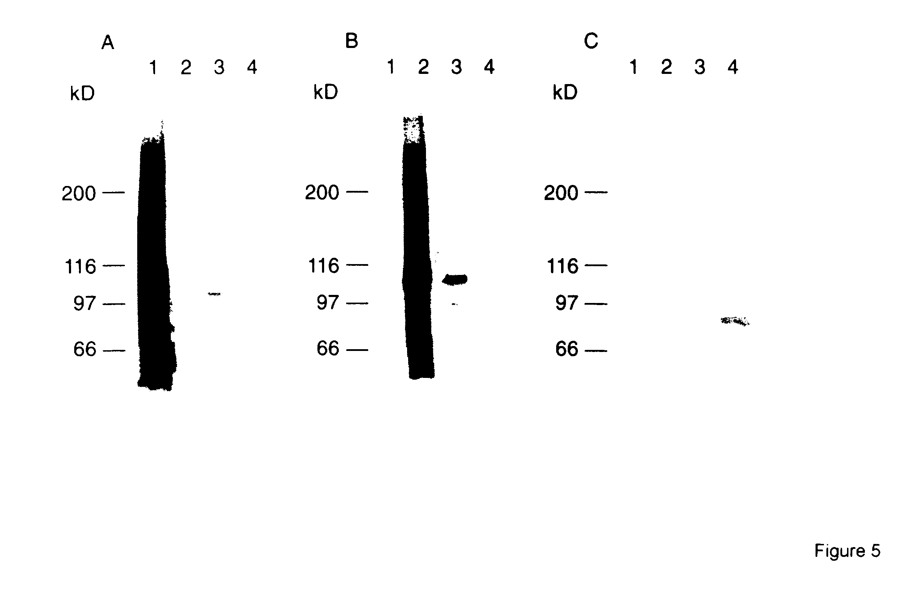

The *A. pleuropneumoniae* reference strain from serotype 1 was grown in Columbia broth supplemented with 10 mg/ml of b-NAD and harvested as described (Beck et al., J. Clin. Microbiol., 32;2749-2754, 1994). Adjacent to lanes comprising ApxIA, ApxIIA and ApxIVA-polyhistidine fusion proteins the concentrated culture supematant was separated by polyacrylamide gel electrophoresis (Laemmli, Nature 227:680-685, 1970) and subjected to a Westem blotting procedure (Towbin et al., Proc. Natl. Acad. Sci. USA 76:4350-4354, 1979). The Westem blot was reacted with anti-ApxlA- and anti-ApxllA monoclonal antibodies as described by Beck et al., (J. Clin. Microbiol., 32;27492754, 1994), and with anti-ApxlV serum 522-409 (see example 3). The isolated RTX toxin fraction of serotype 1 clearly contains ApxlA and ApxllA. The presence of ApxlVA could not be demonstrated (see fig. 5).

### Example 5:

### Expression of apxIV genes in A. pleuropneumoniae in vivo during infection

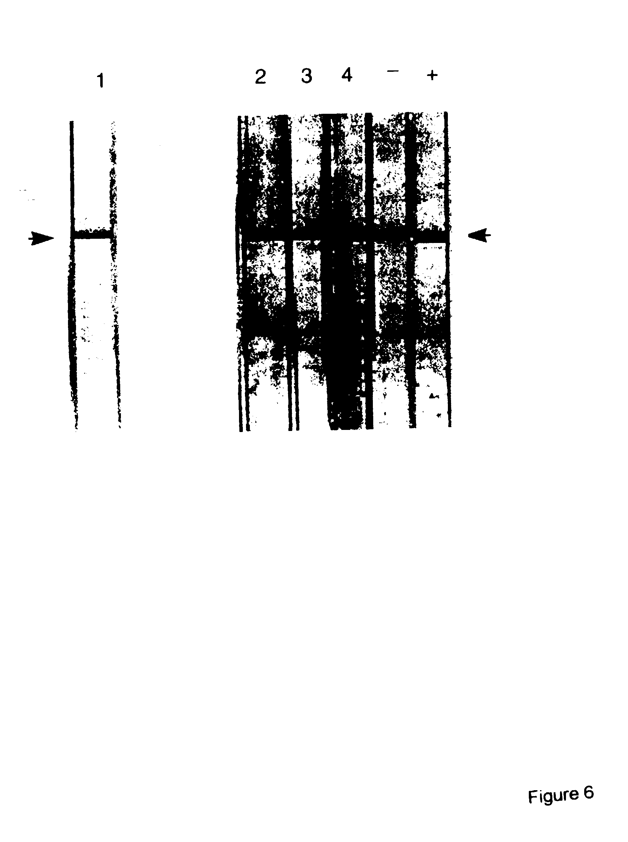

A polyacrylamide gel containing the 80 kD polyhistidine-ApxIV_var3 fusion protein (see example 3) was transferred to a nitrocellulose membrane. The membrane was divided into strips which were reacted with (100-fold dilutions of) convalescent field sera against sero-type 1 or sera from a pig, expenmentally infected with the serotype 1 reference strain (Frey and Nicolet, Vet. Microbiol., 28;61-73, 1991). The reaction was visualised using alkaline phosphatase-labelled conjugate against rabbit IgG (Kirkegaard Perry Inc., Gaithersburg, Md.) and NBT (4-Nitrobluetetrazolium chloride) and BCIP (5-Bromo-4-chloro-3-indolyl-phosphate) colour development (see fig. 6). The serotype 1 field sera, as well as serum from the experimentally infected pig react with the 80 kD polyhistidine-ApxIV_var3 protein. This indicates that the ApxlV protein actually is expressed, is antigenic and induces anti-ApxIV toxin antibodies during *A. pleuropneumoniae* infection in pigs.

### Example 6:

### Presence of apxIV genes in all A. pleuropneumoniae serotypes and the absence thereof in non-pleuropneumoniae Actinobacillus-strains using Southern blotting

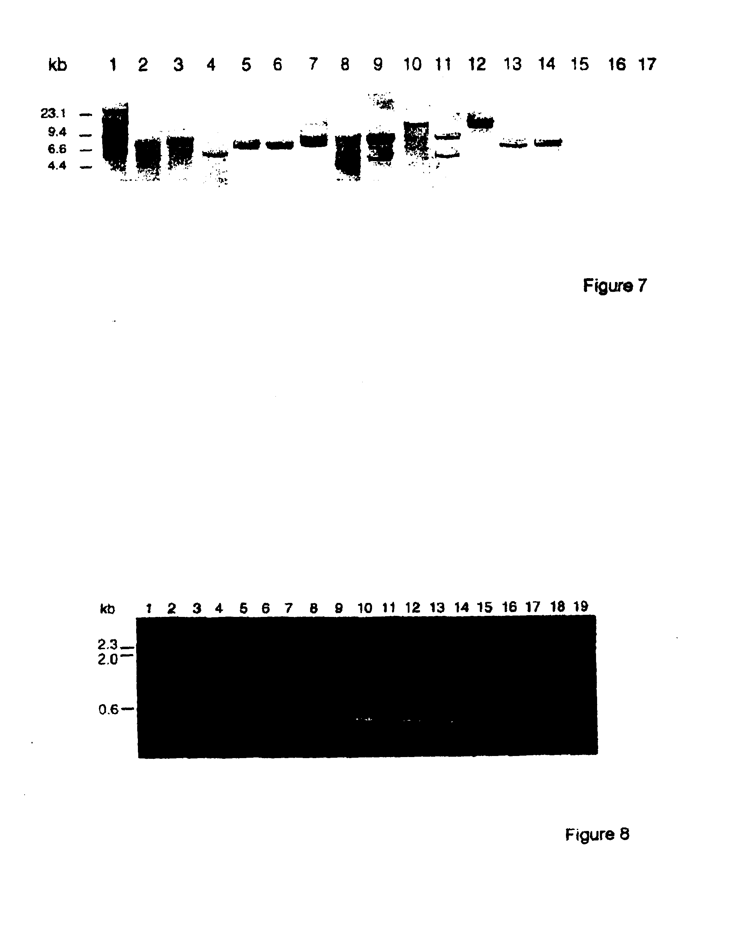

To investigate the presence of the *apxlV* gene in the various *A.pleuropneumoniae* serotypes and related bacteria, three probes were made (see fig. 4). Probe APXIVA is described in example 1. Probe APXIVA2 contains the 2015 bp DNA fragment between the *Bam*HI and *Nru*l sites. The 758 bp probe APPIVA1 was made by PCR amplification with oligos APPIV1-L (5'-GGGACAGTGGCTCAATTAAG-3') and (APPIV1-R (5'-AGCTGTAAACTCCACCAACG-3'). All probes were labelled with Digoxigenin-11-dUTP (Boehringer Mannheim) according to the protocol of the manufacturer and hybridised with Southem blots containing *CIa*l digested chromosomal DNA of all *A. pleuropneumoniae* reference strains and the HV114 field strain, *Actinobacillus suis* (ATCC 15558), *Actinobacillus rossii* (ATCC 27072) and *Actinobacillus equuli* (ATCC 19392). All three probes react similarly (see fig. 7 for the results with the APXIVA2 probe). All *A. pleuropneumoniae* strains react, whereas no hybridisation is observed with the *A. suis, A. equuli* and *A. rossii* strains.

### Example 7:

### Presence of apxIV genes in A. pleuropneumoniae and related strains using PCR amplification

With 50 ng of chromosomal DNA from the various *A. pleuropneumoniae* serotypes, other *Actinobacillus* species and *P. haemolytica* as templates, and primers APXIVA-1L (5'-TGGCACTGACGGTGATGA-3') and APXIVA-1R (5'-GGCCATCGACTCAACCAT-3') PCR amplification was performed. After analysis of the products on an agarose gel, products with the expected size of 442 bp were observed in all *A. pleuropneumoniae* amples, but in none of the other *Actinobacillus* species (fig. 8). This indicates that in addition to the results in example 6, also PCR could be used to discriminate *A. pleuropneumoniae* from other *Actinobacillus* species.

### Example 8:

### Overexpression of ApxIV-var1 polyhistidine fusion protein.

Starting with plasmid pROK-7 (see example 1) as a template and oligonucleotides APX4/II5-L (5'-CGCCATATGACAAAATTAACTATGCAAG) and APX4II6-R (5'-CGCGAAT TCAGCGACACAAGAGATAT) as PCR-primers, a PCR fragment was amplified. A sufficient amount of this fragment was then digested with restriction enzymes Ndel and EcoRI and cloned in expression vector pETHIS-1, digested with the same enzymes as described in Example 3. From the resulting plasmid, designated pJFFApxIVA1His1, a 206 kD polyhistidine fusion protein (MW determined in PAGE) of 1841 amino acid residues was overexpressed in E. coli as described in Example 3. The protein is encoded in the coding region spanning nucleic acid no. 1132 to 6546 as depicted in SEQ ID NO: 5. The amino acid sequence of the protein in given in SEQ ID NO: 6. In Westem blot this product was shown to react with specific anti-ApxlV serum 522-409 (antiserum described in example 3).

### Example 9:

### Protection of mice by vaccination with ApxIV against APP challenge.

The 206 kD polyhistidine-ApxlV fusion protein as described in Example 8 and a comparable 108 kD polyhistidine-ApxlA-fusion protein, both derived from serotype 1 reference strain 4074 genomic material, were overexpressed as described in example 3. The cell pellet of induced E. coli cells was resuspended into PBS buffer (1 g. cell pellet in 6 ml buffer) and sonicated on ice for two times 45 seconds on ice for lysis of the cells. After centrifugation for 20 minutes at 22.000 x g at 4°C, the supematant was discarded and the resulting pellet was washed with a solution of 3M urea (pH 6.3). The urea was removed after centrifugation for 20 minutes at 22.000 x g, and the resulting pellet was solubilized in 6M GuanidiniumHCl (pH 8.0). The protein samples were standardised by specific protein content after densitometry of PAGE gels. The samples were diluted with PBS and formulated with an oil adjuvant.

Three groups of 15 mice each, were intraperitoneally immunised with the ApxlV antigen (36.3 microgram), ApxIA antigen (36.3 microgram), or the adjuvant alone. The vaccines were administered in a volume of 0.4 ml. Twenty-four days after the first vaccination, each group of mice was split in two groups of 7 and 8 mice which were boosted with half the amount of antigen. The groups of 7 mice were boosted intraperitoneally in a volume of 0.2 ml and the groups of 8 mice were vaccinated intramuscularly with 0. 1 ml in each hind leg. Thirteen days after the booster, the mice were challenged intraperitoneally with 1.5 10⁸ cfu of a virulent serotype 1 strain .

### Example 10:

### Poreforming capacity of ApxIV:

Freshly induced E. coli cells expressing ApxlV were used as the source of protein for testing poreformation in artificial lipid bilayers as described by Maier et al., Infect. Immun., 64; 4415-4423(1996). The methods used for black lipid bilayer experiments have been described previously (Benz et al.; Biochim. Biophys. Acta 511: 305-319 (1978)). Membranes were formed from a 1% solution of asolectin (soybean lecitin type IV-S from Sigma, St. Louis MO) in n-decane. Zero current membrane potentials were measured with a Keithley 610 C electrometer 5-10 min. after a 10-fold salt gradient was established across the membranes (Benz et al.; Biochim. Biophys. Acta 551: 238-247 (1979)). The presence of ApxlV resulted in a high frequency of pore formation in the presence of 0.5 % cholesterol, with an average single channel conductance (G) of 4 nS.
These results indicate that the ApxlV induces pores into artificial bilayers and is therefore toxic to eukaryotic cells and thus is a virulence factor for A. pleuropneumoniae.

### Legend to the figures:

Figure 1: Comparison of ApxlVAvar1 and ApxlVAvar3 (var stands for serotype). Graphic representation of the different features found in the C-terminal end. Dashed boxes represent the direct repeat regions in ApxlVA. Bold vertical bars indicate the position of glycine rich nonapeptides, and DNA polymerase family B signatures are indicated by black triangles. The amino acid sequence YSDSANSKK represents the spacer sequence in the ApxlVAvar3 gene. The sequence segment of ApxlVAvar1 which is deleted in ApxlVAvar3 is also indicated in the figure.

figure 2: Alignment of the amino acid sequences of direct repeat 1 (A), direct repeat 2 (B) and direct repeat 3 (C) of ApxlVAvar1 and ApxlVAvar3. The copies of each of the direct repeats 1, 2 and 3 are labeled by letters to distinguish them for the sequence comparison. Variant residues are shown in bold letters.

Figure 3: Partial restriction maps of pBLac7 (Thesis of T.J. Anderson University of Guelph, 1995), pROK7 and pROK5. The different open reading frames (ORF's) are indicated by arrows. The interrupted arrow of *lacZ* in pROK7 indicates partial sequencing of the gene. Potential *rho*-independent transcription terminators are indicated (W). Potential transcription start sites are indicated by a triangle. Restriction sites: A= *Asp700;* B= *Bam*HI; C= *Cla*l; E= *Eco*RV*;* Ec= *Eco*RI*;* H= *Hin*dIII; N= *Nru*l; Nd= *Nde*l*;* Nh= *Nhe*I*;* P= *Pst*I; S= *Spe*l*;* Sm= *Sma*I*.*

Figure 4: Location of the various oligonucleotides and probes on the map of the *apxIVAvar3* gene.

Figure 5: Expression of ApxlV in *in vitro* cultivated serotype 1 reference strain 4074. Panel A was reacted with the anti-ApxlA monoclonal antibody, panel B with anti-ApxllA monoclonal antibody and panel C was reacted with anti-ApxlVA serum 522-409. Lane 1 contains ApxlA-polyhistidine fusion protein, lane 2 contains ApxllA-polyhistidine fusion protein, lane 3 contains strain 4074 concentrated culture supematant, lane 4 contains ApxlVA polyhistidine fusion protein.

Figure 6: Immunoblot showing the reactivities of sera from pigs which were experimentally infected with the reference strain of serotype 1 (lane 1 ) or pig sera from serotype 1 field infections (lane 2-4) with the 80 kD polyhistidine-ApxlV fusion protein. As a positive control (+), serum 522-409 was used, as a negative control (-), polyclonal rabbit serum against Apxl and Apxll (Frey et al., Infect. Immun., 57;2050-2056, 1989) was used as a 1000-fold dilution.

Figure 7: Southem blot of Clal digested genomic DNA hybridised with probe APXIVA2. Lanes 1-13: *A. pleuropneumoniae* reference strains; 1: serotype 1; 2: serotype 2; 3: serotype 3; 4: serotype 4; 5: serotype 5a; 6: serotype 5b; 7: serotype 6; 8: serotype 7; 9: serotype 8; 10: serotype 9; 11: serotype 10; 12: serotype 11; 13: serotype 12; 14: HV114 field strain; 15: *A. suis* (ATCC 15558); 16: *A. rossii* (ATCC 27072); 17: *A. equuli* (ATCC 19392). Molecular size markers are indicated (in kilobasepairs) on the left.

Figure 8: PCR amplification of *apxlV* using primers APXIVA-1L and APXIVA-1 R. Lane assignments: lanes 1 to 13 contain the *A. pleuropneumoniae* reference strains from serotypes1, 2, 3, 4, 5a, 5b, 6, 7, 8, 9, 10, 11 and 12 respectively; lane 14: strain HV114; lane 15: *A. suis* ATCC 15558; lane 16: *A. rossii ATCC* 27072; lane 17: *A. equuli* ATCC 19392; lane 18: *A. lignieresii* ATCC 49236; lane 19: *P. haemolytica* type 1 ATCC 14003. Molecular size markers (in kilobasepairs) are indicated on the left.

## Claims

1. Live attenuated bacterium of the species *Actinobacillus pleuropneumoniae,* characterised in that said bacterium produces no functional ApxlV toxin.

2. Live attenuated bacterium according to claim 1, characterised in that the gene encoding the ApxlV toxin comprises a mutation.

3. Live attenuated bacterium according to claim 2, characterised in that said mutation is an insertion and/or deletion.

4. Live attenuated bacterium according to claim 3, characterised in that the insertion comprises a heterologous gene.

5. Live attenuated bacterium according to claim 4, characterised in that said heterologous gene encodes one or more antigens selected from the group consisting of Porcine Reproductive Respiratory Syndrome (PRRS) virus, Pseudorabies virus, Porcine Influenza virus, Porcine Parvovirus, Transmissible Gastroenteritis virus, rotavirus, *Escherichia coli, Erysipelothrix rhusiopathiae, Pasteurella multocida, Bordetella bronchiseptica, Haemophilus parasuis* and *Streptococcus suis.*

6. Live attenuated bacterium according to claim 4 or 5, characterised in that said heterologous gene is functionally linked to the promotor region of the *apxIV* gene.

7. Nucleotide sequence harbouring the promotor controling the expression of the *apxIV* gene.

8. Nucleotide sequence according to claim 7, characterised in that it comprises the DNA fragment from position 451 to 1132 of SEQ ID NO: 5 or a subfragment thereof still having promotor activity.

9. Nucleotide sequence according to claim 7, characterised in that it comprises the DNA fragment from position 617 to 641 of SEQ ID NO: 5.

10. Subunit vaccine for the protection of animals against infection with a bacterium of the species *Actinobacillus pleuropneumoniae,* characterised in that said vaccine comprises purified ApxlV toxin and a pharmaceutically acceptable carrier.

11. Live attenuated vaccine for the protection of animals against infection with a bacterium of the species *Actinobacillus pleuropneumoniae,* characterised in that said vaccine comprises a live attenuated bacterium according to claims 1-6 and a pharmaceutically acceptable carrier.

12. Vaccine according to claim 10 or 11, characterised in that it comprises an adjuvant.

13. Vaccine according to claim 10-12, characterised in that the vaccine is in a freeze-dried form.

14. Vaccine according to claims 10-13, characterised in that it additionally comprises one or more antigens from pig-pathogenic micro-organisms or viruses.

15. Vaccine according to claim 14, characterised in that it additionally comprises one or more antigens selected from the group consisting of Porcine Reproductive Respiratory Syndrome (PRRS) virus, Pseudorabies virus, Porcine Influenza virus, Porcine Parvovirus, Transmissible Gastroenteritis virus, rotavirus, *Escherichia coli, Erysipelothrix rhusiopathiae, Pasteurella multocida, Bordetella bronchiseptica, Haemophilus parasuis* and *Streptococcus suis.*

16. Method for the protection of a susceptible animal against *Actinobacillus pleuropneumoniae* infection, said method comprising administering a vaccine according to claims 10-15.

17. Method for the preparation of a live attenuated bacterium of the species *Actinobacillus pleuropneumoniae* producing no functional ApxIV toxin, characterised in that said method comprises the introduction of a mutation in the gene encoding the apxlV protein.

18. Method according to claim 17, characterised in that said mutation is obtained by introducing a deletion

19. Method for the preparation of a live attenuated vaccine according to claims 11-15, said method comprising admixing bacteria according to claims 1-6 with a pharmaceutically acceptable carrier.

20. Method for the preparation of a vaccine according to claim 10, said method comprising admixing purified ApxlV toxin with a pharmaceutically acceptable carrier.

21. Diagnostic test for the discrimination between sera from pigs infected with *Actinobacillus pleuropneumoniae* field strains and from pigs vaccinated with a vaccine comprising live attenuated vaccine *Actinobacillus pleuropneumoniae* strains according to claim 1, said test being characterised in that the test comprises purified ApxlV toxin.

22. Diagnostic test for distinguishing *Actinobacillus pleuropneumoniae* infection in pigs from *A. suis* infection in pigs, characterised in that said test comprises purified ApxIV toxin.


### Annex to the description

SEQUENCE LISTING

(1) GENERAL INFORMATION:

(i) APPLICANT:
(A) NAME: AKZO Nobel N.V.
(B) STREET: Velperweg 76
(C) CITY: Arnhem
(E) COUNTRY: The Netherlands
(F) POSTAL CODE (ZIP): 6824 BM

(ii) TITLE OF INVENTION: Live attenuated Actinobacillus
pleuropneumoniae

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 6736 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Actinobacillus pleuropneumoniae
(B) STRAIN: 4074 (serotype 1 reference strain)

(vii) IMMEDIATE SOURCE:
(B) CLONE: pROK7

(ix) FEATURE:
(A) NAME/KEY: CDS
(B) LOCATION:1576..6549
(D) OTHER INFORMATION:/codon_start= 1576
/function= "RTX toxin"
/product= "ApxIV_var1"
/gene= "apxIV_var1"
/number= 1

(xi) SEQUENCE DESCRIPTION: SEQ ID NO: 1:

ATCGATATGC CGCCGGGTAC GGGCGATATC CAACTTACTC TTTCGCAACA AATTCCGGTT 60

ACCGGTGCGG TGGTGGTAAC CACTCCGCAA GATATTGCGT TATTAGATGC GGTGAAAGGT 120

ATTTCAATGT TCCAAAAAGT GTCGGTACCG GTCTTAGGTA TCATTGAAAA TATGAGCGTA 180

CATATCTGCC AAAATTGCGG TCACCACGAA GATATTTTCG GCACCGGCGG TGCGGAGAAA 240

GTGGCGAAGA AATACGGTAC TAAAGTATTA GGACAAATGC CGTTGCATAT TCGCTTACGT 300

CAAGATTTGG ATGCCGGCAC ACCGACCGTC GTTGCGGCAC CGGAACACGA AACCAGCCGA 360

GCCTATATTG AATTAGCGGC AAAAGTCGCT TCGGAATTAT ACTGGCAAGG TTCGGTTATC 420

CCGTCTGAAA TTATGATTCG TGAAGTAAAA TAAGTTTTAA TAACCACGAA AACACAAAGA 480

ACACAAGCGG TAGAATTTGC AGAAAAATTT GCAAATCCTA CCGCTTTTTT ATTAGTACGA 540

TTCGCTGTTG GACTGCTATT TGATTTGGTT TGTCAGGATA TTATGTTATT GTAATGAAAT 600

GTTAGTGAAT TATTTTTATT AATTTGAAAG GAAACAAAAT GAAAATAAAA AAACGTTACA 660

TTGCGCTGTT GGTCTTAGGT GTCGTTATCA GCTATGCCTG GTATCAAAAT TATCAATGGG 720

AACAGCTGAT GTTAAGCGGT TATTGTGAAA AGGACGGAAG TTATTTTGAT GATAGGCATA 780

CGAAGCAAGA ACTGATTGAT AGGGCAATTA ACTATATGCT GGAGCATCAA TCTAAAAAAA 840

CATACGATGC TTATACTGAT GAACCTTTAG AAATAAAACC ATATTTAACA ATAGAGGAAT 900

TTAAAAAACT CAATCCAAAT TGTTGTGAAA TTACCTCATG GCCAGCAGAT GCAGTTCCAC 960

AAGATTGGGA TGTTCGTGTG GAAGGTAAGG CATATAGGTA TGTAATCGTA AAATATTTAA 1020

GAACCTTAGC AAATAGAGAA CCTGAACGAT GGGAAACTAG TATTGTTTTT GATAATTGCG 1080

GCAATCCTAA AAGAGCAAGC TACTTATATT ATTTAAAGAG AGAAATTTAT TATGACAAAA 1140

TTAACTATGC AAGATGTGAC CAATTTATAT TTATATAAAA CGAAAACTCT ACCTAAAGAT 1200

AGATTGGATG ATTCACTTAT TTCTGAAATA GGAAAAGGAG ATGATGATAT TGATAGAAAA 1260

GAATTTATGG TGGGGCCGGG ACGTTTTGTG ACCGCTGATA ACTTTAGCGT TGTAAGAGAT 1320

TTTTTTAATG CTGGGAAATC ACGCATTATT GCGCCGCAAG TCCCGCCTAT TCGTTCACAG 1380

CAGGAAAAAA TCTTGGTCGG TTTAAAACCG GGCAAATATT CCAAAGCGCA GATATTGGAA 1440

ATGCTGGGTT ATACGAAAGG CGGAGAAGTG GTAAATGGCA TGTTTGCCGG TGAAGTCCAG 1500

ACATTAGGCT TTTATGACGA TGGCAAAGGG GATTTACTCG AACGCGCCTA TATCTGGAAT 1560

ACCACAGGAT TTAAA ATG AGC GAC AAT GCC TTT TTT GTT ATA GAA GAA TCA 1611
Met Ser Asp Asn Ala Phe Phe Val Ile Glu Glu Ser
1               5                   10

GGC AAA CGC TAT ATT GAA AAC TTT GGT ATT GAA CCT CTT GGT AAG CAA 1659
Gly Lys Arg Tyr Ile Glu Asn Phe Gly Ile Glu Pro Leu Gly Lys Gln
        15                  20                  25

GAA GAT TTT GAT TTT GTC GGC GGC TTT TGG TCT AAC TTA GTG AAT CGT 1707
Glu Asp Phe Asp Phe Val Gly Gly Phe Trp Ser Asn Leu Val Asn Arg
    30                  35                  40

GGT TTG GAA AGT ATT ATC GAC CCA TCC GGT ATC GGT GGA ACG GTA AAC 1755
Gly Leu Glu Ser Ile Ile Asp Pro Ser Gly Ile Gly Gly Thr Val Asn
45                  50                  55                  60

CTT AAC TTT ACC GGC GAG GTG GAA ACC TAC ACG TTA GAC GAA ACA AGG 1803
Leu Asn Phe Thr Gly Glu Val Glu Thr Tyr Thr Leu Asp Glu Thr Arg
                65                  70                  75

TTT AAA GCG GAA GCG GCG AAG AAA AGC CAT TGG AGT TTA GTG AAT GCG 1851
Phe Lys Ala Glu Ala Ala Lys Lys Ser His Trp Ser Leu Val Asn Ala
            80                  85                  90

GCG AAA GTA TAC GGC GGT TTA GAC CAA ATT ATT AAA AAA CTA TGG GAC 1899
Ala Lys Val Tyr Gly Gly Leu Asp Gln Ile Ile Lys Lys Leu Trp Asp
        95                  100                 105

AGT GGC TCA ATT AAG CAT TTA TAT CAA GAT AAA GAT ACG GGC AAA TTA 1947
Ser Gly Ser Ile Lys His Leu Tyr Gln Asp Lys Asp Thr Gly Lys Leu
    110                 115                 120

AAA CCG ATT ATT TAC GGC ACG GCC GGC AAC GAC AGT AAG ATT GAA GGC 1995
Lys Pro Ile Ile Tyr Gly Thr Ala Gly Asn Asp Ser Lys Ile Glu Gly
125                 130                 135                 140

ACT AAA ATC ACC CGT AGG ATT GCG GGT AAA GAA GTT ACG CTT GAT ATT 2043
Thr Lys Ile Thr Arg Arg Ile Ala Gly Lys Glu Val Thr Leu Asp Ile
                145                 150                 155

GCC AAT CAG AAA ATT GAA AAA GGC GTG TTA GAG AAA TTG GGG CTG TCT 2091
Ala Asn Gln Lys Ile Glu Lys Gly Val Leu Glu Lys Leu Gly Leu Ser
            160                 165                 170

GTT AGT GGT TCG GAT ATC ATT AAA TTG TTG TTT GGA GCA TTG ACT CCA 2139
Val Ser Gly Ser Asp Ile Ile Lys Leu Leu Phe Gly Ala Leu Thr Pro
        175                 180                 185

ACT TTA AAT AGA ATG TTG CTA TCA CAA CTT ATC CAG TCT TTT TCC GAT 2187
Thr Leu Asn Arg Met Leu Leu Ser Gln Leu Ile Gln Ser Phe Ser Asp
    190                 195                 200

AGC TTG GCT AAA CTT GAT AAT CCC TTA GCC CCT TAC ACT AAA AAT GGC 2235
Ser Leu Ala Lys Leu Asp Asn Pro Leu Ala Pro Tyr Thr Lys Asn Gly
205                 210                 215                 220

GTG GTT TAT GTC ACC GGC AAA GGG AAT GAT GTG CTT AAA GGA ACT GAA 2283
Val Val Tyr Val Thr Gly Lys Gly Asn Asp Val Leu Lys Gly Thr Glu
                225                 230                 235

CAT GAG GAT TTG TTT CTC GGT GGT GAG GGG AAT GAT ACT TAT TAT GCG 2331
His Glu Asp Leu Phe Leu Gly Gly Glu Gly Asn Asp Thr Tyr Tyr Ala
            240                 245                 250

AGA GTA GGC GAT ACA ATT GAA GAC GCC GAC GGC AAA GGT AAA GTC TAT 2379
Arg Val Gly Asp Thr Ile Glu Asp Ala Asp Gly Lys Gly Lys Val Tyr
        255                 260                 265

TTT GTG AGA GAA AAA GGG GTA CCT AAG GCG GAT CCT AAG CGG GTA GAG 2427
Phe Val Arg Glu Lys Gly Val Pro Lys Ala Asp Pro Lys Arg Val Glu
    270                 275                 280

TTT AGC GAG TAC ATA ACG AAA GAA GAA ATA AAA GAG GTT GAA AAG GGG 2475
Phe Ser Glu Tyr Ile Thr Lys Glu Glu Ile Lys Glu Val Glu Lys Gly
285                 290                 295                 300

TTA TTA ACT TAC GCA GTT TTA GAA AAT TAT AAT TGG GAA GAG AAA ACG 2523
Leu Leu Thr Tyr Ala Val Leu Glu Asn Tyr Asn Trp Glu Glu Lys Thr
                305                 310                 315

GCG ACT TTC GCT CAT GCG ACT ATG CTT AAT GAG CTT TTT ACT GAT TAT 2571
Ala Thr Phe Ala His Ala Thr Met Leu Asn Glu Leu Phe Thr Asp Tyr
            320                 325                 330

ACT AAT TAT CGT TAT GAA GTT AAA GGA CTA AAA TTG CCC GCC GTT AAA 2619
Thr Asn Tyr Arg Tyr Glu Val Lys Gly Leu Lys Leu Pro Ala Val Lys
        335                 340                 345

AAG TTA AAA AGT CCG TTG GTG GAG TTT ACA GCT GAT TTA TTA ACT GTT 2667
Lys Leu Lys Ser Pro Leu Val Glu Phe Thr Ala Asp Leu Leu Thr Val
    350                 355                 360

ACG CCT ATT GAC GAA AAC GGA AAA GCA CTT AGC GAA AAA AGT ATT ACG 2715
Thr Pro Ile Asp Glu Asn Gly Lys Ala Leu Ser Glu Lys Ser Ile Thr
365                 370                 375                 380

GTT AAA AAT TTT AAA AAT GGT GAT TTA GGA ATA AGG TTG TTG GAT CCT 2763
Val Lys Asn Phe Lys Asn Gly Asp Leu Gly Ile Arg Leu Leu Asp Pro
                385                 390                 395

AAT AGC TAT TAT TAT TTC CTT GAA GGC CAA GAT ACG GGT TTT TAT GGT 2811
Asn Ser Tyr Tyr Tyr Phe Leu Glu Gly Gln Asp Thr Gly Phe Tyr Gly
            400                 405                 410

CCT GCT TTT TAT ATT GAA CGA AAA AAC GGT GGC GGC GCT AAA AAT AAC 2859
Pro Ala Phe Tyr Ile Glu Arg Lys Asn Gly Gly Gly Ala Lys Asn Asn
        415                 420                 425

TCG TCG GGA GCA GGA AAT AGC AAA GAT TGG GGC GGG AAC GGG CAT GGA 2907
Ser Ser Gly Ala Gly Asn Ser Lys Asp Trp Gly Gly Asn Gly His Gly
    430                 435                 440

AAT CAC CGA AAT AAT GCC TCC GAC CTG AAT AAA CCG GAC GGA AAT AAT 2955
Asn His Arg Asn Asn Ala Ser Asp Leu Asn Lys Pro Asp Gly Asn Asn
445                 450                 455                 460

GGG AAT AAC CAA AAT AAC GGA AGC AAT CAA GAT AAT CAT AGC GAT GTG 3003
Gly Asn Asn Gln Asn Asn Gly Ser Asn Gln Asp Asn His Ser Asp Val
                465                 470                 475

AAT GCG CCA AAT AAC CCG GGA CGT AAC TAT GAT ATT TAC GAT CCT TTA 3051
Asn Ala Pro Asn Asn Pro Gly Arg Asn Tyr Asp Ile Tyr Asp Pro Leu
            480                 485                 490

GCT TTA GAT TTA GAT GGA GAT GGG CTT GAA ACC GTG TCG ATG AAC GGG 3099
Ala Leu Asp Leu Asp Gly Asp Gly Leu Glu Thr Val Ser Met Asn Gly
        495                 500                 505

CGA CAA GGC GCG TTA TTC GAT CAT GAA GGA AAA GGT ATT CGT ACC GCA 3147
Arg Gln Gly Ala Leu Phe Asp His Glu Gly Lys Gly Ile Arg Thr Ala
    510                 515                 520

ACG GGC TGG CTC GCT GCG GAT GAC GGT TTT TTA GTG TTA GAT CGT AAC 3195
Thr Gly Trp Leu Ala Ala Asp Asp Gly Phe Leu Val Leu Asp Arg Asn
525                 530                 535                 540

CAA GAC GGC ATT ATT AAT GAT ATA AGC GAG TTA TTT AGT AAT AAA AAT 3243
Gln Asp Gly Ile Ile Asn Asp Ile Ser Glu Leu Phe Ser Asn Lys Asn
                545                 550                 555

CAA CTT TCC GAC GGC AGT ATT TCT GCA CAC GGT TTT GCG ACA TTA GCC 3291
Gln Leu Ser Asp Gly Ser Ile Ser Ala His Gly Phe Ala Thr Leu Ala
            560                 565                 570

GAT TTG GAT ACA AAC CAA GAT CAG CGT ATC GAC CAA AAT GAT AAG CTG 3339
Asp Leu Asp Thr Asn Gln Asp Gln Arg Ile Asp Gln Asn Asp Lys Leu
        575                 580                 585

TTT TCT AAA CTC CAA ATT TGG CGG GAT TTA AAT CAA AAC GGT TTT AGT 3387
Phe Ser Lys Leu Gln Ile Trp Arg Asp Leu Asn Gln Asn Gly Phe Ser
    590                 595                 600

GAA GCG AAT GAG CTG TTT AGC TTA GAA AGT TTG AAT ATT AAA TCT TTA 3435
Glu Ala Asn Glu Leu Phe Ser Leu Glu Ser Leu Asn Ile Lys Ser Leu
605                 610                 615                 620

CAT ACC GCC TAT GAA GAG CGT AAT GAT TTT CTA GCG GGC AAT AAT ATC 3483
His Thr Ala Tyr Glu Glu Arg Asn Asp Phe Leu Ala Gly Asn Asn Ile
                625                 630                 635

CTT GCT CAG CTT GGG AAG TAT GAA AAA ACG GAC GGT ACT TTT GCA CAA 3531
Leu Ala Gln Leu Gly Lys Tyr Glu Lys Thr Asp Gly Thr Phe Ala Gln
            640                 645                 650

ATG GGC GAT TTA AAT TTC AGT TTT AAC CCG TTT TAT AGC CGA TTT ACC 3579
Met Gly Asp Leu Asn Phe Ser Phe Asn Pro Phe Tyr Ser Arg Phe Thr
        655                 660                 665

GAA GCG TTA AAT TTA ACC GAG CAA CAA CGT CGC ACA ATT AAT CTA ACC 3627
Glu Ala Leu Asn Leu Thr Glu Gln Gln Arg Arg Thr Ile Asn Leu Thr
    670                 675                 680

GGC ACC GGT CGG GTT CGG GAT TTG CGT GAA GCC GCC GCA CTT TCT GAG 3675
Gly Thr Gly Arg Val Arg Asp Leu Arg Glu Ala Ala Ala Leu Ser Glu
685                 690                 695                 700

GAG TTG GCT GCT TTA TTA CAA CAG TAC ACT AAG GCC TCC GAT TTT CAG 3723
Glu Leu Ala Ala Leu Leu Gln Gln Tyr Thr Lys Ala Ser Asp Phe Gln
                705                 710                 715

GCA CAA CGA GAA TTA TTG CCT GCC ATT TTA GAT AAA TGG GCG GCA ACG 3771
Ala Gln Arg Glu Leu Leu Pro Ala Ile Leu Asp Lys Trp Ala Ala Thr
            720                 725                 730

GAT TTA CAG TAT CAA CAT TAT GAT AAA ACA TTA CTT AAA ACG GTA GAA 3819
Asp Leu Gln Tyr Gln His Tyr Asp Lys Thr Leu Leu Lys Thr Val Glu
        735                 740                 745

AGT ACC GAT AGT AGT GCT TCT GTC GTT AGA GTC ACG CCT TCT CAA TTA 3867
Ser Thr Asp Ser Ser Ala Ser Val Val Arg Val Thr Pro Ser Gln Leu
    750                 755                 760

AGT AGT ATA CGC AAT GCA AAG CAT GAT CCT ACC GTT ATG CAA AAC TTT 3915
Ser Ser Ile Arg Asn Ala Lys His Asp Pro Thr Val Met Gln Asn Phe
765                 770                 775                 780

GAA CAG AGT AAG GCA AAA ATT GCG ACT TTA AAT TCG CTC TAC GGG TTA 3963
Glu Gln Ser Lys Ala Lys Ile Ala Thr Leu Asn Ser Leu Tyr Gly Leu
                785                 790                 795

AAT ATC GAT CAA CTT TAT TAC ACG ACG GAT AAA GAC ATT CGC TAT ATT 4011
Asn Ile Asp Gln Leu Tyr Tyr Thr Thr Asp Lys Asp Ile Arg Tyr Ile
            800                 805                 810

ACT GAT AAA GTG AAT AAT ATG TAT CAA ACA ACC GTA GAA CTT GCC TAC 4059
Thr Asp Lys Val Asn Asn Met Tyr Gln Thr Thr Val Glu Leu Ala Tyr
        815                 820                 825

CGT TCT TTA CTT TTA CAA ACG CGT TTG AAG AAA TAT GTT TAT AGC GTT 4107
Arg Ser Leu Leu Leu Gln Thr Arg Leu Lys Lys Tyr Val Tyr Ser Val
    830                 835                 840

AAT GCG AAA CAA TTC GAA GGG AAA TGG GTA ACC GAT TAT TCT CGT ACT 4155
Asn Ala Lys Gln Phe Glu Gly Lys Trp Val Thr Asp Tyr Ser Arg Thr
845                 850                 855                 860

GAA GCC TTA TTT AAC TCT ACT TTT AAA CAA TCG CCT GAA AAT GCA TTA 4203
Glu Ala Leu Phe Asn Ser Thr Phe Lys Gln Ser Pro Glu Asn Ala Leu
                865                 870                 875

TAT GAT TTA AGC GAA TAC CTT TCT TTC TTT AAC GAT CCT ACG GAA TGG 4251
Tyr Asp Leu Ser Glu Tyr Leu Ser Phe Phe Asn Asp Pro Thr Glu Trp
            880                 885                 890

AAA GAA GGG CTA TTA CTG TTA AGC CGT TAT ATA GAT TAT GCT AAA GCA 4299
Lys Glu Gly Leu Leu Leu Leu Ser Arg Tyr Ile Asp Tyr Ala Lys Ala
        895                 900                 905

CAA GGA TTT TAT GAA AAC TGG GCG GCT ACT TCT AAC TTA ACT ATT GCC 4347
Gln Gly Phe Tyr Glu Asn Trp Ala Ala Thr Ser Asn Leu Thr Ile Ala
    910                 915                 920

CGT TTA AGA GAG GCT GGA GTA ATT TTT GCA GAA TCG ACG GAT TTA AAA 4395
Arg Leu Arg Glu Ala Gly Val Ile Phe Ala Glu Ser Thr Asp Leu [?]ys
925 930 935 940

GGC GAT GAA AAA AAT AAT ATT TTG TTA GGT AGC CAA AAA GAT AAT AAC 4443
Gly Asp Glu Lys Asn Asn Ile Leu Leu Gly Ser Gln Lys Asp Asn Asn
945 950 955

TTA TCG GGT AGT GCA GGT GAT GAT CTA CTT ATC GGC GGA GAG GGT AAT 4491
Leu Ser Gly Ser Ala Gly Asp Asp Leu Leu Ile Gly Gly Glu Gly Asn
960 965 970

GAT ACG TTA AAA GGC AGC TAC GGT GCA GAC ACC TAT ATC TTT AGC AAA 4539
Asp Thr Leu Lys Gly Ser Tyr Gly Ala Asp Thr Tyr Ile Phe Ser Lys
975 980 985

GGA CAC GGA CAG GAT ATC GTT TAT GAA GAT ACC AAT AAT GAT AAC CGC 4587
Gly His Gly Gln Asp Ile Val Tyr Glu Asp Thr Asn Asn Asp Asn Arg
990 995 1000

GCA AGA GAT ATC GAC ACC TTA AAA TTT ACC GAT GTG AAT TAT GCG GAA 4635
Ala Arg Asp Ile Asp Thr Leu Lys Phe Thr Asp Val Asn Tyr Ala Glu
1005 1010 1015 1020

GTG AAG TTT CGA CGA GTA GAT AAT GAC TTA ATG TTA TTC GGT TAT CAT 4683
Val Lys Phe Arg Arg Val Asp Asn Asp Leu Met Leu Phe Gly Tyr His
1025 1030 1035

GAT ACG GAT TCG GTC ACG GTA AAA TCC TTC TAC AGC CAT GTA GAT TAT 4731
Asp Thr Asp Ser Val Thr Val Lys Ser Phe Tyr Ser His Val Asp Tyr
1040 1045 1050

CAA TTT GAC AAA TTG GAG TTT GCT GAC CGC AGT ATA ACT CGC GAT GAA 4779
Gln Phe Asp Lys Leu Glu Phe Ala Asp Arg Ser Ile Thr Arg Asp Glu
1055 1060 1065

CTG ATT AAA GCA GGG CTT CAT CTA TAC GGC ACC GAT GGC AAT GAT GAT 4827
Leu Ile Lys Ala Gly Leu His Leu Tyr Gly Thr Asp Gly Asn Asp Asp
1070 1075 1080

ATA AAG GAT CAT GCG GAT TGG GAC AGC ATT TTG GAA GGC GGC AAA GGC 4875
Ile Lys Asp His Ala Asp Trp Asp Ser Ile Leu Glu Gly Gly Lys Gly
1085 1090 1095 1100

AAC GAT ATT CTA AGA GGT GGC TAC GGT GCG GAC ACC TAT ATC TTT AGC 4923
Asn Asp Ile Leu Arg Gly Gly Tyr Gly Ala Asp Thr Tyr Ile Phe Ser
1105 1110 1115

AAA GGA CAC GGA CAG GAT ATC GTT TAT GAA GAT ACC AAT AAT GAT AAC 4971
Lys Gly His Gly Gln Asp Ile Val Tyr Glu Asp Thr Asn Asn Asp Asn
1120 1125 1130

CGC GCA AGA GAT ATC GAC ACC TTA AAA TTT ACT GAT GTG AAT TAT GCG 5019
Arg Ala Arg Asp Ile Asp Thr Leu Lys Phe Thr Asp Val Asn Tyr Ala
1135 1140 1145

GAA GTG AAA TTC CGA CGA GTA GAT AAT GAC TTA ATG TTA TTC GGT TAT 5067
Glu Val Lys Phe Arg Arg Val Asp Asn Asp Leu Met Leu Phe Gly Tyr
1150 1155 1160

CAT GAT ACG GAT TCG GTC ACG ATA AAA TCC TTC TAC AAC CAT GTA GAT 5115
His Asp Thr Asp Ser Val Thr Ile Lys Ser Phe Tyr Asn His Val Asp
1165 1170 1175 1180

TAT CAA TTT GAC AAA TTG GAA TTT GCT GAC CGC AGT ATA ACT CGT GAT 5163
Tyr Gln Phe Asp Lys Leu Glu Phe Ala Asp Arg Ser Ile Thr Arg Asp
1185 1190 1195

GAA CTA GGT AAA CAA GGT ATG GCA TTA TTT GGC ACT GAC GGT GAT GAT 5211
Glu Leu Gly L[?]s Gln Gly Met Ala Leu Phe Gly Thr Asp Gly Asp Asp
1200 1205 1210

AAT ATC AAC GAC TGG GGA CGT AAC TCG GTG ATT GAT GCC GGT GCG GGT 5259
Asn Ile Asn Asp Trp Gly Arg Asn Ser Val Ile Asp Ala Gly Ala Gly
1215 1220 1225

AAT GAT ACG GTT AAT GGC GGT AAT GGC GAT GAC ACC CTC ATC GGC GGC 5307
Asn Asp Thr Val Asn Gly Gly Asn Gly Asp Asp Thr Leu Ile Gly Gly
1230 1235 1240

AAA GGT AAT GAT ATT CTA AGA GGT GGC TAC GGT GCG GAC ACC TAT ATC 5355
Lys Gly Asn Asp Ile Leu Arg Gly Gly Tyr Gly Ala Asp Thr Tyr Ile
1245 1250 1255 1260

TTT AGC AAA GGA CAC GGA CAG GAT ATC GTT TAT GAA GAT ACC AAT AAT 5403
Phe Ser Lys Gly His Gly Gln Asp Ile Val Tyr Glu Asp Thr Asn Asn
1265 1270 1275

GAT AAC CGC GCA AGA GAT ATC GAC ACC TTA AAA TTT ACC GAT GTG AAT 5451
Asp Asn Arg Ala Arg Asp Ile Asp Thr Leu Lys Phe Thr Asp Val Asn
1280 1285 1290

TAT GCG GAA GTG AAA TTC CGA CGA GTA GAT AAT GAC TTA ATG TTA TTC 5499
Tyr Ala Glu Val Lys Phe Arg Arg Val Asp Asn Asp Leu Met Leu Phe
1295 1300 1305

GGT TAT CAT GAT ACG GAT TCG GTC ACG GTA AAA TCC TTC TAC AGC CAT 5547
Gly Tyr His Asp Thr Asp Ser Val Thr Val Lys Ser Phe Tyr Ser His
1310 1315 1320

GTA GAT TAT CAA TTT GAC AAA TTG GAG TTT GCT GAC CGC AGT ATA ACT 5595
Val Asp Tyr Gln Phe Asp Lys Leu Glu Phe Ala Asp Arg Ser Ile Thr
1325 1330 1335 1340

CGC GAT GAA CTG ATT AAA GCA GGG CTT CAT CTA TAC GGC ACC GAT GGC 5643
Arg Asp Glu Leu Ile Lys Ala Gly Leu His Leu Tyr Gly Thr Asp Gly
1345 1350 1355

AAT GAT GAT ATA AAG GAT CAT GCG GAT TGG GAC AGC ATT TTG GAA GGC 5691
Asn Asp Asp Ile Lys Asp His Ala Asp Trp Asp Ser Ile Leu Glu Gly
1360 1365 1370

GGC AAA GGC AAC GAT ATT CTA AGA GGT GGC TAC GGT GCG GAC ACC TAT 5739
Gly Lys Gly Asn Asp Ile Leu Arg Gly Gly Tyr Gly Ala Asp Thr Tyr
1375 1380 1385

ATC TTT AGC AAA GGA CAC GGA CAG GAT ATC GTT TAT GAA GAT ACC AAT 5787
Ile Phe Ser Lys Gly His Gly Gln Asp Ile Val Tyr Glu Asp Thr Asn
1390 1395 1400

AAT GAT AAC CGA GCA AGA GAT ATC GAC ACC TTA AAA TTT ACT GAT GTG 5835
Asn Asp Asn Arg Ala Arg Asp Ile Asp Thr Leu Lys Phe Thr Asp Val
1405 1410 1415 1420

AAT TAT GCG GAA GTG AAA TTC CGA CGA GTA GAT AAT GAC TTA ATG TTA 5883
Asn Tyr Ala Glu Val Lys Phe Arg Arg Val Asp Asn Asp Leu Met Leu
1425 1430 1435

TTC GGT TAT CAT GAT ACG GAT TCG GTC ACG ATA AAA TCC TTC TAC AAC 5931
Phe Gly Tyr His Asp Thr Asp Ser Val Thr Ile Lys Ser Phe Tyr Asn
1440 1445 1450

CAT GTA GAT TAT CAA TTT GAC AAA TTG GAA TTT GCT GAC CGC AGT ATA 5979
His Val Asp Tyr Gln Phe Asp Lys Leu Glu Phe Ala Asp Arg Ser Ile
1455 1460 1465

ACT CGT GAT GAA CTA GGT AAA CAA GGT ATG GCA TTA TTT GGC ACT GAC 6027
Thr Arg Asp Glu Leu Gly Lys Gln Gly Met Ala Leu Phe Gly Thr Asp
1470 1475 1480

GGT GAT GAT AAT ATC AAC GAC TGG GGA CGT AAC TCG GTG ATT GAT GCC 6075
Gly Asp Asp Asn Ile Asn Asp Trp Gly Arg Asn Ser Val Ile Asp Ala
1485 1490 1495 1500

GGT GCG GGT AAT GAT ACG GTT AAT GGC GGT AAT GGC GAT GAC ACC CTC 6123
Gly Ala Gly Asn Asp Thr Val Asn Gly Gly Asn Gly Asp Asp Thr Leu
1505 1510 1515

ATC GGC GGC AAA GGT AAT GAT ATT CTA AGA GGT GGC TAC GGT GCG GAC 6171
Ile Gly Gly Lys Gly Asn Asp Ile Leu Arg Gly Gly Tyr Gly Ala Asp
1520 1525 1530

ACC TAT ATC TTT AGC AAA GGA CAC GGA CAG GAT ATC GTT TAT GAA GAT 6219
Thr Tyr Ile Phe Ser Lys Gly His Gly Gln Asp Ile Val Tyr Glu Asp
1535 1540 1545

ACC AAT AAT GAT AAC CGC GCA AGA GAT ATC GAC ACC TTA AAA TTT ACT 6267
Thr Asn Asn Asp Asn Arg Ala Arg Asp Ile Asp Thr Leu Lys Phe Thr
1550 1555 1560

GAT ATT AAT TTA TCC GAA CTT TGG TTT AGC CGA GAA AAT AAC GAT TTG 6315
Asp Ile Asn Leu Ser Glu Leu Trp Phe Ser Arg Glu Asn Asn Asp Leu
1565 1570 1575 1580

ATT ATT AAA TCA TTA TTA AGT GAG GAT AAA GTC ACG GTT CAA AAT TGG 6363
Ile Ile Lys Ser Leu Leu Ser Glu Asp Lys Val Thr Val Gln Asn Trp
1585 1590 1595

TAT TCA CAC CAA GAT CAT AAA ATA GAA AAT ATT CGT TTA TCG AAT GAG 6411
Tyr Ser His Gln Asp His Lys Ile Glu Asn Ile Arg Leu Ser Asn Glu
1600 1605 1610

CAA ACG TTG GTG AGC ACT CAG GTG GAG AAG ATG GTT GAG TCG ATG GCC 6459
Gln Thr Leu Val Ser Thr Gln Val Glu Lys Met Val Glu Ser Met Ala
1615 1620 1625

GGC TTT GCT CAG AAG CAC GGA GGA GAG ATA TCT CTT GTG TCG CTT GAA 6507
Gly Phe Ala Gln Lys His Gly Gly Glu Ile Ser Leu Val Ser Leu Glu
1630 1635 1640

GAG GTA AAA CAA TAT ATC AAT AGC TTA ACA GCT GCT TTA TAA 6549
Glu Val Lys Gln Tyr Ile Asn Ser Leu Thr Ala Ala Leu *
1645 1650 1655

CATACGAAAG AAATCGGCAC AGTTTTTTTG AACTGTGCCG ATTTGATTTT AGTGTAAGAA 6609

TATAGCCTGA TTTTAAGAAA TTTACTCTTG GCTAATAACT ATTTCCCATT TTATAAGTTA 6669

TTGACGGATG GTTTTATCAA ATATGAGATC AAATCTTATT TTAAATTCGC TTTCCATTAA 6729

GCGATAT 6736

(2) INFORMATION FOR SEQ ID NO: 2:

(i) SEQUENCE CHARACTERISTICS:
(A) LENGTH: 1658 amino acids
(B) TYPE: amino acid
(D) TOPOLOGY: linear

(ii) MOLECULE TYPE: protein
(xi) SEQUENCE DESCRIPTION: SEQ ID NO: 2:

Met Ser Asp Asn Ala Phe Phe Val Ile Glu Glu Ser Gly Lys Arg Tyr
1               5                   10                  15

Ile Glu Asn Phe Gly Ile Glu Pro Leu Gly Lys Gln Glu Asp Phe Asp
            20                  25                  30

Phe Val Gly Gly Phe Trp Ser Asn Leu Val Asn Arg Gly Leu Glu Ser
        35                  40                  45

Ile Ile Asp Pro Ser Gly Ile Gly Gly Thr Val Asn Leu Asn Phe Thr
    50                  55                  60

Gly Glu Val Glu Thr Tyr Thr Leu Asp Glu Thr Arg Phe Lys Ala Glu
65                  70                  75                  80

Ala Ala Lys Lys Ser His Trp Ser Leu Val Asn Ala Ala Lys Val Tyr
                85                  90                  95

Gly Gly Leu Asp Gln Ile Ile Lys Lys Leu Trp Asp Ser Gly Ser Ile
            100                 105                 110

Lys His Leu Tyr Gln Asp Lys Asp Thr Gly Lys Leu Lys Pro Ile Ile
        115                 120                 125

Tyr Gly Thr Ala Gly Asn Asp Ser Lys Ile Glu Gly Thr Lys Ile Thr
    130                 135                 140

Arg Arg Ile Ala Gly Lys Glu Val Thr Leu Asp Ile Ala Asn Gln Lys
145                 150                 155                 160

Ile Glu Lys Gly Val Leu Glu Lys Leu Gly Leu Ser Val Ser Gly Ser
                165                 170                 175

Asp Ile Ile Lys Leu Leu Phe Gly Ala Leu Thr Pro Thr Leu Asn Arg
            180                 185                 190

Met Leu Leu Ser Gln Leu Ile Gln Ser Phe Ser Asp Ser Leu Ala Lys
        195                 200                 205

Leu Asp Asn Pro Leu Ala Pro Tyr Thr Lys Asn Gly Val Val Tyr Val
    210                 215                 220

Thr Gly Lys Gly Asn Asp Val Leu Lys Gly Thr Glu His Glu Asp Leu
225                 230                 235                 240

Phe Leu Gly Gly Glu Gly Asn Asp Thr Tyr Tyr Ala Arg Val Gly Asp
                245                 250                 255

Thr Ile Glu Asp Ala Asp Gly Lys Gly Lys Val Tyr Phe Val Arg Glu
            260                 265                 270

Lys Gly Val Pro Lys Ala Asp Pro Lys Arg Val Glu Phe Ser Glu Tyr
        275                 280                 285

Ile Thr Lys Glu Glu Ile Lys Glu Val Glu Lys Gly Leu Leu Thr Tyr
    290                 295                 300

Ala Val Leu Glu Asn Tyr Asn Trp Glu Glu Lys Thr Ala Thr Phe Ala
305                 310                 315                 320

His Ala Thr Met Leu Asn Glu Leu Phe Thr Asp Tyr Thr Asn Tyr Arg
                325                 330                 335

Tyr Glu Val Lys Gly Leu Lys Leu Pro Ala Val Lys Lys Leu Lys Ser
            340                 345                 350

Pro Leu Val Glu Phe Thr Ala Asp Leu Leu Thr Val Thr Pro Ile Asp
        355                 360                 365

Glu Asn Gly Lys Ala Leu Ser Glu Lys Ser Ile Thr Val Lys Asn Phe
    370                 375                 380

Lys Asn Gly Asp Leu Gly Ile Arg Leu Leu Asp Pro Asn Ser Tyr Tyr
385                 390                 395                 400

Tyr Phe Leu Glu Gly Gln Asp Thr Gly Phe Tyr Gly Pro Ala Phe Tyr
                405                 410                 415

Ile Glu Arg Lys Asn Gly Gly Gly Ala Lys Asn Asn Ser Ser Gly Ala
            420                 425                 430

Gly Asn Ser Lys Asp Trp Gly Gly Asn Gly His Gly Asn His Arg Asn
        435                 440                 445

Asn Ala Ser Asp Leu Asn Lys Pro Asp Gly Asn Asn Gly Asn Asn Gln
    450                 455                 460

Asn Asn Gly Ser Asn Gln Asp Asn His Ser Asp Val Asn Ala Pro Asn
465                 470                 475                 480

Asn Pro Gly Arg Asn Tyr Asp Ile Tyr Asp Pro Leu Ala Leu Asp Leu
                485                 490                 495

Asp Gly Asp Gly Leu Glu Thr Val Ser Met Asn Gly Arg Gln Gly Ala
            500                 505                 510

Leu Phe Asp His Glu Gly Lys Gly Ile Arg Thr Ala Thr Gly Trp Leu
        515                 520                 525

Ala Ala Asp Asp Gly Phe Leu Val Leu Asp Arg Asn Gln Asp Gly Ile
    530                 535                 540

Ile Asn Asp Ile Ser Glu Leu Phe Ser Asn Lys Asn Gln Leu Ser Asp
545                 550                 555                 560

Gly Ser Ile Ser Ala His Gly Phe Ala Thr Leu Ala Asp Leu Asp Thr
                565                 570                 575

Asn Gln Asp Gln Arg Ile Asp Gln Asn Asp Lys Leu Phe Ser Lys Leu
            580                 585                 590

Gln Ile Trp Arg Asp Leu Asn Gln Asn Gly Phe Ser Glu Ala Asn Glu
        595                 600                 605

Leu Phe Ser Leu Glu Ser Leu Asn Ile Lys Ser Leu His Thr Ala Tyr
    610                 615                 620

Glu Glu Arg Asn Asp Phe Leu Ala Gly Asn Asn Ile Leu Ala Gln Leu
625                 630                 635                 640

Gly Lys Tyr Glu Lys Thr Asp Gly Thr Phe Ala Gln Met Gly Asp Leu
                645                 650                 655

Asn Phe Ser Phe Asn Pro Phe Tyr Ser Arg Phe Thr Glu Ala Leu Asn
            660                 665                 670

Leu Thr Glu Gln Gln Arg Arg Thr Ile Asn Leu Thr Gly Thr Gly Arg
        675                 680                 685

Val Arg Asp Leu Arg Glu Ala Ala Ala Leu Ser Glu Glu Leu Ala Ala
    690                 695                 700

Leu Leu Gln Gln Tyr Thr Lys Ala Ser Asp Phe Gln Ala Gln Arg Glu
705                 710                 715                 720

Leu Leu Pro Ala Ile Leu Asp Lys Trp Ala Ala Thr Asp Leu Gln Tyr
                725                 730                 735

Gln His Tyr Asp Lys Thr Leu Leu Lys Thr Val Glu Ser Thr Asp Ser
            740                 745                 750

Ser Ala Ser Val Val Arg Val Thr Pro Ser Gln Leu Ser Ser Ile Arg
        755                 760                 765

Asn Ala Lys His Asp Pro Thr Val Met Gln Asn Phe Glu Gln Ser Lys
    770                 775                 780

Ala Lys Ile Ala Thr Leu Asn Ser Leu Tyr Gly Leu Asn Ile Asp Gln
785                 790                 795                 800

Leu Tyr Tyr Thr Thr Asp Lys Asp Ile Arg Tyr Ile Thr Asp Lys Val
                805                 810                 815

Asn Asn Met Tyr Gln Thr Thr Val Glu Leu Ala Tyr Arg Ser Leu Leu
            820                 825                 830

Leu Gln Thr Arg Leu Lys Lys Tyr Val Tyr Ser Val Asn Ala Lys Gln
        835                 840                 845

Phe Glu Gly Lys Trp Val Thr Asp Tyr Ser Arg Thr Glu Ala Leu Phe
    850                 855                 860

Asn Ser Thr Phe Lys Gln Ser Pro Glu Asn Ala Leu Tyr Asp Leu Ser
865                 870                 875                 880

Glu Tyr Leu Ser Phe Phe Asn Asp Pro Thr Glu Trp Lys Glu Gly Leu
                885                 890                 895

Leu Leu Leu Ser Arg Tyr Ile Asp Tyr Ala Lys Ala Gln Gly Phe Tyr
            900                 905                 910

Glu Asn Trp Ala Ala Thr Ser Asn Leu Thr Ile Ala Arg Leu Arg Glu
        915                 920                 925

Ala Gly Val Ile Phe Ala Glu Ser Thr Asp Leu Lys Gly Asp Glu Lys
    930                 935                 940

Asn Asn Ile Leu Leu Gly Ser Gln Lys Asp Asn Asn Leu Ser Gly Ser
945                 950                 955                 960

Ala Gly Asp Asp Leu Leu Ile Gly Gly Glu Gly Asn Asp Thr Leu Lys
                965                 970                 975

Gly Ser Tyr Gly Ala Asp Thr Tyr Ile Phe Ser Lys Gly His Gly Gln
            980                 985                 990

Asp Ile Val Tyr Glu Asp Thr Asn Asn Asp Asn Arg Ala Arg Asp Ile
        995                 1000                1005

Asp Thr Leu Lys Phe Thr Asp Val Asn Tyr Ala Glu Val Lys Phe Arg
    1010                1015                1020

Arg Val Asp Asn Asp Leu Met Leu Phe Gly Tyr His Asp Thr Asp Ser
1025                1030                1035                1040

Val Thr Val Lys Ser Phe Tyr Ser His Val Asp Tyr Gln Phe Asp Lys
                1045                1050                1055

12

Leu Glu Phe Ala Asp Arg Ser Ile Thr Arg Asp Glu Leu Ile Lys Ala
            1060                1065                1070

Gly Leu His Leu Tyr Gly Thr Asp Gly Asn Asp Asp Ile Lys Asp His
        1075                1080                1085

Ala Asp Trp Asp Ser Ile Leu Glu Gly Gly Lys Gly Asn Asp Ile Leu
    1090                1095                1100

Arg Gly Gly Tyr Gly Ala Asp Thr Tyr Ile Phe Ser Lys Gly His Gly
1105                1110                1115                1120

Gln Asp Ile Val Tyr Glu Asp Thr Asn Asn Asp Asn Arg Ala Arg Asp
                1125                1130                1135

Ile Asp Thr Leu Lys Phe Thr Asp Val Asn Tyr Ala Glu Val Lys Phe
            1140                1145                1150

Arg Arg Val Asp Asn Asp Leu Met Leu Phe Gly Tyr His Asp Thr Asp
        1155                1160                1165

Ser Val Thr Ile Lys Ser Phe Tyr Asn His Val Asp Tyr Gln Phe Asp
    1170                1175                1180

Lys Leu Glu Phe Ala Asp Arg Ser Ile Thr Arg Asp Glu Leu Gly Lys
1185                1190                1195                1200

Gln Gly Met Ala Leu Phe Gly Thr Asp Gly Asp Asp Asn Ile Asn Asp
                1205                1210                1215

Trp Gly Arg Asn Ser Val Ile Asp Ala Gly Ala Gly Asn Asp Thr Val
            1220                1225                1230

Asn Gly Gly Asn Gly Asp Asp Thr Leu Ile Gly Gly Lys Gly Asn Asp
        1235                1240                1245

Ile Leu Arg Gly Gly Tyr Gly Ala Asp Thr Tyr Ile Phe Ser Lys Gly
    1250                1255                1260

His Gly Gln Asp Ile Val Tyr Glu Asp Thr Asn Asn Asp Asn Arg Ala
1265                1270                1275                1280

Arg Asp Ile Asp Thr Leu Lys Phe Thr Asp Val Asn Tyr Ala Glu Val
                1285                1290                1295

Lys Phe Arg Arg Val Asp Asn Asp Leu Met Leu Phe Gly Tyr His Asp
            1300                1305                1310

Thr Asp Ser Val Thr Val Lys Ser Phe Tyr Ser His Val Asp Tyr Gln
        1315                1320                1325

Phe Asp Lys Leu Glu Phe Ala Asp Arg Ser Ile Thr Arg Asp Glu Leu
    1330                1335                1340

Ile Lys Ala Gly Leu His Leu Tyr Gly Thr Asp Gly Asn Asp Asp Ile
1345                1350                1355                1360

Lys Asp His Ala Asp Trp Asp Ser Ile Leu Glu Gly Gly Lys Gly Asn
                1365                1370                1375

Asp Ile Leu Arg Gly Gly Tyr Gly Ala Asp Thr Tyr Ile Phe Ser Lys
            1380                1385                1390

Gly His Gly Gln Asp Ile Val Tyr Glu Asp Thr Asn Asn Asp Asn Arg
        1395                1400                1405

Ala Arg Asp Ile Asp Thr Leu Lys Phe Thr Asp Val Asn Tyr Ala Glu
    1410                1415                1420

Val Lys Phe Arg Arg Val Asp Asn Asp Leu Met Leu Phe Gly Tyr His
1425                1430                1435                1440

Asp Thr Asp Ser Val Thr Ile Lys Ser Phe Tyr Asn His Val Asp Tyr
                1445                1450                1455

Gln Phe Asp Lys Leu Glu Phe Ala Asp Arg Ser Ile Thr Arg Asp Glu
            1460                1465                1470

Leu Gly Lys Gln Gly Met Ala Leu Phe Gly Thr Asp Gly Asp Asp Asn
        1475                1480                1485

Ile Asn Asp Trp Gly Arg Asn Ser Val Ile Asp Ala Gly Ala Gly Asn
    1490                1495                1500

Asp Thr Val Asn Gly Gly Asn Gly Asp Asp Thr Leu Ile Gly Gly Lys
1505                1510                1515                1520

Gly Asn Asp Ile Leu Arg Gly Gly Tyr Gly Ala Asp Thr Tyr Ile Phe
                1525                1530                1535

Ser Lys Gly His Gly Gln Asp Ile Val Tyr Glu Asp Thr Asn Asn Asp
            1540                1545                1550

Asn Arg Ala Arg Asp Ile Asp Thr Leu Lys Phe Thr Asp Ile Asn Leu
        1555                1560                1565

Ser Glu Leu Trp Phe Ser Arg Glu Asn Asn Asp Leu Ile Ile Lys Ser
    1570                1575                1580

Leu Leu Ser Glu Asp Lys Val Thr Val Gln Asn Trp Tyr Ser His Gln
1585                1590                1595                1600

Asp His Lys Ile Glu Asn Ile Arg Leu Ser Asn Glu Gln Thr Leu Val
                1605                1610                1615

Ser Thr Gln Val Glu Lys Met Val Glu Ser Met Ala Gly Phe Ala Gln
            1620                1625                1630

Lys His Gly Gly Glu Ile Ser Leu Val Ser Leu Glu Glu Val Lys Gln
        1635                1640                1645

Tyr Ile Asn Ser Leu Thr Ala Ala Leu  *
    1650                1655

(2) INFORMATION FOR SEQ ID NO: 3:

(i) SEQUENCE CHARACTERISTICS:
(A) LENGTH: 7004 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Actinobacillus pleuropneumoniae
(B) STRAIN: HV114 (serotype 3 field strain)

(vii) IMMEDIATE SOURCE:
(B) CLONE: pROK5

(ix) FEATURE:
(A) NAME/KEY: CDS
(B) LOCATION:1566..5714
(D) OTHER INFORMATION:/codon_start= 1566
/function= "RTX-toxin"
/product= "ApxIV_var3"
/gene= "apxIV_var3"
/number= 1

(xi) SEQUENCE DESCRIPTION: SEQ ID NO: 3:
   ATCGATATGC CGCCGGGTAC GGGCGATATC CAACTTACTC TTTCGCAACA AATTCCGGTT 60
   ACCGGTGCGG TAGTGGTAAC TACTCCGCAA GATATTGCGT TATTAGATGC GGTGAAAGGT 120
   ATTTCAATGT TCCAAAAAGT GTCGGTACCG GTCTTAGGTA TCATTGAAAA TATGAGCGTG 180
   CATATCTGCC AAAATTGCGG TCACCACGAA GATATTTTCG GCACCGGCGG TGCGGAGAAA 240
   GTGGCGAAGA AATACGGTAC TAAAGTATTA GGACAAATGC CGTTGCATAT TCGCTTACGT 300
   CAAGATTTGG ATGCCGGCAC ACCGACCGTC GTTGCGGCAC CGGAACACGA CACCAGCAGA 360
   GCCTATATTG AATTAGCGGC AAAAGTCGCT TCGGAATTAT ACTGGCAAGG TTCGGTTATC 420
   CCGTCTGAAA TTATGATTCG TGAAGTAAAA TAAGCCTACA TAACCACGGA ATACCAGATA 480
   ACACAGAAGG AAAACAAGCG GTAGAATTTG CAGAAAAAGT TGCAAATTCT ACCGCTTTTT 540
   TATTAGTACG ATTCGCTGTT GGACTGCCAT TTGATTTGGT TTGTCAGGAT ATTATGTTAT 600
   TGTAATGAAA TGTTAGTGAA TTATTTTTAT TAATTTGAAA GGAGACAAAA TGAAAATAAA 660
   AAAACGTTAC ATTGCGCTGC TAGCTTTAGG CAGTGTTATT GGCTATGCCT GGTATCAAAA 720
   TTATCAATGG GAACAGTTGA TGTTAAGTGG CTATTGTGAA AAGGACGGAA GCTATTGTGA 780
   TGATAGGCAT ACGAAGCAGG AACTGATTGA TAGGGCAATT AACTATGTGC TGGAAAATCA 840
   AATTCAACAG ACATATGAAG GTGATGACCT TGTGGATATA AAACAATATT CAACAATAGA 900
   GGAATTTAAA AAACTAAATC CGAATTGTTG TAAGGTAGAT TCTTGGCCGG ATGATGCTGT 960
   TCGTGAGGAT GCTGATTTAC AGCGAGAGGG CAAAGCGTAT AAATACGTAA AAGTCAAATA 1020
   TTTAAGAACC TATTTAGCGA ATAGAGAACC TGAACAATGG GAAAATTACA TAGTATTTGA 1080
   TAATTGCAGT GGAATTAAAG AAAGACACCA ACTGTATTAA AAATAGATTA GATGGAGACA 1140
   ACACGATGAC AAAACTAACT ATCCAAGATG TGACTAATTT ATATTTATAT AAGCAAAGAA 1200
   CTTTACCTAC GGATAGGTTA GATGATTCGC TTATTAGCAA AACAGGAAAA GGGGAAAATA 1260
   TTGATAAAAA GGAATTTATG GCGGGGCCGG GACGTTTTGT GACGGCCGAT AATTTTAGTG 1320
   TTGTAAAAGA CTTTTTTACT GCAAAGGATT CATTAATAAA CCTAAGCTTG CAGACTCGTA 1380
   TATTAGCGAA TTTAAAGCCG GGCAAATATT CCAAAGCGCA GATATTAGAA ATGTTGGGCT 1440
   ATACGAAAAA TGGAGAAAAG GTAGATGGCA TGTTTACCGG TGAAGTCCAG ACATTAGGCT 1500
   TTTATGACGA TGGCAAAGGG GATTTACTCG AACGCGCCTA TATCTGAAAT ACCACAGGAT 1560
   TTAAA ATG AGC GAC AAT GCC TTT TTT GTT ATA GAA GAA TCA GGC AAA 1607
   Met Ser Asp Asn Ala Phe Phe Val Ile Glu Glu Ser Gly Lys
   1660 1665 1670
   CGC TAT ATT GAA AAC TTT GGT ATT GAA CCT CTT GGT AAG CAA GAA GAT 1655
   Arg Tyr Ile Glu Asn Phe Gly Ile Glu Pro Leu Gly Lys Gln Glu Asp
   1675 1680 1685
   TTT GAT TTT GTC GGC GGC TTT TGG TCT AAC TTA GTG AAT CGT GGT TTG 1703
   Phe Asp Phe Val Gly Gly Phe Trp Ser Asn Leu Val Asn Arg Gly Leu
   1690 1695 1700
   GAA AGT ATT ATC GAC CCA TCC GGT ATC GGT GGA ACG GTA AAC CTT AAC 1751
   Glu Ser Ile Ile Asp Pro Ser Gly Ile Gly Gly Thr Val Asn Leu Asn
   1705 1710 1715 1720
   TTT ACC GGC GAG GTG GAA ACC TAC ACG TTA GAC GAA ACA AGG TTT AAA 1799
   Phe Thr Gly Glu Val Glu Thr Tyr Thr Leu Asp Glu Thr Arg Phe Lys
   1725 1730 1735
   GCG GAA GCG GCG AAG AAA AGC CAT TGG AGT TTA GTG AAT GCG GCG AAA 1847
   Ala Glu Ala Ala Lys Lys Ser His Trp Ser Leu Val Asn Ala Ala Lys
   1740 1745 1750
   GTA TAC GGC GGT TTA GAC CAA ATT ATT AAA AAA CTA TGG GAC AGT GGC 1895
   Val Tyr Gly Gly Leu Asp Gln Ile Ile Lys Lys Leu Trp Asp Ser Gly
   1755 1760 1765
   TCA ATT AAG CAT TTA TAT CAA GAT AAA GAT ACG GGC AAA TTA AAA CCG 1943
   Ser Ile Lys His Leu Tyr Gln Asp Lys Asp Thr Gly Lys Leu Lys Pro
   1770 1775 1780
   ATT ATT TAC GGC ACG GCC GGC AAC GAC AGT AAG ATT GAA GGC ACT AAA 1991
   Ile Ile Tyr Gly Thr Ala Gly Asn Asp Ser Lys Ile Glu Gly Thr Lys
   1785 1790 1795 1800
   ATC ACC CGT AGG ATT GCG GGT AAA GAA GTT ACG CTT GAT ATT GCC AAT 2039
   Ile Thr Arg Arg Ile Ala Gly Lys Glu Val Thr Leu Asp Ile Ala Asn
   1805 1810 1815
   CAG AAA ATT GAA AAA GGC GTG TTA GAG AAA TTG GGG CTG TCT GTT AGT 2087
   Gln Lys Ile Glu Lys Gly Val Leu Glu Lys Leu Gly Leu Ser Val Ser
   1820 1825 1830
   GGT TCG GAT ATC ATT AAA TTG TTG TTT GGA GCA TTG ACT CCA ACT TTA 2135
   Gly Ser Asp Ile Ile Lys Leu Leu Phe Gly Ala Leu Thr Pro Thr Leu
   1835 1840 1845
   AAT AGA ATG TTG CTA TCA CAA CTT ATC CAG TCT TTT TCC GAT AGC TTG 2183
   Asn Arg Met Leu Leu Ser Gln Leu Ile Gln Ser Phe Ser Asp Ser Leu
   1850 1855 1860
   GCT AAA CTT GAT AAT CCC TTA GCC CCT TAC ACT AAA AAT GGC GTG GTT 2231
   Ala Lys Leu Asp Asn Pro Leu Ala Pro Tyr Thr Lys Asn Gly Val Val
   1865 1870 1875 1880
   TAT GTC ACC GGC AAA GGG AAT GAT GTG CTT AAA GGA ACT GAA CAT GAG 2279
   Tyr Val Thr Gly Lys Gly Asn Asp Val Leu Lys Gly Thr Glu His Glu
   1885 1890 1895
   GAT TTG TTT CTC GGT GGT GAG GGG AAT GAT ACT TAT TAT GCG AGA GTA 2327
   Asp Leu Phe Leu Gly Gly Glu Gly Asn Asp Thr Tyr Tyr Ala Arg Val
   1900 1905 1910
   GGC GAT ACA ATT GAA GAC GCC GAC GGC AAA GGT AAA GTC TAT TTT GTG 2375
   Gly Asp Thr Ile Glu Asp Ala Asp Gly Lys Gly Lys Val Tyr Phe Val
   1915 1920 1925
   AGA GAA AAA GGG GTA CCT AAG GCG GAT CCT AAG CGG GTA GAG TTT AGC 2423
   Arg Glu Lys Gly Val Pro Lys Ala Asp Pro Lys Arg Val Glu Phe Ser
   1930 1935 1940
   GAG TAC ATA ACG AAA GAA GAA ATA AAA GAG GTT GAA AAG GGG TTA TTA 2471
   Glu Tyr Ile Thr Lys Glu Glu Ile Lys Glu Val Glu Lys Gly Leu Leu
   1945 1950 1955 1960
   ACT TAC GCA GTT TTA GAA AAT TAT AAT TGG GAA GAG AAA ACG GCG ACT 2519
   Thr Tyr Ala Val Leu Glu Asn Tyr Asn Trp Glu Glu Lys Thr Ala Thr
   1965 1970 1975
   TTC GCT CAT GCG ACT ATG CTT AAT GAG CTT TTT ACT GAT TAT ACT AAT 2567
   Phe Ala His Ala Thr Met Leu Asn Glu Leu Phe Thr Asp Tyr Thr Asn
   1980 1985 1990
   TAT CGT TAT GAA GTT AAA GGA CTA AAA TTG CCC GCC GTT AAA AAG TTA 2615
   Tyr Arg Tyr Glu Val Lys Gly Leu Lys Leu Pro Ala Val Lys Lys Leu
   1995 2000 2005
   AAA AGT CCG TTG GTG GAG TTT ACA GCT GAT TTA TTA ACT GTT ACG CCT 2663
   Lys Ser Pro Leu Val Glu Phe Thr Ala Asp Leu Leu Thr Val Thr Pro
   2010 2015 2020
   ATT GAC GAA AAC GGA AAA GCA CTT AGC GAA AAA AGT ATT ACG GTT AAA 2711
   Ile Asp Glu Asn Gly Lys Ala Leu Ser Glu Lys Ser Ile Thr Val Lys
   2025 2030 2035 2040
   AAT TTT AAA AAT GGT GAT TTA GGA ATA AGG TTG TTG GAT CCT AAT AGC 2759
   Asn Phe Lys Asn Gly Asp Leu Gly Ile Arg Leu Leu Asp Pro Asn Ser
   2045 2050 2055
   TAT TAT TAT TTC CTT GAA GGC CAA GAT ACG GGT TTT TAT GGT CCT GCT 2807
   Tyr Tyr Tyr Phe Leu Glu Gly Gln Asp Thr Gly Phe Tyr Gly Pro Ala
   2060 2065 2070
   TTT TAT ATT GAA CGA AAA AAC GGT GGA GGC TCT AAA AAT AAC TCG TCG 2855
   Phe Tyr Ile Glu Arg Lys Asn Gly Gly Gly Ser Lys Asn Asn Ser Ser
   2075 2080 2085
   GGA GCA GGA AAT AGC AAA GAT TGG GGC GGG AAC GGG CAT GGA AAT CAC 2903
   Gly Ala Gly Asn Ser Lys Asp Trp Gly Gly Asn Gly His Gly Asn His
   2090 2095 2100
   CGA AAT AAT GCC TCC GAC CTG AAT AAA CCG GAC GGA AAT AAT GGG AAT 2951
   Arg Asn Asn Ala Ser Asp Leu Asn Lys Pro Asp Gly Asn Asn Gly Asn
   2105 2110 2115 2120
   AAC CAA AAT AAC GGA AGC AAT CAA GAT AAT CAT AGC GAT GTG AAT GCG 2999
   Asn Gln Asn Asn Gly Ser Asn Gln Asp Asn His Ser Asp Val Asn Ala
   2125 2130 2135
   CCA AAT AAC CCG GGA CGT AAC TAT GAT ATT TAC GAT CCT TTA GCT TTA 3047
   Pro Asn Asn Pro Gly Arg Asn Tyr Asp Ile Tyr Asp Pro Leu Ala Leu
   2140 2145 2150
   GAT TTA GAT GGA GAT GGG CTT GAA ACC GTG TCG ATG AAC GGG CGA CAA 3095
   Asp Leu Asp Gly Asp Gly Leu Glu Thr Val Ser Met Asn Gly Arg Gln
   2155 2160 2165
   GGC GCG TTA TTC GAT CAT GAA GGA AAA GGT ATT CGT ACC GCA ACG GGC 3143
   Gly Ala Leu Phe Asp His Glu Gly Lys Gly Ile Arg Thr Ala Thr Gly
   2170 2175 2180
   TGG CTC GCT GCG GAT GAC GGT TTT TTA GTG TTA GAT CGT AAC CAA GAC 3191
   Trp Leu Ala Ala Asp Asp Gly Phe Leu Val Leu Asp Arg Asn Gln Asp
   2185 2190 2195 2200
   GGC ATT ATT AAT GAT ATA AGC GAG TTA TTT AGT AAT AAA AAT CAA CTT 3239
   Gly Ile Ile Asn Asp Ile Ser Glu Leu Phe Ser Asn Lys Asn Gln Leu
   2205 2210 2215
   TCC GAC GGG AGT ATT TCT GCA CAC GGT TTT GCG ACA TTA GCC GAT TTG 3287
   Ser Asp Gly Ser Ile Ser Ala His Gly Phe Ala Thr Leu Ala Asp Leu
   2220 2225 2230
   GAT ACA AAC CAA GAT CAG CGT ATC GAC CAA AAT GAT AAG CTG TTT TCT 3335
   Asp Thr Asn Gln Asp Gln Arg Ile Asp Gln Asn Asp Lys Leu Phe Ser
   2235 2240 2245
   AAA CTC CAA ATT TGG CGG GAT TTA AAT CAA AAC GGT TTT AGT GAA GCG 3383
   Lys Leu Gln Ile Trp Arg Asp Leu Asn Gln Asn Gly Phe Ser Glu Ala
   2250 2255 2260
   AAT GAG CTG TTT AGC TTA GAA AGT TTG AAT ATT AAA TCT TTA CAT ACC 3431
   Asn Glu Leu Phe Ser Leu Glu Ser Leu Asn Ile Lys Ser Leu His Thr
   2265 2270 2275 2280
   GCC TAT GAA GAG CGT AAT GAT TTT CTA GCG GGC AAT AAT ATC CTT GCT 3479
   Ala Tyr Glu Glu Arg Asn Asp Phe Leu Ala Gly Asn Asn Ile Leu Ala
   2285 2290 2295
   CAG CTT GGG AAG TAT GAA AAA ACG GAC GGT ACT TTT GGA CAA ATG GGC 3527
   Gln Leu Gly Lys Tyr Glu Lys Thr Asp Gly Thr Phe Gly Gln Met Gly
   2300 2305 2310
   GAT TTA AAT TTC AGT TTT AAC CCG TTT TAT AGC CGA TTT ACC GAA GCG 3575
   Asp Leu Asn Phe Ser Phe Asn Pro Phe Tyr Ser Arg Phe Thr Glu Ala
   2315 2320 2325
   TTA AAT TTA ACC GAG CAA CAA CGT CGC ACA ATT AAT CTA ACC GGC ACC 3623
   Leu Asn Leu Thr Glu Gln Gln Arg Arg Thr Ile Asn Leu Thr Gly Thr
   2330 2335 2340
   GGT CGG GTT CGG GAT TTG CGT GAA GCC GCC GCA CTT TCT GAG GAG TTG 3671
   Gly Arg Val Arg Asp Leu Arg Glu Ala Ala Ala Leu Ser Glu Glu Leu
   2345 2350 2355 2360
   GCT GCT TTA TTA CAA CAG TAC ACT AAG GGC TCC GAT TTT CAG GCA CAA 3719
   Ala Ala Leu Leu Gln Gln Tyr Thr Lys Gly Ser Asp Phe Gln Ala Gln
   2365 2370 2375
   CGA GAA TTA TTG CCT GCC ATT TTA GAT AAA TGG GCG GCA ACG GAT TTA 3767
   Arg Glu Leu Leu Pro Ala Ile Leu Asp Lys Trp Ala Ala Thr Asp Leu
   2380 2385 2390
   CAG TAT CAA CAT TAT GAT AAA ACA TTA CTT AAA ACG GTA GAA AGT ACC 3815
   Gln Tyr Gln His Tyr Asp Lys Thr Leu Leu Lys Thr Val Glu Ser Thr
   2395 2400 2405
   GAT AGT AGT GCT TCT GTC GTT AGA GTC ACG CCT TCT CAA TTA AGT AGT 3863
   Asp Ser Ser Ala Ser Val Val Arg Val Thr Pro Ser Gln Leu Ser Ser
   2410 2415 2420
   ATA CGC AAT GTA AAG CAT GAT CCT ACC GTT ATG CAA AAC TGT GAA CAA 3911
   Ile Arg Asn Val Lys His Asp Pro Thr Val Met Gln Asn Cys Glu Gln
   2425 2430 2435 2440
   AGT AAG GCA AAA ATT GCG ACT TTA AAT TCG CTC TAC GGG TTA AAT ATT 3959
   Ser Lys Ala Lys Ile Ala Thr Leu Asn Ser Leu Tyr Gly Leu Asn Ile
   2445 2450 2455
   GAT CAA CTT TAT TAT ACG ACG GAT AAA GAC ATT CGT TAT ATT ACT GAC 4007
   Asp Gln Leu Tyr Tyr Thr Thr Asp Lys Asp Ile Arg Tyr Ile Thr Asp
   2460 2465 2470
   AAA GTG AAT AAT ATG TAT CAA ACA ACC GGA GAA CTC GGC TAT CGT TCT 4055
   Lys Val Asn Asn Met Tyr Gln Thr Thr Gly Glu Leu Gly Tyr Arg Ser
   2475 2480 2485
   TTA CTT TTA CAA ACG CGT GTG AAG AAA TAT GTT TAT AGC GTT AAT GCG 4103
   Leu Leu Leu Gln Thr Arg Val Lys Lys Tyr Val Tyr Ser Val Asn Ala
   2490 2495 2500
   AAA CAA TTC GAA GGG AAA TGG GTA GCC GAT TAT TCT CGT ACT GAA GCC 4151
   Lys Gln Phe Glu Gly Lys Trp Val Ala Asp Tyr Ser Arg Thr Glu Ala
   2505 2510 2515 2520
   TTA TTT AAC TCT ACT TAT AAA CAA TCG CCC GAA AAT GTA TTA TAT GAT 4199
   Leu Phe Asn Ser Thr Tyr Lys Gln Ser Pro Glu Asn Val Leu Tyr Asp
   2525 2530 2535
   TTA CGC GAA TAC CTT TCT TTC TAT AAC GAC CCT ACG GAA TGG AAA GAA 4247
   Leu Arg Glu Tyr Leu Ser Phe Tyr Asn Asp Pro Thr Glu Trp Lys Glu
   2540 2545 2550
   GGG CTA TTA CTG TTA AGC CGT TAT ATA GAT TAT GCT AAA GCA CAA GGA 4295
   Gly Leu Leu Leu Leu Ser Arg Tyr Ile Asp Tyr Ala Lys Ala Gln Gly
   2555 2560 2565
   TTT TAT GAA AAC TGG GCG GCT ACT TCT AAC TTA ACT ATT GCC CGT TTA 4343
   Phe Tyr Glu Asn Trp Ala Ala Thr Ser Asn Leu Thr Ile Ala Arg Leu
   2570 2575 2580
   AGA GAG GCT GGA GTA ATT TGT GCA GAA TCG ACG GAT TTA AAA GGC GAT 4391
   Arg Glu Ala Gly Val Ile Cys Ala Glu Ser Thr Asp Leu Lys Gly Asp
   2585 2590 2595 2600
   GAA AAA AAT AAT ATT GTG TTA GGT AGC CAA AAA GAT AAT AAC TTA TCG 4439
   Glu Lys Asn Asn Ile Val Leu Gly Ser Gln Lys Asp Asn Asn Leu Ser
   2605 2610 2615
   GGT AGT GCA GGT GAT GAT CTA CTT ATC GGC GGA GAG GGT AAT GAT ACG 4487
   Gly Ser Ala Gly Asp Asp Leu Leu Ile Gly Gly Glu Gly Asn Asp Thr
   2620 2625 2630
   TTA AAA GGC AGC TAC GGT GCA GAC ACC TAT ATC TTT AGC AAA GGG CAT 4535
   Leu Lys Gly Ser Tyr Gly Ala Asp Thr Tyr Ile Phe Ser Lys Gly His
   2635 2640 2645
   GGA CAA GAT GTA ATT TAT GAA TAT TCC GAC AGT GCA AAC TCT AAA AAA 4583
   Gly Gln Asp Val Ile Tyr Glu Tyr Ser Asp Ser Ala Asn Ser Lys Lys
   2650 2655 2660
   GAT ATT GAT ACC TTA AAA TTT ACC GAT GTG AAT TAT GCG GAA GTG AAG 4631
   Asp Ile Asp Thr Leu Lys Phe Thr Asp Val Asn Tyr Ala Glu Val Lys
   2665 2670 2675 2680
   TTT CGA CGA GTA GAT AAT GAC TTA ATG TTA TTC GGT TAT CAT GAT ACG 4679
   Phe Arg Arg Val Asp Asn Asp Leu Met Leu Phe Gly Tyr His Asp Thr
   2685 2690 2695
   GAT TCG GTC ACG GTA AAA TCC TTC TAC AGC CAT GTA GAT TAT CAA TTT 4727
   Asp Ser Val Thr Val Lys Ser Phe Tyr Ser His Val Asp Tyr Gln Phe
   2700 2705 2710
   GAC AAA TTG GAG TTT GCT GAC CGC AGT ATA ACT CGC GAT GAA CTG ATT 4775
   Asp Lys Leu Glu Phe Ala Asp Arg Ser Ile Thr Arg Asp Glu Leu Ile
   2715 2720 2725
   AAA GCA GGG CTT CAT CTA TAC GGC ACC GAT GGC AAT GAT GAT ATA AAG 4823
   Lys Ala Gly Leu His Leu Tyr Gly Thr Asp Gly Asn Asp Asp Ile Lys
   2730 2735 2740
   GAT CAT GCG GAT TGG GAC AGC ATT GTG GAA GGC GGC AAA GGC AAC GAT 4871
   Asp His Ala Asp Trp Asp Ser Ile Val Glu Gly Gly Lys Gly Asn Asp
   2745 2750 2755 2760
   ATT CTA AGA GGT GGC TAC GGT GCG GAC ACC TAT ATC TTT AGC AAA GGA 4919
   Ile Leu Arg Gly Gly Tyr Gly Ala Asp Thr Tyr Ile Phe Ser Lys Gly
   2765 2770 2775
   CAC GGA CAG GAT ATC GTT TAT GAA GAT ACC AAT AAT GAT AAC CGA GCA 4967
   His Gly Gln Asp Ile Val Tyr Glu Asp Thr Asn Asn Asp Asn Arg Ala
   2780 2785 2790
   AGA GAT ATC GAC ACC TTA ACA TTT ACT GAT GTG AAT TAT GCG GAA GTG 5015
   Arg Asp Ile Asp Thr Leu Thr Phe Thr Asp Val Asn Tyr Ala Glu Val
   2795 2800 2805
   AAA TTC CGA CGA GTA GAT AAT GAC TTA ATG TTA TTC GGT TAT CAT GAT 5063
   Lys Phe Arg Arg Val Asp Asn Asp Leu Met Leu Phe Gly Tyr His Asp
   2810 2815 2820
   ACG GAT TCG GTC ACG ATA AAA TCC TTC TAC AAC CAT GTA GAT TAT CAA 5111
   Thr Asp Ser Val Thr Ile Lys Ser Phe Tyr Asn His Val Asp Tyr Gln
   2825 2830 2835 2840
   TGT GAC AAA TTG GAC TTT GCT GAC CGC AGT ATA ACT CGT GAT GAA CTA 5159
   Cys Asp Lys Leu Asp Phe Ala Asp Arg Ser Ile Thr Arg Asp Glu Leu
   2845 2850 2855
   GGT AAA CAA GGT ATG GCA TTA TTT GGC ACT GAC GGC GAT GAT AAT ATC 5207
   Gly Lys Gln Gly Met Ala Leu Phe Gly Thr Asp Gly Asp Asp Asn Ile
   2860 2865 2870
   AAC GAC TGG GGA CGT AAC TCG GTG ATT GAT GCC GGT GCG GGT AAT GAT 5255
   Asn Asp Trp Gly Arg Asn Ser Val Ile Asp Ala Gly Ala Gly Asn Asp
   2875 2880 2885
   ACG GTT AAT GGC GGT AAT GGC GAT GAC ACC CTC ATC GGC GGC AAA GGT 5303
   Thr Val Asn Gly Gly Asn Gly Asp Asp Thr Leu Ile Gly Gly Lys Gly
   2890 2895 2900
   AAT GAT ATT CTA AGA GGT GGC TAC GGT GCG GAC ACC TAT ATC TTT AGC 5351
   Asn Asp Ile Leu Arg Gly Gly Tyr Gly Ala Asp Thr Tyr Ile Phe Ser
   2905 2910 2915 2920
   AAA GGA CAC GGA CAG GAT ATC GTT TAT GAA GAT ACC AAT AAT GAT AAC 5399
   Lys Gly His Gly Gln Asp Ile Val Tyr Glu Asp Thr Asn Asn Asp Asn
   2925 2930 2935
   CGC GCA AGA GAT ATC GAC ACC TTA AAA TTT ACT GAT ATT AAT TTA TCC 5447
   Arg Ala Arg Asp Ile Asp Thr Leu Lys Phe Thr Asp Ile Asn Leu Ser
   2940 2945 2950
   GAA CTT TGG TTT AGC CGA GAA AAT AAC GAT TTG ATT ATT AAA TCA TTA 5495
   Glu Leu Trp Phe Ser Arg Glu Asn Asn Asp Leu Ile Ile Lys Ser Leu
   2955 2960 2965
   TTA AGT GAG GAT AAA GTC ACG GTT CAA AAT TGG TAT TCA CAC CAA GAT 5543
   Leu Ser Glu Asp Lys Val Thr Val Gln Asn Trp Tyr Ser His Gln Asp
   2970 2975 2980
   CAT AAA ATA GAA AAT ATT CGT TTA TCG AAT GAG CAA ATG TTG GTG AGC 5591
   His Lys Ile Glu Asn Ile Arg Leu Ser Asn Glu Gln Met Leu Val Ser
   2985 2990 2995 3000
   ACT CAG GTG GAG AAG ATG GTT GAG TCG ATG GCC GGC TTT GCT CAG AAG 5639
   Thr Gln Val Glu Lys Met Val Glu Ser Met Ala Gly Phe Ala Gln Lys
   3005 3010 3015
   CAC GGA GGA GAG ATA TCT CTT CTG TCG CCT GAA GAG GTA AAA CAA TAT 5687
   His Gly Gly Glu Ile Ser Leu Leu Ser Pro Glu Glu Val Lys Gln Tyr
   3020 3025 3030
   ATC AAT AGC TTA ACA GCT GCT TTA TAA CATACGAAAG AAATCGGCAC 5734
   Ile Asn Ser Leu Thr Ala Ala Leu *
   3035 3040
   AGTTTTTGTG AACTGTGCCG ATTTGATTTT AGTGTAAGAA TATAGCCTGA TTTTAAGAAA 5794
   TTTACTCTTG GCTAATAACT ATTTCCCATT TTATAAGTTA TTGACGGATG GTTTTATCAA 5854
   ATATGAGATC AAATCTTATT TTAAATTCGC TTTCCATTAA GCGATATTGA TCTTTTAAGT 5914
   TTGGGGCCGC ATGAGTTTGG AACCGATACC ACTCATTGTG GGAATCAATA CACAATACGC 5974
   TGTAATCGGA CTCTTGCAGT TCATAATAAT GCTTTCTCTC CGTTAATTCT TCTTGCGTAT 6034
   ATGGCGAGAG ATTAAAGCTG AATGGCTGGT TCGCACTAAC AAACAGGTTC TCCGATTTCA 6094
   GATATTCACA ACCGTAATGG CTACCGGTTT CCTGCGGTTT TACATAATTG GTATGATTTT 6154
   GTTTAGCTGT TATACGGTAG ATGCCTAATT GTGGTAAATT GCGTGTGTCA ATATAGCTTT 6214
   CTTGTTCTCC GTAACCGAAA TACTCAATGG CGTTTTCTGT TTTAGCTAAG AAGAAACGTA 6274
   AGCCGAAGCG GGGTAAATAC GGTAATTCGA TCGGGCGAAT AGCGTTAATT TCAACCGAAA 6334
   GTTGTCCGTC ATTGAAGATA CGATAACGAA TATCCAGTGT TAAAATGCGA CCGCGAGAAA 6394
   TTGACACAAT TGCAGATTTT ACTGAAAATT CGACCGCTTG TTCGCTTTGC TGCCACTGAA 6454
   TTTCATACGC TCTGGTATAG GCTTTATCGT AGCCGGCATT TTGGCACGCC TCACGAATGA 6514
   GGCGATCATT GTCGGTTGGC GCACGCCAAA TATTAAAATC TAACGATTGT TGGATAATCG 6574
   CTTTACCGGC TTTTTCAATA CGGGTGAAAA TCCCTTTCTG TTTATCTAAT TGATAACTAA 6634
   ATTGACCGTT GTGTACGTTA ATGTGGAAGC GATCTTCTTG TACTTCAAAT GCACTGTTCT 6694
   CAATTGTGAA TTGTGGTAAT ACTAATTTAT TTTCGCTAAA TAAATTGAGC TGCTCGAAGC 6754
   CAAGTGAATG TGCTTCGTCT AATAATTCGA CCGCGGTATT TAAGCGATAA TTTAAATTCA 6814
   GTAGCCATAA ATGCCCGTTA TTTTTTGGTA ACTCAATCGG TAATACTACG CTGCCGTGCG 6874
   GTTGGCAAGA AACGGATAAA TTCCCACCGC TTGTCACCAC GCCGTTTTCG ACAAATTCGT 6934
   AATCAATCGT TAAATAATCG GCAAGATCAG TGAAATCCAA GTAGTTGTGG ATCACAATTT 6994
   GGTTATCGAT 7004

(2) INFORMATION FOR SEQ ID NO: 4:

(i) SEQUENCE CHARACTERISTICS:
(A) LENGTH: 1383 amino acids
(B) TYPE: amino acid
(D) TOPOLOGY: linear

(ii) MOLECULE TYPE: protein
(xi) SEQUENCE DESCRIPTION: SEQ ID NO: 4:

Met Ser Asp Asn Ala Phe Phe Val Ile Glu Glu Ser Gly Lys Arg Tyr
1               5                   10                  15

Ile Glu Asn Phe Gly Ile Glu Pro Leu Gly Lys Gln Glu Asp Phe Asp
            20                  25                  30

Phe Val Gly Gly Phe Trp Ser Asn Leu Val Asn Arg Gly Leu Glu Ser
        35                  40                  45

Ile Ile Asp Pro Ser Gly Ile Gly Gly Thr Val Asn Leu Asn Phe Thr
    50                  55                  60

Gly Glu Val Glu Thr Tyr Thr Leu Asp Glu Thr Arg Phe Lys Ala Glu
65                  70                  75                  80

Ala Ala Lys Lys Ser His Trp Ser Leu Val Asn Ala Ala Lys Val Tyr
                85                  90                  95

Gly Gly Leu Asp Gln Ile Ile Lys Lys Leu Trp Asp Ser Gly Ser Ile
            100                 105                 110

Lys His Leu Tyr Gln Asp Lys Asp Thr Gly Lys Leu Lys Pro Ile Ile
        115                 120                 125

Tyr Gly Thr Ala Gly Asn Asp Ser Lys Ile Glu Gly Thr Lys Ile Thr
    130                 135                 140

Arg Arg Ile Ala Gly Lys Glu Val Thr Leu Asp Ile Ala Asn Gln Lys
145                 150                 155                 160

Ile Glu Lys Gly Val Leu Glu Lys Leu Gly Leu Ser Val Ser Gly Ser
                165                 170                 175

Asp Ile Ile Lys Leu Leu Phe Gly Ala Leu Thr Pro Thr Leu Asn Arg
            180                 185                 190

Met Leu Leu Ser Gln Leu Ile Gln Ser Phe Ser Asp Ser Leu Ala Lys
        195                 200                 205

Leu Asp Asn Pro Leu Ala Pro Tyr Thr Lys Asn Gly Val Val Tyr Val
    210                 215                 220

Thr Gly Lys Gly Asn Asp Val Leu Lys Gly Thr Glu His Glu Asp Leu
225                 230                 235                 240

Phe Leu Gly Gly Glu Gly Asn Asp Thr Tyr Tyr Ala Arg Val Gly Asp
                245                 250                 255

Thr Ile Glu Asp Ala Asp Gly Lys Gly Lys Val Tyr Phe Val Arg Glu
            260                 265                 270

Lys Gly Val Pro Lys Ala Asp Pro Lys Arg Val Glu Phe Ser Glu Tyr
        275                 280                 285

Ile Thr Lys Glu Glu Ile Lys Glu Val Glu Lys Gly Leu Leu Thr Tyr
    290                 295                 300

Ala Val Leu Glu Asn Tyr Asn Trp Glu Glu Lys Thr Ala Thr Phe Ala
305                 310                 315                 320

His Ala Thr Met Leu Asn Glu Leu Phe Thr Asp Tyr Thr Asn Tyr Arg
                325                 330                 335

Tyr Glu Val Lys Gly Leu Lys Leu Pro Ala Val Lys Lys Leu Lys Ser
            340                 345                 350

Pro Leu Val Glu Phe Thr Ala Asp Leu Leu Thr Val Thr Pro Ile Asp
        355                 360                 365

Glu Asn Gly Lys Ala Leu Ser Glu Lys Ser Ile Thr Val Lys Asn Phe
    370                 375                 380

Lys Asn Gly Asp Leu Gly Ile Arg Leu Leu Asp Pro Asn Ser Tyr Tyr
385                 390                 395                 400

Tyr Phe Leu Glu Gly Gln Asp Thr Gly Phe Tyr Gly Pro Ala Phe Tyr
                405                 410                 415

Ile Glu Arg Lys Asn Gly Gly Gly Ser Lys Asn Asn Ser Ser Gly Ala
            420                 425                 430

Gly Asn Ser Lys Asp Trp Gly Gly Asn Gly His Gly Asn His Arg Asn
        435                 440                 445

Asn Ala Ser Asp Leu Asn Lys Pro Asp Gly Asn Asn Gly Asn Asn Gln
    450                 455                 460

Asn Asn Gly Ser Asn Gln Asp Asn His Ser Asp Val Asn Ala Pro Asn
465                 470                 475                 480

Asn Pro Gly Arg Asn Tyr Asp Ile Tyr Asp Pro Leu Ala Leu Asp Leu
                485                 490                 495

Asp Gly Asp Gly Leu Glu Thr Val Ser Met Asn Gly Arg Gln Gly Ala
            500                 505                 510

Leu Phe Asp His Glu Gly Lys Gly Ile Arg Thr Ala Thr Gly Trp Leu
        515                 520                 525

Ala Ala Asp Asp Gly Phe Leu Val Leu Asp Arg Asn Gln Asp Gly Ile
    530                 535                 540

Ile Asn Asp Ile Ser Glu Leu Phe Ser Asn Lys Asn Gln Leu Ser Asp
545                 550                 555                 560

Gly Ser Ile Ser Ala His Gly Phe Ala Thr Leu Ala Asp Leu Asp Thr
                565                 570                 575

Asn Gln Asp Gln Arg Ile Asp Gln Asn Asp Lys Leu Phe Ser Lys Leu
            580                 585                 590

Gln Ile Trp Arg Asp Leu Asn Gln Asn Gly Phe Ser Glu Ala Asn Glu
        595                 600                 605

Leu Phe Ser Leu Glu Ser Leu Asn Ile Lys Ser Leu His Thr Ala Tyr
    610                 615                 620

Glu Glu Arg Asn Asp Phe Leu Ala Gly Asn Asn Ile Leu Ala Gln Leu
625                 630                 635                 640

Gly Lys Tyr Glu Lys Thr Asp Gly Thr Phe Gly Gln Met Gly Asp Leu
                645                 650                 655

Asn Phe Ser Phe Asn Pro Phe Tyr Ser Arg Phe Thr Glu Ala Leu Asn
            660                 665                 670

Leu Thr Glu Gln Gln Arg Arg Thr Ile Asn Leu Thr Gly Thr Gly Arg
        675                 680                 685

Val Arg Asp Leu Arg Glu Ala Ala Ala Leu Ser Glu Glu Leu Ala Ala
    690                 695                 700

Leu Leu Gln Gln Tyr Thr Lys Gly Ser Asp Phe Gln Ala Gln Arg Glu
705                 710                 715                 720

Leu Leu Pro Ala Ile Leu Asp Lys Trp Ala Ala Thr Asp Leu Gln Tyr
                725                 730                 735

Gln His Tyr Asp Lys Thr Leu Leu Lys Thr Val Glu Ser Thr Asp Ser
            740                 745                 750

Ser Ala Ser Val Val Arg Val Thr Pro Ser Gln Leu Ser Ser Ile Arg
        755                 760                 765

Asn Val Lys His Asp Pro Thr Val Met Gln Asn Cys Glu Gln Ser Lys
    770                 775                 780

Ala Lys Ile Ala Thr Leu Asn Ser Leu Tyr Gly Leu Asn Ile Asp Gln
785                 790                 795                 800

Leu Tyr Tyr Thr Thr Asp Lys Asp Ile Arg Tyr Ile Thr Asp Lys Val
                805                 810                 815

Asn Asn Met Tyr Gln Thr Thr Gly Glu Leu Gly Tyr Arg Ser Leu Leu
            820                 825                 830

Leu Gln Thr Arg Val Lys Lys Tyr Val Tyr Ser Val Asn Ala Lys Gln
        835                 840                 845

Phe Glu Gly Lys Trp Val Ala Asp Tyr Ser Arg Thr Glu Ala Leu Phe
    850                 855                 860

Asn Ser Thr Tyr Lys Gln Ser Pro Glu Asn Val Leu Tyr Asp Leu Arg
865                 870                 875                 880

Glu Tyr Leu Ser Phe Tyr Asn Asp Pro Thr Glu Trp Lys Glu Gly Leu
                885                 890                 895

Leu Leu Leu Ser Arg Tyr Ile Asp Tyr Ala Lys Ala Gln Gly Phe Tyr
            900                 905                 910

Glu Asn Trp Ala Ala Thr Ser Asn Leu Thr Ile Ala Arg Leu Arg Glu
        915                 920                 925

Ala Gly Val Ile Cys Ala Glu Ser Thr Asp Leu Lys Gly Asp Glu Lys
    930                 935                 940

Asn Asn Ile Val Leu Gly Ser Gln Lys Asp Asn Asn Leu Ser Gly Ser
945                 950                 955                 960

Ala Gly Asp Asp Leu Leu Ile Gly Gly Glu Gly Asn Asp Thr Leu Lys
                965                 970                 975

Gly Ser Tyr Gly Ala Asp Thr Tyr Ile Phe Ser Lys Gly His Gly Gln
            980                 985                 990

Asp Val Ile Tyr Glu Tyr Ser Asp Ser Ala Asn Ser Lys Lys Asp Ile
        995                 1000                1005

Asp Thr Leu Lys Phe Thr Asp Val Asn Tyr Ala Glu Val Lys Phe Arg
    1010                1015                1020

Arg Val Asp Asn Asp Leu Met Leu Phe Gly Tyr His Asp Thr Asp Ser
1025                1030                1035                1040

Val Thr Val Lys Ser Phe Tyr Ser His Val Asp Tyr Gln Phe Asp Lys
                1045                1050                1055

Leu Glu Phe Ala Asp Arg Ser Ile Thr Arg Asp Glu Leu Ile Lys Ala
            1060                1065                1070

Gly Leu His Leu Tyr Gly Thr Asp Gly Asn Asp Asp Ile Lys Asp His
        1075                1080                1085

Ala Asp Trp Asp Ser Ile Val Glu Gly Gly Lys Gly Asn Asp Ile Leu
    1090                1095                1100

Arg Gly Gly Tyr Gly Ala Asp Thr Tyr Ile Phe Ser Lys Gly His Gly
1105                1110                1115                1120

Gln Asp Ile Val Tyr Glu Asp Thr Asn Asn Asp Asn Arg Ala Arg Asp
                1125                1130                1135

Ile Asp Thr Leu Thr Phe Thr Asp Val Asn Tyr Ala Glu Val Lys Phe
            1140                1145                1150

Arg Arg Val Asp Asn Asp Leu Met Leu Phe Gly Tyr His Asp Thr Asp
        1155                1160                1165

Ser Val Thr Ile Lys Ser Phe Tyr Asn His Val Asp Tyr Gln Cys Asp
    1170                1175                1180

Lys Leu Asp Phe Ala Asp Arg Ser Ile Thr Arg Asp Glu Leu Gly Lys
1185                1190                1195                1200

Gln Gly Met Ala Leu Phe Gly Thr Asp Gly Asp Asp Asn Ile Asn Asp
                1205                1210                1215

Trp Gly Arg Asn Ser Val Ile Asp Ala Gly Ala Gly Asn Asp Thr Val
            1220                1225                1230

Asn Gly Gly Asn Gly Asp Asp Thr Leu Ile Gly Gly Lys Gly Asn Asp
        1235                1240                1245

Ile Leu Arg Gly Gly Tyr Gly Ala Asp Thr Tyr Ile Phe Ser Lys Gly
    1250                1255                1260

His Gly Gln Asp Ile Val Tyr Glu Asp Thr Asn Asn Asp Asn Arg Ala
1265                1270                1275                1280

Arg Asp Ile Asp Thr Leu Lys Phe Thr Asp Ile Asn Leu Ser Glu Leu
                1285                1290                1295

Trp Phe Ser Arg Glu Asn Asn Asp Leu Ile Ile Lys Ser Leu Leu Ser
            1300                1305                1310

Glu Asp Lys Val Thr Val Gln Asn Trp Tyr Ser His Gln Asp His Lys
        1315                1320                1325

Ile Glu Asn Ile Arg Leu Ser Asn Glu Gln Met Leu Val Ser Thr Gln
    1330                1335                1340

Val Glu Lys Met Val Glu Ser Met Ala Gly Phe Ala Gln Lys His Gly
1345                1350                1355                1360

Gly Glu Ile Ser Leu Leu Ser Pro Glu Glu Val Lys Gln Tyr Ile Asn
                1365                1370                1375

Ser Leu Thr Ala Ala Leu *
            1380

(2) INFORMATION FOR SEQ ID NO: 5:

(i) SEQUENCE CHARACTERISTICS:
(A) LENGTH: 6736 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Actinobacillus pleuropneumoniae
(B) STRAIN: 4074 (serotype 1 reference strain)

(vii) IMMEDIATE SOURCE:
(B) CLONE: pROK7

(ix) FEATURE:
(A) NAME/KEY: CDS
(B) LOCATION:1132..6549
(C) IDENTIFICATION METHOD: experimental
(D) OTHER INFORMATION:/codon_start= 1132
/function= "RTX toxin"
/product= "ApxIV"
/evidence= EXPERIMENTAL
/gene= "ApxIV_v1"

(ix) FEATURE:
(A) NAME/KEY: CDS
(B) LOCATION:639..1178
(D) OTHER INFORMATION:/codon_start= 639
/function= "unknown"
/product= "ORF1"
/gene= "orf1"

(ix) FEATURE:
(A) NAME/KEY: CDS
(B) LOCATION:1..453
(D) OTHER INFORMATION:/partial
/product= "Met-G"
/gene= "mrp"
/standard_name= "mrp"
/label= mrp

(ix) FEATURE:
(A) NAME/KEY: -10_signal
(B) LOCATION:617..623
(D) OTHER INFORMATION:/standard_name= "-10"
/label= -10_s

(ix) FEATURE:
(A) NAME/KEY: -35_signal
(B) LOCATION:594..599
(D) OTHER INFORMATION:/standard_name= "-35_s"
/label= -35_s

(ix) FEATURE:
(A) NAME/KEY: promoter
(B) LOCATION:454..1131
(D) OTHER INFORMATION:/function= "Promoter"
/standard_name= "promoter ApxIV"
/label= promoter

(xi) SEQUENCE DESCRIPTION: SEQ ID NO: 5:

ATC GAT ATG CCG CCG GGT ACG GGC GAT ATC CAA CTT ACT CTT TCG CAA 48
Ile Asp Met Pro Pro Gly Thr Gly Asp Ile Gln Leu Thr Leu Ser Gln
1               5                   10                  15

CAA ATT CCG GTT ACC GGT GCG GTG GTG GTA ACC ACT CCG CAA GAT ATT 96
Gln Ile Pro Val Thr Gly Ala Val Val Val Thr Thr Pro Gln Asp Ile
            20                  25                  30

GCG TTA TTA GAT GCG GTG AAA GGT ATT TCA ATG TTC CAA AAA GTG TCG 144
Ala Leu Leu Asp Ala Val Lys Gly Ile Ser Met Phe Gln Lys Val Ser
        35                  40                  45

GTA CCG GTC TTA GGT ATC ATT GAA AAT ATG AGC GTA CAT ATC TGC CAA 192
Val Pro Val Leu Gly Ile Ile Glu Asn Met Ser Val His Ile Cys Gln
    50                  55                  60

AAT TGC GGT CAC CAC GAA GAT ATT TTC GGC ACC GGC GGT GCG GAG AAA 240
Asn Cys Gly His His Glu Asp Ile Phe Gly Thr Gly Gly Ala Glu Lys
65                  70                  75                  80

GTG GCG AAG AAA TAC GGT ACT AAA GTA TTA GGA CAA ATG CCG TTG CAT 288
Val Ala Lys Lys Tyr Gly Thr Lys Val Leu Gly Gln Met Pro Leu His
                85                  90                  95

ATT CGC TTA CGT CAA GAT TTG GAT GCC GGC ACA CCG ACC GTC GTT GCG 336
Ile Arg Leu Arg Gln Asp Leu Asp Ala Gly Thr Pro Thr Val Val Ala
            100                 105                 110

GCA CCG GAA CAC GAA ACC AGC CGA GCC TAT ATT GAA TTA GCG GCA AAA 384
Ala Pro Glu His Glu Thr Ser Arg Ala Tyr Ile Glu Leu Ala Ala Lys
        115                 120                 125

GTC GCT TCG GAA TTA TAC TGG CAA GGT TCG GTT ATC CCG TCT GAA ATT 432
Val Ala Ser Glu Leu Tyr Trp Gln Gly Ser Val Ile Pro Ser Glu Ile
    130                 135                 140

ATG ATT CGT GAA GTA AAA TAA GTTTTAATAA CCACGAAAAC ACAAAGAACA 483
Met Ile Arg Glu Val Lys *
145                 150

CAAGCGGTAG AATTTGCAGA AAAATTTGCA AATCCTACCG CTTTTTTATT AGTACGATTC 543

GCTGTTGGAC TGCTATTTGA TTTGGTTTGT CAGGATATTA TGTTATTGTA ATGAAATGTT 603

AGTGAATTAT TTTTATTAAT TTGAAAGGAA ACAAAATGAA AATAAAAAAA CGTTACATTG 663

CGCTGTTGGT CTTAGGTGTC GTTATCAGCT ATGCCTGGTA TCAAAATTAT CAATGGGAAC 723

AGCTGATGTT AAGCGGTTAT TGTGAAAAGG ACGGAAGTTA TTTTGATGAT AGGCATACGA 783

AGCAAGAACT GATTGATAGG GCAATTAACT ATATGCTGGA GCATCAATCT AAAAAAACAT 843

ACGATGCTTA TACTGATGAA CCTTTAGAAA TAAAACCATA TTTAACAATA GAGGAATTTA 903

AAAAACTCAA TCCAAATTGT TGTGAAATTA CCTCATGGCC AGCAGATGCA GTTCCACAAG 963

ATTGGGATGT TCGTGTGGAA GGTAAGGCAT ATAGGTATGT AATCGTAAAA TATTTAAGAA 1023

CCTTAGCAAA TAGAGAACCT GAACGATGGG AAACTAGTAT TGTTTTTGAT AATTGCGGCA 1083

ATCCTAAAAG AGCAAGCTAC TTATATTATT TAAAGAGAGA AATTTATT ATG ACA AAA 1140
Met Thr Lys
1

TTA ACT ATG CAA GAT GTG ACC AAT TTA TAT TTA TAT AAA ACG AAA ACT 1188
Leu Thr Met Gln Asp Val Thr Asn Leu Tyr Leu Tyr Lys Thr Lys Thr
5 10 15

CTA CCT AAA GAT AGA TTG GAT GAT TCA CTT ATT TCT GAA ATA GGA AAA 1236
Leu Pro Lys Asp Arg Leu Asp Asp Ser Leu Ile Ser Glu Ile Gly Lys
20 25 30 35

GGA GAT GAT GAT ATT GAT AGA AAA GAA TTT ATG GTG GGG CCG GGA CGT 1284
Gly Asp Asp Asp Ile Asp Arg Lys Glu Phe Met Val Gly Pro Gly Arg
40 45 50

TTT GTG ACC GCT GAT AAC TTT AGC GTT GTA AGA GAT TTT TTT AAT GCT 1332
Phe Val Thr Ala Asp Asn Phe Ser Val Val Arg Asp Phe Phe Asn Ala
55 60 65

GGG AAA TCA CGC ATT ATT GCG CCG CAA GTC CCG CCT ATT CGT TCA CAG 1380
Gly Lys Ser Arg Ile Ile Ala Pro Gln Val Pro Pro Ile Arg Ser Gln
70 75 80

CAG GAA AAA ATC TTG GTC GGT TTA AAA CCG GGC AAA TAT TCC AAA GCG 1428
Gln Glu Lys Ile Leu Val Gly Leu Lys Pro Gly Lys Tyr Ser Lys Ala
85 90 95

CAG ATA TTG GAA ATG CTG GGT TAT ACG AAA GGC GGA GAA GTG GTA AAT 1476
Gln Ile Leu Glu Met Leu Gly Tyr Thr Lys Gly Gly Glu Val Val Asn
100 105 110 115

GGC ATG TTT GCC GGT GAA GTC CAG ACA TTA GGC TTT TAT GAC GAT GGC 1524
Gly Met Phe Ala Gly Glu Val Gln Thr Leu Gly Phe Tyr Asp Asp Gly
120 125 130

AAA GGG GAT TTA CTC GAA CGC GCC TAT ATC TGG AAT ACC ACA GGA TTT 1572
Lys Gly Asp Leu Leu Glu Arg Ala Tyr Ile Trp Asn Thr Thr Gly Phe
135 140 145

AAA ATG AGC GAC AAT GCC TTT TTT GTT ATA GAA GAA TCA GGC AAA CGC 1620

Lys Met Ser Asp Asn Ala Phe Phe Val Ile Glu Glu Ser Gly Lys Arg
150 155 160

TAT ATT GAA AAC TTT GGT ATT GAA CCT CTT GGT AAG CAA GAA GAT TTT 1668
Tyr Ile Glu Asn Phe Gly Ile Glu Pro Leu Gly Lys Gln Glu Asp Phe
165 170 175

GAT TTT GTC GGC GGC TTT TGG TCT AAC TTA GTG AAT CGT GGT TTG GAA 1716
Asp Phe Val Gly Gly Phe Trp Ser Asn Leu Val Asn Arg Gly Leu Glu
180 185 190 195

AGT ATT ATC GAC CCA TCC GGT ATC GGT GGA ACG GTA AAC CTT AAC TTT 1764
Ser Ile Ile Asp Pro Ser Gly Ile Gly Gly Thr Val Asn Leu Asn Phe
200 205 210

ACC GGC GAG GTG GAA ACC TAC ACG TTA GAC GAA ACA AGG TTT AAA GCG 1812
Thr Gly Glu Val Glu Thr Tyr Thr Leu Asp Glu Thr Arg Phe Lys Ala
215 220 225

GAA GCG GCG AAG AAA AGC CAT TGG AGT TTA GTG AAT GCG GCG AAA GTA 1860
Glu Ala Ala Lys Lys Ser His Trp Ser Leu Val Asn Ala Ala Lys Val
230 235 240

TAC GGC GGT TTA GAC CAA ATT ATT AAA AAA CTA TGG GAC AGT GGC TCA 1908
Tyr Gly Gly Leu Asp Gln Ile Ile Lys Lys Leu Trp Asp Ser Gly Ser
245 250 255

ATT AAG CAT TTA TAT CAA GAT AAA GAT ACG GGC AAA TTA AAA CCG ATT 1956
Ile Lys His Leu Tyr Gln Asp Lys Asp Thr Gly Lys Leu Lys Pro Ile
260 265 270 275

ATT TAC GGC ACG GCC GGC AAC GAC AGT AAG ATT GAA GGC ACT AAA ATC 2004
Ile Tyr Gly Thr Ala Gly Asn Asp Ser Lys Ile Glu Gly Thr Lys Ile
280 285 290

ACC CGT AGG ATT GCG GGT AAA GAA GTT ACG CTT GAT ATT GCC AAT CAG 2052
Thr Arg Arg Ile Ala Gly Lys Glu Val Thr Leu Asp Ile Ala Asn Gln
295 300 305

AAA ATT GAA AAA GGC GTG TTA GAG AAA TTG GGG CTG TCT GTT AGT GGT 2100
Lys Ile Glu Lys Gly Val Leu Glu Lys Leu Gly Leu Ser Val Ser Gly
310 315 320

TCG GAT ATC ATT AAA TTG TTG TTT GGA GCA TTG ACT CCA ACT TTA AAT 2148
Ser Asp Ile Ile Lys Leu Leu Phe Gly Ala Leu Thr Pro Thr Leu Asn
325 330 335

AGA ATG TTG CTA TCA CAA CTT ATC CAG TCT TTT TCC GAT AGC TTG GCT 2196
Arg Met Leu Leu Ser Gln Leu Ile Gln Ser Phe Ser Asp Ser Leu Ala
340 345 350 355

AAA CTT GAT AAT CCC TTA GCC CCT TAC ACT AAA AAT GGC GTG GTT TAT 2244
Lys Leu Asp Asn Pro Leu Ala Pro Tyr Thr Lys Asn Gly Val Val Tyr
360 365 370

GTC ACC GGC AAA GGG AAT GAT GTG CTT AAA GGA ACT GAA CAT GAG GAT 2292
Val Thr Gly Lys Gly Asn Asp Val Leu Lys Gly Thr Glu His Glu Asp
375 380 385

TTG TTT CTC GGT GGT GAG GGG AAT GAT ACT TAT TAT GCG AGA GTA GGC 2340
Leu Phe Leu Gly Gly Glu Gly Asn Asp Thr Tyr Tyr Ala Arg Val Gly
390 395 400

GAT ACA ATT GAA GAC GCC GAC GGC AAA GGT AAA GTC TAT TTT GTG AGA 2388
Asp Thr Ile Glu Asp Ala Asp Gly Lys Gly Lys Val Tyr Phe Val Arg
405 410 415

GAA AAA GGG GTA CCT AAG GCG GAT CCT AAG CGG GTA GAG TTT AGC GAG 2436
Glu Lys Gly Val Pro Lys Ala Asp Pro Lys Arg Val Glu Phe Ser Glu
420 425 430 435

TAC ATA ACG AAA GAA GAA ATA AAA GAG GTT GAA AAG GGG TTA TTA ACT 2484
Tyr Ile Thr Lys Glu Glu Ile Lys Glu Val Glu Lys Gly Leu Leu Thr
440 445 450

TAC GCA GTT TTA GAA AAT TAT AAT TGG GAA GAG AAA ACG GCG ACT TTC 2532
Tyr Ala Val Leu Glu Asn Tyr Asn Trp Glu Glu Lys Thr Ala Thr Phe
455 460 465

GCT CAT GCG ACT ATG CTT AAT GAG CTT TTT ACT GAT TAT ACT AAT TAT 2580
Ala His Ala Thr Met Leu Asn Glu Leu Phe Thr Asp Tyr Thr Asn Tyr
470 475 480

CGT TAT GAA GTT AAA GGA CTA AAA TTG CCC GCC GTT AAA AAG TTA AAA 2628
Arg Tyr Glu Val Lys Gly Leu Lys Leu Pro Ala Val Lys Lys Leu Lys

485                    490                    495

AGT CCG TTG GTG GAG TTT ACA GCT GAT TTA TTA ACT GTT ACG CCT ATT          2676
Ser Pro Leu Val Glu Phe Thr Ala Asp Leu Leu Thr Val Thr Pro Ile
500                    505                    510                    515

GAC GAA AAC GGA AAA GCA CTT AGC GAA AAA AGT ATT ACG GTT AAA AAT          2724
Asp Glu Asn Gly Lys Ala Leu Ser Glu Lys Ser Ile Thr Val Lys Asn
                520                    525                    530

TTT AAA AAT GGT GAT TTA GGA ATA AGG TTG TTG GAT CCT AAT AGC TAT          2772
Phe Lys Asn Gly Asp Leu Gly Ile Arg Leu Leu Asp Pro Asn Ser Tyr
            535                    540                    545

TAT TAT TTC CTT GAA GGC CAA GAT ACG GGT TTT TAT GGT CCT GCT TTT          2820
Tyr Tyr Phe Leu Glu Gly Gln Asp Thr Gly Phe Tyr Gly Pro Ala Phe
        550                    555                    560

TAT ATT GAA CGA AAA AAC GGT GGC GGC GCT AAA AAT AAC TCG TCG GGA          2868
Tyr Ile Glu Arg Lys Asn Gly Gly Gly Ala Lys Asn Asn Ser Ser Gly
    565                    570                    575

GCA GGA AAT AGC AAA GAT TGG GGC GGG AAC GGG CAT GGA AAT CAC CGA          2916
Ala Gly Asn Ser Lys Asp Trp Gly Gly Asn Gly His Gly Asn His Arg
580                    585                    590                    595

AAT AAT GCC TCC GAC CTG AAT AAA CCG GAC GGA AAT AAT GGG AAT AAC          2964
Asn Asn Ala Ser Asp Leu Asn Lys Pro Asp Gly Asn Asn Gly Asn Asn
                600                    605                    610

CAA AAT AAC GGA AGC AAT CAA GAT AAT CAT AGC GAT GTG AAT GCG CCA          3012
Gln Asn Asn Gly Ser Asn Gln Asp Asn His Ser Asp Val Asn Ala Pro
            615                    620                    625

AAT AAC CCG GGA CGT AAC TAT GAT ATT TAC GAT CCT TTA GCT TTA GAT          3060
Asn Asn Pro Gly Arg Asn Tyr Asp Ile Tyr Asp Pro Leu Ala Leu Asp
        630                    635                    640

TTA GAT GGA GAT GGG CTT GAA ACC GTG TCG ATG AAC GGG CGA CAA GGC          3108
Leu Asp Gly Asp Gly Leu Glu Thr Val Ser Met Asn Gly Arg Gln Gly
    645                    650                    655

GCG TTA TTC GAT CAT GAA GGA AAA GGT ATT CGT ACC GCA ACG GGC TGG          3156
Ala Leu Phe Asp His Glu Gly Lys Gly Ile Arg Thr Ala Thr Gly Trp
660                    665                    670                    675

CTC GCT GCG GAT GAC GGT TTT TTA GTG TTA GAT CGT AAC CAA GAC GGC          3204
Leu Ala Ala Asp Asp Gly Phe Leu Val Leu Asp Arg Asn Gln Asp Gly
                680                    685                    690

ATT ATT AAT GAT ATA AGC GAG TTA TTT AGT AAT AAA AAT CAA CTT TCC          3252
Ile Ile Asn Asp Ile Ser Glu Leu Phe Ser Asn Lys Asn Gln Leu Ser
            695                    700                    705

GAC GGC AGT ATT TCT GCA CAC GGT TTT GCG ACA TTA GCC GAT TTG GAT          3300
Asp Gly Ser Ile Ser Ala His Gly Phe Ala Thr Leu Ala Asp Leu Asp
        710                    715                    720

ACA AAC CAA GAT CAG CGT ATC GAC CAA AAT GAT AAG CTG TTT TCT AAA          3348
Thr Asn Gln Asp Gln Arg Ile Asp Gln Asn Asp Lys Leu Phe Ser Lys
    725                    730                    735

CTC CAA ATT TGG CGG GAT TTA AAT CAA AAC GGT TTT AGT GAA GCG AAT          3396
Leu Gln Ile Trp Arg Asp Leu Asn Gln Asn Gly Phe Ser Glu Ala Asn
740                    745                    750                    755

GAG CTG TTT AGC TTA GAA AGT TTG AAT ATT AAA TCT TTA CAT ACC GCC          3444
Glu Leu Phe Ser Leu Glu Ser Leu Asn Ile Lys Ser Leu His Thr Ala
                760                    765                    770

TAT GAA GAG CGT AAT GAT TTT CTA GCG GGC AAT AAT ATC CTT GCT CAG          3492
Tyr Glu Glu Arg Asn Asp Phe Leu Ala Gly Asn Asn Ile Leu Ala Gln
            775                    780                    785

CTT GGG AAG TAT GAA AAA ACG GAC GGT ACT TTT GCA CAA ATG GGC GAT          3540
Leu Gly Lys Tyr Glu Lys Thr Asp Gly Thr Phe Ala Gln Met Gly Asp
        790                    795                    800

TTA AAT TTC AGT TTT AAC CCG TTT TAT AGC CGA TTT ACC GAA GCG TTA          3588
Leu Asn Phe Ser Phe Asn Pro Phe Tyr Ser Arg Phe Thr Glu Ala Leu
    805                    810                    815

AAT TTA ACC GAG CAA CAA CGT CGC ACA ATT AAT CTA ACC GGC ACC GGT          3636
Asn Leu Thr Glu Gln Gln Arg Arg Thr Ile Asn Leu Thr Gly Thr Gly
820                    825                    830                    835

CGG GTT CGG GAT TTG CGT GAA GCC GCC GCA CTT TCT GAG GAG TTG GCT 3684
Arg Val Arg Asp Leu Arg Glu Ala Ala Ala Leu Ser Glu Glu Leu Ala
840 845 850

GCT TTA TTA CAA CAG TAC ACT AAG GCC TCC GAT TTT CAG GCA CAA CGA 3732
Ala Leu Leu Gln Gln Tyr Thr Lys Ala Ser Asp Phe Gln Ala Gln Arg
855 860 865

GAA TTA TTG CCT GCC ATT TTA GAT AAA TGG GCG GCA ACG GAT TTA CAG 3780
Glu Leu Leu Pro Ala Ile Leu Asp Lys Trp Ala Ala Thr Asp Leu Gln
870 875 880

TAT CAA CAT TAT GAT AAA ACA TTA CTT AAA ACG GTA GAA AGT ACC GAT 3828
Tyr Gln His Tyr Asp Lys Thr Leu Leu Lys Thr Val Glu Ser Thr Asp
885 890 895

AGT AGT GCT TCT GTC GTT AGA GTC ACG CCT TCT CAA TTA AGT AGT ATA 3876
Ser Ser Ala Ser Val Val Arg Val Thr Pro Ser Gln Leu Ser Ser Ile
900 905 910 915

CGC AAT GCA AAG CAT GAT CCT ACC GTT ATG CAA AAC TTT GAA CAG AGT 3924
Arg Asn Ala Lys His Asp Pro Thr Val Met Gln Asn Phe Glu Gln Ser
920 925 930

AAG GCA AAA ATT GCG ACT TTA AAT TCG CTC TAC GGG TTA AAT ATC GAT 3972
Lys Ala Lys Ile Ala Thr Leu Asn Ser Leu Tyr Gly Leu Asn Ile Asp
935 940 945

CAA CTT TAT TAC ACG ACG GAT AAA GAC ATT CGC TAT ATT ACT GAT AAA 4020
Gln Leu Tyr Tyr Thr Thr Asp Lys Asp Ile Arg Tyr Ile Thr Asp Lys
950 955 960

GTG AAT AAT ATG TAT CAA ACA ACC GTA GAA CTT GCC TAC CGT TCT TTA 4068
Val Asn Asn Met Tyr Gln Thr Thr Val Glu Leu Ala Tyr Arg Ser Leu
965 970 975

CTT TTA CAA ACG CGT TTG AAG AAA TAT GTT TAT AGC GTT AAT GCG AAA 4116
Leu Leu Gln Thr Arg Leu Lys Lys Tyr Val Tyr Ser Val Asn Ala Lys
980 985 990 995

CAA TTC GAA GGG AAA TGG GTA ACC GAT TAT TCT CGT ACT GAA GCC TTA 4164
Gln Phe Glu Gly Lys Trp Val Thr Asp Tyr Ser Arg Thr Glu Ala Leu
1000 1005 1010

TTT AAC TCT ACT TTT AAA CAA TCG CCT GAA AAT GCA TTA TAT GAT TTA 4212
Phe Asn Ser Thr Phe Lys Gln Ser Pro Glu Asn Ala Leu Tyr Asp Leu
1015 1020 1025

AGC GAA TAC CTT TCT TTC TTT AAC GAT CCT ACG GAA TGG AAA GAA GGG 4260
Ser Glu Tyr Leu Ser Phe Phe Asn Asp Pro Thr Glu Trp Lys Glu Gly
1030 1035 1040

CTA TTA CTG TTA AGC CGT TAT ATA GAT TAT GCT AAA GCA CAA GGA TTT 4308
Leu Leu Leu Leu Ser Arg Tyr Ile Asp Tyr Ala Lys Ala Gln Gly Phe
1045 1050 1055

TAT GAA AAC TGG GCG GCT ACT TCT AAC TTA ACT ATT GCC CGT TTA AGA 4356
Tyr Glu Asn Trp Ala Ala Thr Ser Asn Leu Thr Ile Ala Arg Leu Arg
1060 1065 1070 1075

GAG GCT GGA GTA ATT TTT GCA GAA TCG ACG GAT TTA AAA GGC GAT GAA 4404
Glu Ala Gly Val Ile Phe Ala Glu Ser Thr Asp Leu Lys Gly Asp Glu
1080 1085 1090

AAA AAT AAT ATT TTG TTA GGT AGC CAA AAA GAT AAT AAC TTA TCG GGT 4452
Lys Asn Asn Ile Leu Leu Gly Ser Gln Lys Asp Asn Asn Leu Ser Gly
1095 1100 1105

AGT GCA GGT GAT GAT CTA CTT ATC GGC GGA GAG GGT AAT GAT ACG TTA 4500
Ser Ala Gly Asp Asp Leu Leu Ile Gly Gly Glu Gly Asn Asp Thr Leu
1110 1115 1120

AAA GGC AGC TAC GGT GCA GAC ACC TAT ATC TTT AGC AAA GGA CAC GGA 4548
Lys Gly Ser Tyr Gly Ala Asp Thr Tyr Ile Phe Ser Lys Gly His Gly
1125 1130 1135

CAG GAT ATC GTT TAT GAA GAT ACC AAT AAT GAT AAC CGC GCA AGA GAT 4596
Gln Asp Ile Val Tyr Glu Asp Thr Asn Asn Asp Asn Arg Ala Arg Asp
1140 1145 1150 1155

ATC GAC ACC TTA AAA TTT ACC GAT GTG AAT TAT GCG GAA GTG AAG TTT 4644
Ile Asp Thr Leu Lys Phe Thr Asp Val Asn Tyr Ala Glu Val Lys Phe
1160 1165 1170

CGA CGA GTA GAT AAT GAC TTA ATG TTA TTC GGT TAT CAT GAT ACG GAT 4692

Arg Arg Val Asp Asn Asp Leu Met Leu Phe Gly Tyr His Asp Thr Asp
1175 1180 1185

TCG GTC ACG GTA AAA TCC TTC TAC AGC CAT GTA GAT TAT CAA TTT GAC 4740
Ser Val Thr Val Lys Ser Phe Tyr Ser His Val Asp Tyr Gln Phe Asp
1190 1195 1200

AAA TTG GAG TTT GCT GAC CGC AGT ATA ACT CGC GAT GAA CTG ATT AAA 4788
Lys Leu Glu Phe Ala Asp Arg Ser Ile Thr Arg Asp Glu Leu Ile Lys
1205 1210 1215

GCA GGG CTT CAT CTA TAC GGC ACC GAT GGC AAT GAT GAT ATA AAG GAT 4836
Ala Gly Leu His Leu Tyr Gly Thr Asp Gly Asn Asp Asp Ile Lys Asp
1220 1225 1230 1235

CAT GCG GAT TGG GAC AGC ATT TTG GAA GGC GGC AAA GGC AAC GAT ATT 4884
His Ala Asp Trp Asp Ser Ile Leu Glu Gly Gly Lys Gly Asn Asp Ile
1240 1245 1250

CTA AGA GGT GGC TAC GGT GCG GAC ACC TAT ATC TTT AGC AAA GGA CAC 4932
Leu Arg Gly Gly Tyr Gly Ala Asp Thr Tyr Ile Phe Ser Lys Gly His
1255 1260 1265

GGA CAG GAT ATC GTT TAT GAA GAT ACC AAT AAT GAT AAC CGC GCA AGA 4980
Gly Gln Asp Ile Val Tyr Glu Asp Thr Asn Asn Asp Asn Arg Ala Arg
1270 1275 1280

GAT ATC GAC ACC TTA AAA TTT ACT GAT GTG AAT TAT GCG GAA GTG AAA 5028
Asp Ile Asp Thr Leu Lys Phe Thr Asp Val Asn Tyr Ala Glu Val Lys
1285 1290 1295

TTC CGA CGA GTA GAT AAT GAC TTA ATG TTA TTC GGT TAT CAT GAT ACG 5076
Phe Arg Arg Val Asp Asn Asp Leu Met Leu Phe Gly Tyr His Asp Thr
1300 1305 1310 1315

GAT TCG GTC ACG ATA AAA TCC TTC TAC AAC CAT GTA GAT TAT CAA TTT 5124
Asp Ser Val Thr Ile Lys Ser Phe Tyr Asn His Val Asp Tyr Gln Phe
1320 1325 1330

GAC AAA TTG GAA TTT GCT GAC CGC AGT ATA ACT CGT GAT GAA CTA GGT 5172
Asp Lys Leu Glu Phe Ala Asp Arg Ser Ile Thr Arg Asp Glu Leu Gly
1335 1340 1345

AAA CAA GGT ATG GCA TTA TTT GGC ACT GAC GGT GAT GAT AAT ATC AAC 5220
Lys Gln Gly Met Ala Leu Phe Gly Thr Asp Gly Asp Asp Asn Ile Asn
1350 1355 1360

GAC TGG GGA CGT AAC TCG GTG ATT GAT GCC GGT GCG GGT AAT GAT ACG 5268
Asp Trp Gly Arg Asn Ser Val Ile Asp Ala Gly Ala Gly Asn Asp Thr
1365 1370 1375

GTT AAT GGC GGT AAT GGC GAT GAC ACC CTC ATC GGC GGC AAA GGT AAT 5316
Val Asn Gly Gly Asn Gly Asp Asp Thr Leu Ile Gly Gly Lys Gly Asn
1380 1385 1390 1395

GAT ATT CTA AGA GGT GGC TAC GGT GCG GAC ACC TAT ATC TTT AGC AAA 5364
Asp Ile Leu Arg Gly Gly Tyr Gly Ala Asp Thr Tyr Ile Phe Ser Lys
1400 1405 1410

GGA CAC GGA CAG GAT ATC GTT TAT GAA GAT ACC AAT AAT GAT AAC CGC 5412
Gly His Gly Gln Asp Ile Val Tyr Glu Asp Thr Asn Asn Asp Asn Arg
1415 1420 1425

GCA AGA GAT ATC GAC ACC TTA AAA TTT ACC GAT GTG AAT TAT GCG GAA 5460
Ala Arg Asp Ile Asp Thr Leu Lys Phe Thr Asp Val Asn Tyr Ala Glu
1430 1435 1440

GTG AAA TTC CGA CGA GTA GAT AAT GAC TTA ATG TTA TTC GGT TAT CAT 5508
Val Lys Phe Arg Arg Val Asp Asn Asp Leu Met Leu Phe Gly Tyr His
1445 1450 1455

GAT ACG GAT TCG GTC ACG GTA AAA TCC TTC TAC AGC CAT GTA GAT TAT 5556
Asp Thr Asp Ser Val Thr Val Lys Ser Phe Tyr Ser His Val Asp Tyr
1460 1465 1470 1475

CAA TTT GAC AAA TTG GAG TTT GCT GAC CGC AGT ATA ACT CGC GAT GAA 5604
Gln Phe Asp Lys Leu Glu Phe Ala Asp Arg Ser Ile Thr Arg Asp Glu
1480 1485 1490

CTG ATT AAA GCA GGG CTT CAT CTA TAC GGC ACC GAT GGC AAT GAT GAT 5652
Leu Ile Lys Ala Gly Leu His Leu Tyr Gly Thr Asp Gly Asn Asp Asp
1495 1500 1505

ATA AAG GAT CAT GCG GAT TGG GAC AGC ATT TTG GAA GGC GGC AAA GGC 5700
Ile Lys Asp His Ala Asp Trp Asp Ser Ile Leu Glu Gly Gly Lys Gly

1510                    1515                    1520

AAC GAT ATT CTA AGA GGT GGC TAC GGT GCG GAC ACC TAT ATC TTT AGC          5748
Asn Asp Ile Leu Arg Gly Gly Tyr Gly Ala Asp Thr Tyr Ile Phe Ser
    1525                    1530                    1535

AAA GGA CAC GGA CAG GAT ATC GTT TAT GAA GAT ACC AAT AAT GAT AAC          5796
Lys Gly His Gly Gln Asp Ile Val Tyr Glu Asp Thr Asn Asn Asp Asn
1540                    1545                    1550                    1555

CGA GCA AGA GAT ATC GAC ACC TTA AAA TTT ACT GAT GTG AAT TAT GCG          5844
Arg Ala Arg Asp Ile Asp Thr Leu Lys Phe Thr Asp Val Asn Tyr Ala
                1560                    1565                    1570

GAA GTG AAA TTC CGA CGA GTA GAT AAT GAC TTA ATG TTA TTC GGT TAT          5892
Glu Val Lys Phe Arg Arg Val Asp Asn Asp Leu Met Leu Phe Gly Tyr
            1575                    1580                    1585

CAT GAT ACG GAT TCG GTC ACG ATA AAA TCC TTC TAC AAC CAT GTA GAT          5940
His Asp Thr Asp Ser Val Thr Ile Lys Ser Phe Tyr Asn His Val Asp
        1590                    1595                    1600

TAT CAA TTT GAC AAA TTG GAA TTT GCT GAC CGC AGT ATA ACT CGT GAT          5988
Tyr Gln Phe Asp Lys Leu Glu Phe Ala Asp Arg Ser Ile Thr Arg Asp
    1605                    1610                    1615

GAA CTA GGT AAA CAA GGT ATG GCA TTA TTT GGC ACT GAC GGT GAT GAT          6036
Glu Leu Gly Lys Gln Gly Met Ala Leu Phe Gly Thr Asp Gly Asp Asp
1620                    1625                    1630                    1635

AAT ATC AAC GAC TGG GGA CGT AAC TCG GTG ATT GAT GCC GGT GCG GGT          6084
Asn Ile Asn Asp Trp Gly Arg Asn Ser Val Ile Asp Ala Gly Ala Gly
                1640                    1645                    1650

AAT GAT ACG GTT AAT GGC GGT AAT GGC GAT GAC ACC CTC ATC GGC GGC          6132
Asn Asp Thr Val Asn Gly Gly Asn Gly Asp Asp Thr Leu Ile Gly Gly
            1655                    1660                    1665

AAA GGT AAT GAT ATT CTA AGA GGT GGC TAC GGT GCG GAC ACC TAT ATC          6180
Lys Gly Asn Asp Ile Leu Arg Gly Gly Tyr Gly Ala Asp Thr Tyr Ile
        1670                    1675                    1680

TTT AGC AAA GGA CAC GGA CAG GAT ATC GTT TAT GAA GAT ACC AAT AAT          6228
Phe Ser Lys Gly His Gly Gln Asp Ile Val Tyr Glu Asp Thr Asn Asn
    1685                    1690                    1695

GAT AAC CGC GCA AGA GAT ATC GAC ACC TTA AAA TTT ACT GAT ATT AAT          6276
Asp Asn Arg Ala Arg Asp Ile Asp Thr Leu Lys Phe Thr Asp Ile Asn
1700                    1705                    1710                    1715

TTA TCC GAA CTT TGG TTT AGC CGA GAA AAT AAC GAT TTG ATT ATT AAA          6324
Leu Ser Glu Leu Trp Phe Ser Arg Glu Asn Asn Asp Leu Ile Ile Lys
                1720                    1725                    1730

TCA TTA TTA AGT GAG GAT AAA GTC ACG GTT CAA AAT TGG TAT TCA CAC          6372
Ser Leu Leu Ser Glu Asp Lys Val Thr Val Gln Asn Trp Tyr Ser His
            1735                    1740                    1745

CAA GAT CAT AAA ATA GAA AAT ATT CGT TTA TCG AAT GAG CAA ACG TTG          6420
Gln Asp His Lys Ile Glu Asn Ile Arg Leu Ser Asn Glu Gln Thr Leu
        1750                    1755                    1760

GTG AGC ACT CAG GTG GAG AAG ATG GTT GAG TCG ATG GCC GGC TTT GCT          6468
Val Ser Thr Gln Val Glu Lys Met Val Glu Ser Met Ala Gly Phe Ala
    1765                    1770                    1775

CAG AAG CAC GGA GGA GAG ATA TCT CTT GTG TCG CTT GAA GAG GTA AAA          6516
Gln Lys His Gly Gly Glu Ile Ser Leu Val Ser Leu Glu Glu Val Lys
1780                    1785                    1790                    1795

CAA TAT ATC AAT AGC TTA ACA GCT GCT TTA TAA CATACGAAAG AAATCGGCAC          6569
Gln Tyr Ile Asn Ser Leu Thr Ala Ala Leu  *
                1800                    1805

AGTTTTTTTG AACTGTGCCG ATTTGATTTT AGTGTAAGAA TATAGCCTGA TTTTAAGAAA          6629

TTTACTCTTG GCTAATAACT ATTTCCCATT TTATAAGTTA TTGACGGATG GTTTTATCAA          6689

ATATGAGATC AAATCTTATT TTAAATTCGC TTTCCATTAA GCGATAT                    6736

(2) INFORMATION FOR SEQ ID NO: 6:

    (i) SEQUENCE CHARACTERISTICS:
        (A) LENGTH: 151 amino acids

(B) TYPE: amino acid
(D) TOPOLOGY: linear

(ii) MOLECULE TYPF: protein
(xi) SEQUENCE DESCRIPTION: SEQ ID NO: 6:

Ile Asp Met Pro Pro Gly Thr Gly Asp Ile Gln Leu Thr Leu Ser Gln
1               5                   10                  15

Gln Ile Pro Val Thr Gly Ala Val Val Val Thr Thr Pro Gln Asp Ile
            20                  25                  30

Ala Leu Leu Asp Ala Val Lys Gly Ile Ser Met Phe Gln Lys Val Ser
        35                  40                  45

Val Pro Val Leu Gly Ile Ile Glu Asn Met Ser Val His Ile Cys Gln
    50                  55                  60

Asn Cys Gly His His Glu Asp Ile Phe Gly Thr Gly Gly Ala Glu Lys
65                  70                  75                  80

Val Ala Lys Lys Tyr Gly Thr Lys Val Leu Gly Gln Met Pro Leu His
                85                  90                  95

Ile Arg Leu Arg Gln Asp Leu Asp Ala Gly Thr Pro Thr Val Val Ala
            100                 105                 110

Ala Pro Glu His Glu Thr Ser Arg Ala Tyr Ile Glu Leu Ala Ala Lys
        115                 120                 125

Val Ala Ser Glu Leu Tyr Trp Gln Gly Ser Val Ile Pro Ser Glu Ile
    130                 135                 140

Met Ile Arg Glu Val Lys  *
145                 150

(2) INFORMATION FOR SEQ ID NO: 7:

(i) SEQUENCE CHARACTERISTICS:
(A) LENGTH: 1806 amino acids
(B) TYPE: amino acid
(D) TOPOLOGY: linear

(ii) MOLECULE TYPE: protein
(xi) SEQUENCE DESCRIPTION: SEQ ID NO: 7:

Met Thr Lys Leu Thr Met Gln Asp Val Thr Asn Leu Tyr Leu Tyr Lys
1               5                   10                  15

Thr Lys Thr Leu Pro Lys Asp Arg Leu Asp Asp Ser Leu Ile Ser Glu
            20                  25                  30

Ile Gly Lys Gly Asp Asp Asp Ile Asp Arg Lys Glu Phe Met Val Gly
        35                  40                  45

Pro Gly Arg Phe Val Thr Ala Asp Asn Phe Ser Val Val Arg Asp Phe
    50                  55                  60

Phe Asn Ala Gly Lys Ser Arg Ile Ile Ala Pro Gln Val Pro Pro Ile
65                  70                  75                  80

Arg Ser Gln Gln Glu Lys Ile Leu Val Gly Leu Lys Pro Gly Lys Tyr
                85                  90                  95

Ser Lys Ala Gln Ile Leu Glu Met Leu Gly Tyr Thr Lys Gly Gly Glu
            100                 105                 110

Val Val Asn Gly Met Phe Ala Gly Glu Val Gln Thr Leu Gly Phe Tyr
        115                 120                 125

Asp Asp Gly Lys Gly Asp Leu Leu Glu Arg Ala Tyr Ile Trp Asn Thr
    130                 135                 140

Thr Gly Phe Lys Met Ser Asp Asn Ala Phe Phe Val Ile Glu Glu Ser
145                 150                 155                 160

Gly Lys Arg Tyr Ile Glu Asn Phe Gly Ile Glu Pro Leu Gly Lys Gln
                165                 170                 175

Glu Asp Phe Asp Phe Val Gly Gly Phe Trp Ser Asn Leu Val Asn Arg
            180                 185                 190

Gly Leu Glu Ser Ile Ile Asp Pro Ser Gly Ile Gly Gly Thr Val Asn
        195                 200                 205

Leu Asn Phe Thr Gly Glu Val Glu Thr Tyr Thr Leu Asp Glu Thr Arg
    210                 215                 220

Phe Lys Ala Glu Ala Ala Lys Lys Ser His Trp Ser Leu Val Asn Ala
225                 230                 235                 240

Ala Lys Val Tyr Gly Gly Leu Asp Gln Ile Ile Lys Lys Leu Trp Asp
                245                 250                 255

Ser Gly Ser Ile Lys His Leu Tyr Gln Asp Lys Asp Thr Gly Lys Leu
            260                 265                 270

Lys Pro Ile Ile Tyr Gly Thr Ala Gly Asn Asp Ser Lys Ile Glu Gly
        275                 280                 285

Thr Lys Ile Thr Arg Arg Ile Ala Gly Lys Glu Val Thr Leu Asp Ile
    290                 295                 300

Ala Asn Gln Lys Ile Glu Lys Gly Val Leu Glu Lys Leu Gly Leu Ser
305                 310                 315                 320

Val Ser Gly Ser Asp Ile Ile Lys Leu Leu Phe Gly Ala Leu Thr Pro
                325                 330                 335

Thr Leu Asn Arg Met Leu Leu Ser Gln Leu Ile Gln Ser Phe Ser Asp
            340                 345                 350

Ser Leu Ala Lys Leu Asp Asn Pro Leu Ala Pro Tyr Thr Lys Asn Gly
        355                 360                 365

Val Val Tyr Val Thr Gly Lys Gly Asn Asp Val Leu Lys Gly Thr Glu
    370                 375                 380

His Glu Asp Leu Phe Leu Gly Gly Glu Gly Asn Asp Thr Tyr Tyr Ala
385                 390                 395                 400

Arg Val Gly Asp Thr Ile Glu Asp Ala Asp Gly Lys Gly Lys Val Tyr
                405                 410                 415

Phe Val Arg Glu Lys Gly Val Pro Lys Ala Asp Pro Lys Arg Val Glu
            420                 425                 430

Phe Ser Glu Tyr Ile Thr Lys Glu Glu Ile Lys Glu Val Glu Lys Gly
        435                 440                 445

Leu Leu Thr Tyr Ala Val Leu Glu Asn Tyr Asn Trp Glu Glu Lys Thr
    450                 455                 460

Ala Thr Phe Ala His Ala Thr Met Leu Asn Glu Leu Phe Thr Asp Tyr
465                 470                 475                 480

Thr Asn Tyr Arg Tyr Glu Val Lys Gly Leu Lys Leu Pro Ala Val Lys
                485                 490                 495

Lys Leu Lys Ser Pro Leu Val Glu Phe Thr Ala Asp Leu Leu Thr Val
            500                 505                 510

Thr Pro Ile Asp Glu Asn Gly Lys Ala Leu Ser Glu Lys Ser Ile Thr
        515                 520                 525

Val Lys Asn Phe Lys Asn Gly Asp Leu Gly Ile Arg Leu Leu Asp Pro
    530                 535                 540

Asn Ser Tyr Tyr Tyr Phe Leu Glu Gly Gln Asp Thr Gly Phe Tyr Gly
545                 550                 555                 560

Pro Ala Phe Tyr Ile Glu Arg Lys Asn Gly Gly Gly Ala Lys Asn Asn
                565                 570                 575

Ser Ser Gly Ala Gly Asn Ser Lys Asp Trp Gly Gly Asn Gly His Gly
            580                 585                 590

Asn His Arg Asn Asn Ala Ser Asp Leu Asn Lys Pro Asp Gly Asn Asn
        595                 600                 605

Gly Asn Asn Gln Asn Asn Gly Ser Asn Gln Asp Asn His Ser Asp Val
    610                 615                 620

Asn Ala Pro Asn Asn Pro Gly Arg Asn Tyr Asp Ile Tyr Asp Pro Leu
625                 630                 635                 640

Ala Leu Asp Leu Asp Gly Asp Gly Leu Glu Thr Val Ser Met Asn Gly
                645                 650                 655

Arg Gln Gly Ala Leu Phe Asp His Glu Gly Lys Gly Ile Arg Thr Ala

            660                 665                 670

Thr Gly Trp Leu Ala Ala Asp Asp Gly Phe Leu Val Leu Asp Arg Asn
        675                 680                 685

Gln Asp Gly Ile Ile Asn Asp Ile Ser Glu Leu Phe Ser Asn Lys Asn
    690                 695                 700

Gln Leu Ser Asp Gly Ser Ile Ser Ala His Gly Phe Ala Thr Leu Ala
705                 710                 715                 720

Asp Leu Asp Thr Asn Gln Asp Gln Arg Ile Asp Gln Asn Asp Lys Leu
                725                 730                 735

Phe Ser Lys Leu Gln Ile Trp Arg Asp Leu Asn Gln Asn Gly Phe Ser
            740                 745                 750

Glu Ala Asn Glu Leu Phe Ser Leu Glu Ser Leu Asn Ile Lys Ser Leu
        755                 760                 765

His Thr Ala Tyr Glu Glu Arg Asn Asp Phe Leu Ala Gly Asn Asn Ile
    770                 775                 780

Leu Ala Gln Leu Gly Lys Tyr Glu Lys Thr Asp Gly Thr Phe Ala Gln
785                 790                 795                 800

Met Gly Asp Leu Asn Phe Ser Phe Asn Pro Phe Tyr Ser Arg Phe Thr
                805                 810                 815

Glu Ala Leu Asn Leu Thr Glu Gln Gln Arg Arg Thr Ile Asn Leu Thr
            820                 825                 830

Gly Thr Gly Arg Val Arg Asp Leu Arg Glu Ala Ala Ala Leu Ser Glu
        835                 840                 845

Glu Leu Ala Ala Leu Leu Gln Gln Tyr Thr Lys Ala Ser Asp Phe Gln
    850                 855                 860

Ala Gln Arg Glu Leu Leu Pro Ala Ile Leu Asp Lys Trp Ala Ala Thr
865                 870                 875                 880

Asp Leu Gln Tyr Gln His Tyr Asp Lys Thr Leu Leu Lys Thr Val Glu
                885                 890                 895

Ser Thr Asp Ser Ser Ala Ser Val Val Arg Val Thr Pro Ser Gln Leu
            900                 905                 910

Ser Ser Ile Arg Asn Ala Lys His Asp Pro Thr Val Met Gln Asn Phe
        915                 920                 925

Glu Gln Ser Lys Ala Lys Ile Ala Thr Leu Asn Ser Leu Tyr Gly Leu
    930                 935                 940

Asn Ile Asp Gln Leu Tyr Tyr Thr Thr Asp Lys Asp Ile Arg Tyr Ile
945                 950                 955                 960

Thr Asp Lys Val Asn Asn Met Tyr Gln Thr Thr Val Glu Leu Ala Tyr
                965                 970                 975

Arg Ser Leu Leu Leu Gln Thr Arg Leu Lys Lys Tyr Val Tyr Ser Val
            980                 985                 990

Asn Ala Lys Gln Phe Glu Gly Lys Trp Val Thr Asp Tyr Ser Arg Thr
        995                 1000                1005

Glu Ala Leu Phe Asn Ser Thr Phe Lys Gln Ser Pro Glu Asn Ala Leu
    1010                1015                1020

Tyr Asp Leu Ser Glu Tyr Leu Ser Phe Phe Asn Asp Pro Thr Glu Trp
1025                1030                1035                1040

Lys Glu Gly Leu Leu Leu Leu Ser Arg Tyr Ile Asp Tyr Ala Lys Ala
                1045                1050                1055

Gln Gly Phe Tyr Glu Asn Trp Ala Ala Thr Ser Asn Leu Thr Ile Ala
            1060                1065                1070

Arg Leu Arg Glu Ala Gly Val Ile Phe Ala Glu Ser Thr Asp Leu Lys
        1075                1080                1085

Gly Asp Glu Lys Asn Asn Ile Leu Leu Gly Ser Gln Lys Asp Asn Asn
    1090                1095                1100

Leu Ser Gly Ser Ala Gly Asp Asp Leu Leu Ile Gly Gly Glu Gly Asn
1105                1110                1115                1120

Asp Thr Leu Lys Gly Ser Tyr Gly Ala Asp Thr Tyr Ile Phe Ser Lys
                1125                1130                1135

Gly His Gly Gln Asp Ile Val Tyr Glu Asp Thr Asn Asn Asp Asn Arg
            1140                1145                1150

Ala Arg Asp Ile Asp Thr Leu Lys Phe Thr Asp Val Asn Tyr Ala Glu
        1155                1160                1165

Val Lys Phe Arg Arg Val Asp Asn Asp Leu Met Leu Phe Gly Tyr His
    1170                1175                1180

Asp Thr Asp Ser Val Thr Val Lys Ser Phe Tyr Ser His Val Asp Tyr
1185                1190                1195                1200

Gln Phe Asp Lys Leu Glu Phe Ala Asp Arg Ser Ile Thr Arg Asp Glu
                1205                1210                1215

Leu Ile Lys Ala Gly Leu His Leu Tyr Gly Thr Asp Gly Asn Asp Asp
            1220                1225                1230

Ile Lys Asp His Ala Asp Trp Asp Ser Ile Leu Glu Gly Gly Lys Gly
        1235                1240                1245

Asn Asp Ile Leu Arg Gly Gly Tyr Gly Ala Asp Thr Tyr Ile Phe Ser
    1250                1255                1260

Lys Gly His Gly Gln Asp Ile Val Tyr Glu Asp Thr Asn Asn Asp Asn
1265                1270                1275                1280

Arg Ala Arg Asp Ile Asp Thr Leu Lys Phe Thr Asp Val Asn Tyr Ala
                1285                1290                1295

Glu Val Lys Phe Arg Arg Val Asp Asn Asp Leu Met Leu Phe Gly Tyr
            1300                1305                1310

His Asp Thr Asp Ser Val Thr Ile Lys Ser Phe Tyr Asn His Val Asp
        1315                1320                1325

Tyr Gln Phe Asp Lys Leu Glu Phe Ala Asp Arg Ser Ile Thr Arg Asp
    1330                1335                1340

Glu Leu Gly Lys Gln Gly Met Ala Leu Phe Gly Thr Asp Gly Asp Asp
1345                1350                1355                1360

Asn Ile Asn Asp Trp Gly Arg Asn Ser Val Ile Asp Ala Gly Ala Gly
                1365                1370                1375

Asn Asp Thr Val Asn Gly Gly Asn Gly Asp Asp Thr Leu Ile Gly Gly
            1380                1385                1390

Lys Gly Asn Asp Ile Leu Arg Gly Gly Tyr Gly Ala Asp Thr Tyr Ile
        1395                1400                1405

Phe Ser Lys Gly His Gly Gln Asp Ile Val Tyr Glu Asp Thr Asn Asn
    1410                1415                1420

Asp Asn Arg Ala Arg Asp Ile Asp Thr Leu Lys Phe Thr Asp Val Asn
1425                1430                1435                1440

Tyr Ala Glu Val Lys Phe Arg Arg Val Asp Asn Asp Leu Met Leu Phe
                1445                1450                1455

Gly Tyr His Asp Thr Asp Ser Val Thr Val Lys Ser Phe Tyr Ser His
            1460                1465                1470

Val Asp Tyr Gln Phe Asp Lys Leu Glu Phe Ala Asp Arg Ser Ile Thr
        1475                1480                1485

Arg Asp Glu Leu Ile Lys Ala Gly Leu His Leu Tyr Gly Thr Asp Gly
    1490                1495                1500

Asn Asp Asp Ile Lys Asp His Ala Asp Trp Asp Ser Ile Leu Glu Gly
1505                1510                1515                1520

Gly Lys Gly Asn Asp Ile Leu Arg Gly Gly Tyr Gly Ala Asp Thr Tyr
                1525                1530                1535

Ile Phe Ser Lys Gly His Gly Gln Asp Ile Val Tyr Glu Asp Thr Asn
            1540                1545                1550

Asn Asp Asn Arg Ala Arg Asp Ile Asp Thr Leu Lys Phe Thr Asp Val
        1555                1560                1565

Asn Tyr Ala Glu Val Lys Phe Arg Arg Val Asp Asn Asp Leu Met Leu

1570                1575                1580

Phe Gly Tyr His Asp Thr Asp Ser Val Thr Ile Lys Ser Phe Tyr Asn
1585                1590                1595                1600

His Val Asp Tyr Gln Phe Asp Lys Leu Glu Phe Ala Asp Arg Ser Ile
                1605                1610                1615

Thr Arg Asp Glu Leu Gly Lys Gln Gly Met Ala Leu Phe Gly Thr Asp
            1620                1625                1630

Gly Asp Asp Asn Ile Asn Asp Trp Gly Arg Asn Ser Val Ile Asp Ala
        1635                1640                1645

Gly Ala Gly Asn Asp Thr Val Asn Gly Gly Asn Gly Asp Asp Thr Leu
    1650                1655                1660

Ile Gly Gly Lys Gly Asn Asp Ile Leu Arg Gly Gly Tyr Gly Ala Asp
1665                1670                1675                1680

Thr Tyr Ile Phe Ser Lys Gly His Gly Gln Asp Ile Val Tyr Glu Asp
                1685                1690                1695

Thr Asn Asn Asp Asn Arg Ala Arg Asp Ile Asp Thr Leu Lys Phe Thr
            1700                1705                1710

Asp Ile Asn Leu Ser Glu Leu Trp Phe Ser Arg Glu Asn Asn Asp Leu
        1715                1720                1725

Ile Ile Lys Ser Leu Leu Ser Glu Asp Lys Val Thr Val Gln Asn Trp
    1730                1735                1740

Tyr Ser His Gln Asp His Lys Ile Glu Asn Ile Arg Leu Ser Asn Glu
1745                1750                1755                1760

Gln Thr Leu Val Ser Thr Gln Val Glu Lys Met Val Glu Ser Met Ala
                1765                1770                1775

Gly Phe Ala Gln Lys His Gly Gly Glu Ile Ser Leu Val Ser Leu Glu
            1780                1785                1790

Glu Val Lys Gln Tyr Ile Asn Ser Leu Thr Ala Ala Leu  *
        1795                1800                1805